# EUROPEAN PATENT SPECIFICATION

(11) **EP 4 669 337 B1**
(45) Date of publication and mention of the grant of the patent: **24.06.2026**
(21) Application number: 24776593.6
(22) Date of filing: 25.09.2024
(51) Int. Cl.: A61K 38/16, C07K 14/215

(54) **AGENTS USEFUL FOR THE PREVENTION OR TREATMENT OF ALLERGIC & INFLAMMATORY DISORDERS**
MITTEL ZUR PRÄVENTION ODER BEHANDLUNG VON ALLERGISCHEN UND ENTZÜNDLICHEN ERKRANKUNGEN
AGENTS UTILES POUR LA PRÉVENTION OU LE TRAITEMENT DE TROUBLES ALLERGIQUES ET INFLAMMATOIRES

(30) Priority: 26.09.2023 EP 23199913
(43) Date of publication of application: 31.12.2025
(73) Proprietor: ATOPIA THERAPEUTICS SA, 1202 Genève (CH)
(72) Inventor: SHAW, Jeffrey Paoletti, 1234 Vessy (CH)
(74) Representative: reuteler & cie SA
(86) International application number: PCT/EP2024/076948
(87) International publication number: WO 2025/068296

(56) References cited:
- EP-A1- 2 902 037
- WO-A1-2015/114576

## Description

### Field of the invention

The present invention relates to agents presenting anti-allergic, anti-inflammatory and antifibrotic activity useful for the prevention or treatment of allergic and inflammatory disorders.

### Background of the Invention

The prevalence of allergic disorders such as allergic or atopic asthma, atopic dermatitis (eczema), atopic rhinitis (hay fever), food allergies (Warren et al., 2020, Curr Allergy Asthma Rep, 20(2), p. 6), occupational allergies, allergic conjunctivitis, allergic broncho-pulmonary aspergillosis, eosinophilic esophagitis, and other IgE-mediated diseases have reached epidemic proportions in both developed and developing populations. The so-called atopic march is a working concept illustrating the sequential onset and natural history of atopic diseases in predisposed individuals (Spergel et al., 2003, J Allergy Clin Immunol, 112(6 Suppl): p. S118-27*;* Zheng et al., 2011, Allergy Asthma Immunol Res, 3(2): p. 67-73). Atopic dermatitis and food allergy are typically diagnosed during infancy, while allergic asthma and allergic rhinitis develop later during childhood. The pathophysiology of the atopic march involves multiple immunological pathways, including host response to allergen exposure, environmental pollutants, skin barrier dysfunction, type 2 inflammation, and oxidative stress, which all promote its progression (Tsuge et al., 2021, Current Insights into Atopic March. Children (Basel), 8(11*)*). Many hypotheses for this prevalence increase have been proposed, including a decrease of early childhood infections or microbial exposure due to improved sanitation and urban dwelling, as well as the gradual loss of the indigenous microbiota through the use of antibiotics, improved hygiene, and birth by caesarean (von Mutius, 2007, Immunobiology, 212(6): p. 433-9*;* Strachan, 1989, BMJ, 1989. 299(6710): p. 1259-60*;* Strachan, 2000, Thorax, 55 Suppl 1: p. S2-10).

All the current therapeutic options for the allergic disorders discussed below target the symptoms, but never the causes of these disorders, with the exception of allergen immunotherapy (AIT), which is the only disease-modifying treatment available for some of the IgE-mediated allergic disorders. Many of the current treatments have been available for decades, while some of the more recent have severe side-effects that limit their use to the more severe forms of allergic disease and are very expensive. There is thus an important need for new strategies for the prevention of allergy development or its treatment, particularly for children and young people that present an atopic background.

Asthma is now the most prevalent chronic disease in childhood in developed countries. In 2019, approximately 262 million people globally were believed to suffer from asthma, and it is believed to have caused 455,000 deaths (Diseases, G.B.D. and C. Injuries, Global burden of 369 diseases and injuries in 204 countries and territories, 1990-2019: a systematic analysis for the Global Burden of Disease Study 2019. Lancet, 2020, 396(10258), 1204-1222). The Global Initiative of Asthma (GINA) defines asthma as a chronic inflammatory disorder of the airways and associated with airway hyper-responsiveness, which leads to the classical symptoms of asthma: recurrent episodes of wheezing, breathlessness, chest tightness and coughing. The most common clinical phenotype is allergic asthma. In childhood, more than 90% of patients with severe asthma are allergic; among asthmatic adults, 60% are sensitized to common aeroallergens (Johansson et al., 2001, Allergy, 56(9): p. 813-24*;* Akar-Ghibril et al., 2020, J Allergy Clin Immunol Pract, 8(2): p. 429-440). In allergic asthma, inflammation and airway obstruction are triggered by allergen exposure in atopic individuals.

The current treatment of acute symptoms of asthma involves inhaled or oral corticosteroids, short- or long-lasting β2-adrenergic receptor agonists (such as salbutamol), antihistamines and leukotriene receptor antagonists (Castillo et al., 2017, J Allergy Clin Immunol Pract, 5(4): p. 918-927). In very severe cases, intravenous administration of corticosteroids or immunomodulatory drugs such as neutralizing antibodies to interleukins may be required. Anti-IgE (omalizumab), anti-IL5 (mepolizumab), anti-IL5Rα (benralizumab), anti-IL4Rα (dupilumab), anti-IL5 (reslizumab) and anti-TSLP (tezepelumab) antibodies have all been approved for the treatment of severe forms of asthma, although with notable side-effects (Kardas et al., 2022, Front Immunol, 13: p. 983852*;* Brusselle et al., 2022, Biologic Therapies for Severe Asthma. N Engl J Med, 386(2): p. 157-171).

Atopic dermatitis (AD), also called atopic eczema, is a common inflammatory skin disease, often with an important allergic element. AD is a chronic, relapsing inflammatory skin condition affecting around 20% of children, and up to 10% of adults in high-income countries and believed to affect up to 2.4% of the world's population (Urban et al., 2021, JAAD Int, 2: p. 12-18) with a 10% lifetime prevalence. The pathogenesis is complex, involving genetic susceptibility, impaired skin barrier function, dysfunctional cell-mediated immunity, and environmental and lifestyle factors. AD is also associated with sleep disruption (mainly due to pruritus), decreased work productivity, depression, anxiety, and poor quality of life, which all carry additional health and economic burdens for the patients, their families and society. In 2017, the estimated number of new patients with AD was 27 million (Xue et al., 2022, Front Cell Infect Microbiol, 12: p. 861053). The prevalence of AD also varies by race: in the USA, the prevalence among whites (11%) is lower than that among African Americans (17%) while the prevalence of AD in infancy in China can be as high as 30.5% (Guo et al., 2019, J Eur Acad Dermatol Venereol, 33(8): p. 1569-1576). There is also a definite link between asthma and atopic dermatitis, with 25.7% of patients suffering from atopic dermatitis also suffering from asthma (95% CI, 23.7 - 27.7), compared to 8.1% for reference patients not suffering from AD (OR 3.03, 95% CI 2.64 - 3.47) (Ravnborg et al., 2021, J Am Acad Dermatol, 84(2): p. 471-478). AD is also associated with an increased prevalence of rhinitis with a pooled prevalence of 40.5% (95% CI 39.0 - 42.0) in patients with AD, compared to 18% in patients without AD (95% 2.26 - 4.66). Furthermore, the pooled prevalence of having both rhinitis and asthma was 14.2% (95% CI 13.0-15.5) in patients with AD. There was an association between AD and rhinitis (OR 3.00, 95% CI 2.83-3.18), allergic rhinitis (OR 3.25, 95% CI 2.26-4.66), and nonallergic rhinitis (OR 1.99, 95% CI 1.39-2.86), respectively (Knudgaard et al., 2021, Ann Allergy Asthma Immunol, 127(1): p. 49-56 *e1*)*.* Atopic dermatitis typically starts in childhood, with 60% of patients developing AD before one year of age and 90% by five years of age (Fichenfield, et al., 2014, J Am Acad Dermatol, 71(1): p. 116-32). Compared with children who do not have AD, those who have the condition are more likely to develop food and environmental allergies (15% vs. 4%), asthma (25% vs. 12%), and allergic rhinitis (34% vs. 14%) (Silverberg et al., 2013, Pediatr. Allergy Immunol, 24(5): p. 476-86).

The treatment of AD depends very much on the degree of severity of the disease. The baseline therapy includes the use of emollients and avoidance of allergens, while mild cases include use of topical calcineurin inhibitors, topical antihistamines, and corticosteroids, in moderate case, the use of narrow band ultraviolet B or UVA1 can be added. In severe cases of AD, azathioprine, cyclosporine, dupilumab (anti-IL-4Rα, blocks IL4 & IL13) approved by the FDA for moderate to severe AD in patients down to 6 months old, tralokinumab (anti-IL13) approved by the FDA in moderate to severe AD in patients 18 years and older, JAK inhibitors (abrocitinib, baricitinib and upadacitinib), systemic corticosteroids and methotrexate may be required (Wollenberg et al., 2022, J Eur Acad Dermatol Venereol, 36(9): p. 1409-1431*;* Wollenberg et al., 2022, J Eur Acad Dermatol Venereol, 36(11): p. 1904-1926).

Allergic rhinitis (AR) is an IgE-mediated inflammatory nasal condition resulting from allergen introduction in a sensitized individual and is defined as a process which includes 3 cardinal symptoms: sneezing, nasal obstruction, and mucus discharge. Symptoms typically occur with allergen exposure in the allergic patient. The prevalence of AR is approximately 10% to 40% depending on geographic location, with the highest incidence occurring in children (Bauchau et al., 2004, Eur Respir J, 24(5): p. 758-64*;* Asher et al., 2006, Lancet, 368(9537): p. 733-43).

The treatment of AR is similar to that of the other allergic disorders of the atopic march, and includes nasal/oral glucocorticoids, antihistamines, leukotriene receptor antagonists, mast cell membrane stabilizers, and decongestants (Meng et al., 2019, Allergy, 74(12): p. 2320-2328). AIT techniques, including crude allergen extracts, purified or recombinant allergens and modified allergens like allergoids, purified peptides, and new adjuvants have been employed. While both sublingual and subcutaneous allergen-specific immunotherapy (AIT) have been used for years in the treatment of AR, they suffer from the same drawbacks as for the other allergic disorders, i.e. long-lasting medication time, occasional severe side-effects, low patient adherence and high costs over long periods (Meng et al., 2020, Allergy, 75(12): p. 3069-3076).

Food allergies, coupled with atopic dermatitis, are a key early component of the atopic march. While the rise in the prevalence of these disorders is undisputed, their actual prevalence is difficult to determine. The vast majority of data concerning the prevalence is questionnaire-based and is often not supported by IgE-mediated symptoms. In the US, a population-based cross-sectional prevalence survey of over 50,000 households published in 2018 estimated that IgE-mediated food allergy is likely to affect approximately 1 in 10 adults (Gupta et al., 2019, JAMA Netw Open, 2(1): p. e185630) and 1 in 12 children (Gupta et al., 2018, Pediatrics, 142(6*)*). Altogether, these data indicate that over 10% of the US population is likely to suffer from at least one IgE-mediated food allergy - with even more individuals reporting current food allergy in the absence of convincingly IgE-mediated symptoms. The data from other countries is similarly difficult to obtain and specially to compare. Nonetheless, a primary prevalence of 5% to 10% of the juvenile population is generally accepted. An example of a more stringent study can be found in the HealthNuts study (Osborne et al., 2011, J Allergy Clin Immunol, 127(3): p. 668-76 *e1-2*) in which the challenge-proven food allergy prevalence was found to be over 10% in a population-based cohort of 12-month-old infants. While the methods for estimating paediatric food allergy prevalence varies between countries, most estimates remain in the 5 to 10% window (*Warren et al., 2020, supra*). The annual economic cost of food allergy in the US has been estimated to be USD 24.8 billion in a study published in 2013 (Gupta et al., 2013, JAMA Pediatr, 167(11): p. 1026-31) with annual out-of-pocket costs related to food allergy adding a further USD 14.2 billion to the burden.

While the most common treatment for food allergies is an elimination diet, in which the offending food is avoided, there are now several types of other treatments available. In severe cases of food allergy, symptomatic treatments such as antihistamines and epinephrin autoinjectors may be required. However, more long-term AIT strategies are also available, if the offending allergen has been identified, and are generally performed by the allergist by administering increasing doses of the allergen. There is also a single recent (2020) FDA-approved medication for the treatment of peanut butter allergy, Palforzia^{®}, which is an OIT treatment.

Eosinophilic esophagitis (EoE) is a type 2, antigen driven disease in which chronic, eosinophil rich inflammation causes symptoms of esophageal dysfunction (Racca et al., 2021, Front Physiol, 12: p. 815842). EoE symptoms include heartburn/regurgitation, vomiting, dysphagia, food impactions, and even abdominal pain, and the untreated disease can progress to esophageal remodelling, rigidity, and luminal narrowing (Gonsalves et al., 2020, J Allergy Clin Immunol, 145(1): p. 1-7). There are relatively few options for the treatment of EoE. Acid blockers, such as proton pump inhibitors were first prescribed but with relatively poor efficacy. Topical steroids can also be tested, and the FDA recently (2022) approved dupilumab (anti Il-4Rα mAb) for treatment of adults and children older than 12 years with EoE (Dellon et al., 2022, N Engl J Med, 387(25), 2317-2330).

*Helicobacter pylori* is a bacterium colonizing the gastric mucosa of humans. It is typically acquired at birth, usually persists for the entire life span of the host and currently infects almost 50% of the world's population (Robinson et al., 2021, Annu Rev Pathol, 16, 123-144). It is capable of resisting the human adaptive immune response driven in large part by Th1 and/or Th17-polarized effector T-cells by adapting and manipulating the human innate and adaptive immune systems (Salama et al., 2013, Nat Rev Microbiol., 11(6), 385-99).

*H. pylori* infection protects effectively against allergen-induced asthma that is induced by allergen sensitization and challenge (Arnold et al., 2011, J Clin Invest, 121(8),3088-93*;* Reuter et al., 2023, Front Immunol, 14: p. 1092801) in mice models of asthma. Mechanistically, asthma protection is due to the development of (Treg-mediated) immune tolerance to H. pylori, which also protects against other allergen-specific Th2 responses (*Reuter et al., 2023, supra;* Altobelli et al., 2019, mBio, 10(2*)*).

Aside from Tregs, dendritic cells (DCs) have emerged as a critical cell type required for immune tolerance. H. pylori-experienced DCs are re-programmed towards a tolerance-promoting phenotype *in vitro* and *in vivo* (Oertli et al., 2013, Proc Natl Acad Sci USA, 110(8), 3047 - 3052).

It has been observed that the vacuolating cytotoxin (VacA) is sufficient to induce the DC re-programming since *H. pylori* mutants lacking VacA (but otherwise wild type), fail to re-program DCs *in vivo* and *in vitro* and therefore cannot induce Tregs with suppressive activity in mice (*Oertli et al., 2013, supra)*. The potential use of VacA as an extract of *H. pylori,* as purified extract of *H. pylori,* or as a purified polypeptide has been the subject of a first patent WO 2015/114575.

The VacA protein is exclusively expressed by *H. pylori* strains, with the closest non-Helicobacter analogues, based on amino acid sequence alignment, having less than 25% identity. All *H. pylori* strains contain a single chromosomal vacA gene. The genus Helicobacter includes at least 20 different species, but intact vacA genes are present only in *H. pylori* and *H. cetorum*, a species isolated from the stomach or fecal contents of marine mammals *(*Kersulyte, 2013, PLoS One, 8(12), e83177). The VacA protein is expressed as a 140 kDa precursor, which is post-translationally cleaved at both the N-terminus (Sec-dependent removal of a 33-amino acid secretion signal) and the C-terminus (removal of 50 kDa fragment, which is believed to be an autotransporter β-barrel involved in the secretion of VacA into the extracellular space (Schmitt et al., 1994, Mol Microbiol, 12(2): p. 307-19) probably by a type V mode of secretion) to produce the mature secreted 88 kDa protein.

VacA proteins can be identified as belonging either to the s1m1 family, or the s2m2 family (Atherton et al., 1995, J Biol Chem, 270(30): 17771-77*;* Atherton et al., 1997,. Gastroenterology, 112 (1), 92-99*;* Rhead et al., 2007, Gastroenterology, 133(3), 926-36). The s1m1 family is the best characterized, and the most widespread, while the s2m2 family is relatively smaller and less well studied. The alignment of the amino acid sequences of 1259 VacA proteins described in Soyfoo et al., 2021, BMC Mol Cell Biol, 22(1): p. 4 clearly separates these two families, with 784 proteins clearly of the s1m1 family, and 213 being clearly s2m2 (the remaining 262 appeared to be mainly incomplete sequences. Both major families (s1m1 and s2m2) of VacA have the anti-allergic properties, since the anti-allergic activities of the s1m1 family have been observed in allergic asthma (Engler et al., 2014, Proc Natl Acad Sci U S A, 111(32), 11810-5) and food allergy (Kyburz et al., 2017, Clin Exp Allergy, 47(10), 1331-1341) by administration of a purified s1m1 family member and the anti-allergic properties of the s2m2 family were confirmed using an intact living *H. pylori strain* expressing only the s2m2 version compared to the properties of a ΔvacA strain in which the vacA gene had been deleted (*Oertli et al., 2013, supra)*.

While the immunomodulatory activity of VacA had been described, no studies had been performed to determine whether this activity requires the pore-forming activity or whether it was independent of it. In fact, there were no studies on the structure-function relationship for the immunomodulatory properties, while such a study had been performed for the pore-forming activity (Ivie et al., 2008, Infect Immun 76(7), 2843-51). The unusual properties and unique sequence of the VacA family members has prompted numerous efforts to determine the three-dimensional structure of this protein. The physicochemical properties of the VacA protein and its relatively large size hindered such efforts.

Since all the current treatments of allergic disorders induce more or less severe side effects or are limited for some of those disorders, alternative treatment strategies are needed. Therefore, there are important needs for new strategies of prevention of development of those disorders.

### Summary of the invention

The invention relates to the unexpected identification of regions, motifs, and amino acids of the VacA protein that are absolutely required for its anti-allergic activity and are distinct from any other biological activities that have been ascribed to the VacA protein, notably its vacuole-inducing properties (Szabo et al., 1999, The EMBO Journal, 18(20), 5517-5527). This identification led to the design of polypeptides whose activities were confirmed in cellular assays performed on human PBMC-derived dendritic cells or macrophages, which have been previously identified as being the cells targeted by the wild-type VacA protein (*Reuter et al., 2023, supra*).

Indeed, the study of the biological activity of VacA proteins has been hampered by their propensity to oligomerize at pH above that of the stomach. The studies performed in cell assays always use a physiological pH of 7.4, since this what is required for cells to grow. However, at pH 7.4 VacA proteins oligomerize into hexamers and subsequently into dimer of hexamers to produce very large molecular weight insoluble oligomeric complexes (Cover et al., 1997, J. Cell Biol, 138, 759-69). These studies at pH 7.4 have led to the discovery of the pore-forming abilities of the VacA, and the ion channel pores that it can create, either in the cell membrane or in the mitochondrial membrane (*Ivie et al., 2008, supra*). However, VacA is secreted by *H. pylori* into the stomach, where the pH is 1.5 to 3.5 and at this pH, the protein is essentially monomeric. In order to study the biochemistry and immunotolerance properties of VacA in cellular assays, it would be necessary to identify and remove regions of the protein that are responsible for the oligomerisation, while maintaining the region of the protein required for immunotolerance. This invention describes new polypeptides of the VacA protein that lack any oligomerisation or pore-forming properties, are stable at a physiological pH of 7.4, and which maintain their immunotolerance properties.

The unexpectedly very different physicochemical properties of these molecules compared to the wild-type has allowed their study in cell-based assays, which has ultimately led to the identification of cell-surface receptors through which they signal and thereby induce immunotolerance (earlier reports of possible receptors of VacA proteins were never confirmed (for example, Fujikawa et al., 2003, Nat Genet, 33(3) 375-81). The study of the polypeptides of the invention has further led to the identification of their binding to, and signalling through, human TGF-β receptor subunits.

It was therefore unexpected to find polypeptides of the invention which maintain the immunotolerizing activity of VacA, while having lost the other properties (pore-forming and vacuolisation activities). The polypeptides of the invention bind and induce signal transduction in their target cells (dendritic cells and macrophages) in the lamina propria) through their specific binding to cell-surface receptors of the TGF-β family. The binding of VacA protein to TGF-β receptors has never been described in the literature and is entirely novel and unexpected from the prior art.

A first aspect of the invention provides a polypeptide of the invention and variants thereof.

Another aspect of the invention provides a polypeptide of the invention for inducing a tolerization response to an allergen.

Another aspect of the invention provides a polypeptide of the invention for reducing an inflammatory response to an allergen.

Another aspect of the invention provides a polypeptide of the invention, a fragment, or a variant thereof for use in the prevention and/or treatment of an allergic disorder, in particular atopic asthma and/or inducing a tolerization response to an allergen and/or reducing an inflammatory response to an allergen.

Another aspect of the invention provides a polypeptide of the invention, a fragment, or a variant thereof for use in the prevention and/or treatment of an autoimmune disease or disorder or a fibrotic disease or disorder.

Another aspect of the invention provides a polypeptide of the invention, a fragment, or a variant thereof for the preparation of a medicament for the prevention and/or treatment of an allergic disorder, in particular atopic asthma and/or inducing a tolerization response to an allergen and/or reducing an inflammatory response to an allergen and/or the prevention and/or treatment of an autoimmune disease or disorder or a fibrotic disease or disorder.

Another aspect of the invention provides a pharmaceutical formulation comprising a polypeptide of the invention, a fragment, or a variant thereof and at least one pharmaceutically acceptable carrier.

Another aspect of the invention provides a method of inducing a tolerization response to an allergen or reducing an inflammatory response to an allergen in a subject, said method comprising administering in a subject in need thereof an effective amount a polypeptide selected from a polypeptide of the invention, a fragment, or a variant thereof, or a pharmaceutical formulation thereof.

Another aspect of the invention provides a method of preventing, repressing, or treating an allergic response, in particular, an allergic disorder in a subject, said method comprising administering in a subject in need thereof a therapeutically effective amount a polypeptide selected from a polypeptide of the invention, a fragment or a variant thereof, or a pharmaceutical formulation thereof.

### Description of the figures

**Figure 1** shows the induction of expression of IL-10 and TGF-β in THP-1 derived M2-macrophages by polypeptides of the invention as described in Example 1.
**Figure 2** shows the induction of expression of IL-10 and TGF-β by murine bone marrow-derived dendritic cells by polypeptides of the invention.

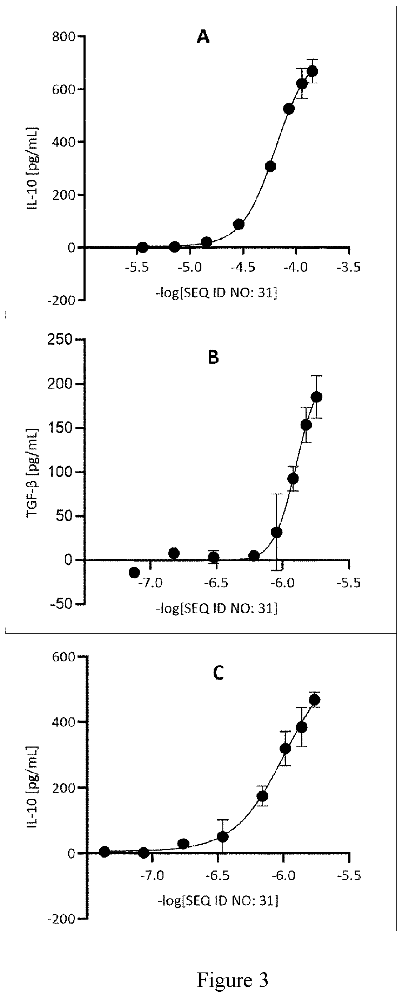

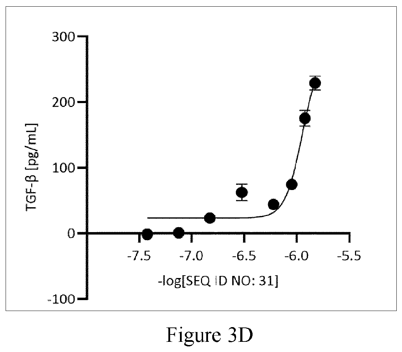

**Figure 3** shows the IC₅₀ values for SEQ ID NO:31 against IL-10 and TGF-β expression in murine bone marrow-derived M2-macrophages or dendritic cells as determined in Example 1. **A:** IL-10 levels in supernatants of murine bone marrow-derived dendritic cells after addition of different peptide concentrations; **B:** TGF-β levels in supernatants of murine bone marrow-derived dendritic cells after addition of different peptide concentrations; **C:** IL-10 levels in supernatants of murine bone marrow-derived M2-macrophages after addition of different peptide concentrations; **D:** TGF-β levels in supernatants of murine bone marrow-derived M2-macrophages after addition of different peptide concentrations.

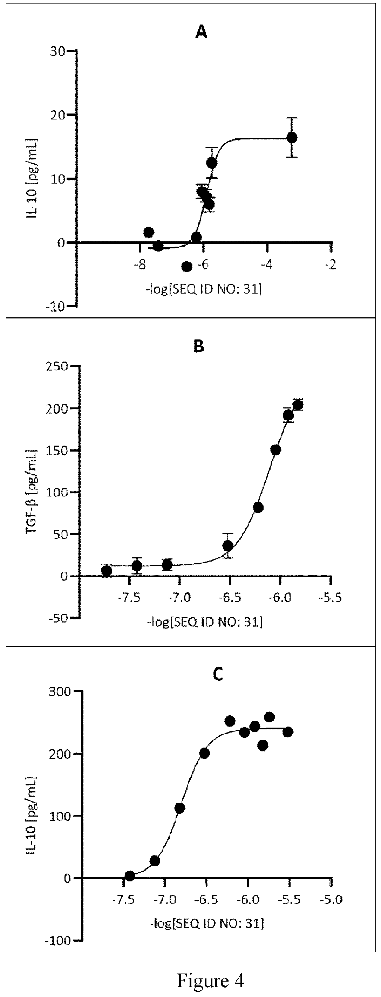

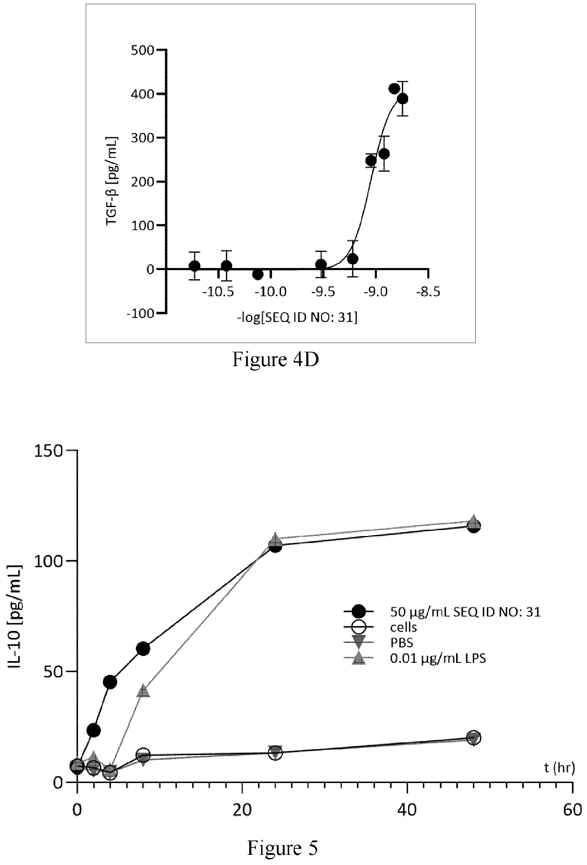

**Figure 4** shows the IC₅₀ values for SEQ ID NO:31 against IL-10 and TGF-β expression in human PBMC (hPBMC)-derived M2-macrophages or dendritic cells measured as described in Example 1. **A:** IL-10 levels in supernatants of hPBMC-derived dendritic cells after addition of increasing concentrations of SEQ ID NO:31; **B:** TGF-β levels in supernatants of hPBMC-derived dendritic cells after addition of increasing concentrations of a polypeptide of SEQ ID NO:31; **C:** IL-10 levels in supernatants of hPBMC-derived M2-macrophages after addition of increasing concentrations of SEQ ID NO:31; **D:** TGF-β levels in supernatants of hPBMC-derived M2-macrophages after addition of increasing concentrations of a polypeptide of SEQ ID NO:31.
**Figure 5** represents the time course of the induction of expression of IL-10 in human PBMC-derived M2-macrophages by SEQ ID NO:31 as described in Example 1.
**Figure 6** represents the effect of SEQ ID NO:31 on lung function in a murine model of acute allergic asthma as described in Example 2. **(A)** Airway resistance (Rrs) in response to MCh-provocation (measured by FlexiVent^{®}) in mice challenged with HDM in the absence or presence of treatment with a polypeptide of SEQ ID NO:37. **(B)** Airway compliance (Crs) in response to MCh-provocation (measured by FlexiVent^{®}) in mice challenged with HDM in the absence or presence of treatment with a polypeptide of SEQID NO:37. Data represent means ± SEM, each group representing n=10-12 animals.
**Figure 7** represents an overview of the HDM sensitisation and challenge protocol.
**Figure 8** represents the optical density at 650 nm upon the addition of the peptide of SEQ ID NO: 1 to commercial engineered human embryonic kidney (HEK-293) TGF-β reporter cells as described in Example 3.

### Detailed description

The term "allergic disorder or disease" refers to allergic settings and hypersensitivity to allergens such as allergen-induced or atopic asthma, atopic dermatitis, atopic rhinitis, allergic conjunctivitis, food allergy, occupational allergy, allergic broncho-pulmonal aspergillosis and eosinophilic esophagitis. The term "allergic disorders" can also refer to any disease displaying symptoms and biomarkers that are hallmarks of allergic disease, including high levels of antigen-specific IgE serum levels,

The term "type-2 inflammatory disorders or diseases" refers to diseases or disorders in which an erroneous adaptive immune response with differentiated T helper cells driving eosinophil recruitment and immunoglobulin production via the secretion of a distinct repertoire of T_{H}2 cytokines that include IL-4, IL-5, and IL-13 and/or secretion of these cytokines by some populations of innate lymphoid cells (ILCs) is caused by a variety of external stimuli, including numerous allergens.

The term "asthma" refers to a disorder of the airways characterized by airway inflammation, hyper-responsiveness, and obstruction which causes spasms of the bronchial smooth muscle system and affects both the upper and lower respiratory tracts. There are several forms of asthma phenotypes (clinical presentations), characterized by varying degrees of severity. Mild asthma is currently defined as asthma that is well controlled with low-intensity treatment, as needed low dose ICS-formoterol, or low dose ICS plus as needed SABA while moderate asthma is currently defined as athma that is well controlled with low or medium dose ICS-LABA in either treatment track while severe asthma is defined as asthma that remains uncontrolled despite optimized treatment with high dose ICS-LABA, or that requires high dose ICS-LABA to prevent it from becoming uncontrolled. A condition known as status asthmaticus is the most severe form of asthma, and generally requires intensive hospital care, and may even prove fatal. The disease may occur as a result of both allergic and non-allergic mechanisms.

The term "allergen induced asthma", "atopic asthma", or "eosinophilic asthma" refers to asthma endotypes (underlying mechanisms) resulting from a hypersensitivity to an antigen/allergen. This includes, but is not limited to, all inhalable airborne allergens including pollen from trees, grass, or weeds or other group of allergens such as house dust mite, animal dander, cockroach, molds and other fungi. Furthermore, occupational allergens, such as flour, soy, latex and different mites (*Tyrophagus putrescentiae*; *Lepidoglyphus destructor; Acarus siro*) are included. Hypersensitivity to allergens, in particular antigen/allergen-induced asthma is usually diagnosed on the basis of the pattern of symptoms such as coughing, sneezing, irritation/itching of the nose or eyes, increased lacrimation and running nose, itching of the skin with formation of an eczema as well as nausea, vomiting, abdominal pain and discomfort and diarrhea in food allergy. Atopic asthma is clinically classified according to the frequency of symptoms, decreased forced expiratory volume in one second (FEV1), or peak expiratory flow rate (Peak Flow), increased Peak Flow variability, airway hyper-responsiveness and increased levels of allergen specific IgE.

The term "atopic dermatitis" (AD) refers to a chronic inflammatory skin disease characterized by an impaired skin barrier, the upregulation of type 2 immune responses and increased *Staphylococcus aureus* colonization. Patients have dry skin, eczematous lesions and intense itching leading to excoriation and lichenification.

The term "allergic rhinitis" (AR) refers to an inflammatory condition of the nasal cavity caused by an IgE-mediated type 1 hypersensitivity reaction to a specific external airborne allergen, characterized by 4 symptoms: watery rhinorrhea, nasal congestion, itching, and sneezing.

The term "chronic rhinosinusitis with nasal polyps" (CRSwNP) refers to a condition in which patients display anterior or posterior rhinorrhea, nasal congestion, hyposmia and/or facial pressure or pain lasting for greater than 12 weeks duration. This disease is generally categorized by a type-2 inflammatory response with enhanced tissue eosinophilia. Levels of eosinophilic granule proteins (e.g., ECP, IL-5, Eotaxin-1, Eotaxin-2, Eotaxin-3, MCP-4) are all elevated in CRSwNP compared to healthy controls. Studies have also reported CRSwNP patients to have increased numbers of basophils, innate type-2 lymphoid cells and mast cells. Additionally, type-2 cytokines including IL-5 and IL-13 as well as the epithelial cell-derived thymic stromal lymphopoietin (TSLP) are often elevated in CRSwNP.

The term "food allergy" (FA) refers to an immune system reaction that occurs soon after eating a certain food (and includes both the "immediate" and "delayed" versions). Even a tiny amount of the allergy-causing food can trigger signs and symptoms such as digestive problems, hives, or swollen airways. In some people, a FA can cause severe symptoms or even a life-threatening reaction known as anaphylaxis.

The term "eosinophilic gastrointestinal disorders", which includes "eosinophilic esophagitis" (EoE) refers to a gastrointestinal inflammatory entity that cause chronic eosinophilic inflammation and dysfunction in the esophagus based on excessive Th2-dominant immunological responses to dietary antigens. EoE has been widely recognized as a major cause of dysphagia and food impaction in adolescents and adults.

The term "chronic urticaria", which includes both the inducible and spontaneous forms, and refers to a type-2 chronic inflammatory condition characterized by the recurrent occurrence of urticaria and/or angioedema. Chronic urticaria, defined by symptoms persisting more than six weeks, accounts for fewer than 5% of all cases of urticaria (Jafilan et. al., 2015, Primary Care. 42 (4): 473-83). Urticaria (also called "hives") is a type of skin rash with red, raised, itchy bumps, which can burn or sting. The patches of rash may appear on different body parts, with variable duration from minutes to days, and do not leave any long-lasting skin change. Chronic urticaria involves allergic, autoimmune, or physico-chemical mechanisms, while acute urticaria frequently occurs following an infection or as a result of an allergic reaction. Risk factors include having conditions such as hay fever or asthma. Diagnosis is typically based on appearance and patch testing may be useful to determine the allergen.

"The term auto-immune disease(s)" refers to diseases characterized by a pathologic response to self-antigens. Autoimmunity or autoreactivity underlie a wide range of clinical disorders. These disorders may be generalized, such as systemic rheumatic diseases like systemic lupus erythematosus, systemic sclerosis, Sjogren syndrome, vasculitis, and others, or organ-specific, such as endocrine and neurologic disorders, including autoimmune thyroiditis and multiple sclerosis, respectively, and other conditions. Autoimmune diseases can be either acute or chronic and can affect essentially all organ.

The term "fibrotic diseases" refers to a wide spectrum of clinical entities including systemic fibrotic diseases such as systemic sclerosis, sclerodermatous graft vs. host disease, as well as numerous organ-specific disorders including radiation-induced fibrosis and cardiac, pulmonary, liver, and kidney fibrosis. These diseases share the common feature of an uncontrolled and progressive accumulation of fibrotic tissue in affected organs causing their dysfunction and ultimate failure.

The term "fibrosis" refers to an excessive deposition of connective tissue components that can affect virtually every organ system, including the skin, lungs, liver and kidney. Fibrotic tissue remodeling often leads to organ dysfunction and is commonly associated with high morbidity and mortality.

The term "inflammatory bowel disease (IBD) refers to a group of inflammatory conditions of the colon and small intestine. The main forms of IBD are Crohn's disease and ulcerative colitis (UC). The main difference between Crohn's disease and UC is the location and nature of the inflammatory changes. Crohn's disease can affect any part of the gastrointestinal tract, from mouth to anus, although a majority of the cases start in the terminal ileum. Ulcerative colitis, in contrast, is restricted to the colon and the rectum. Both disorders may present with any of the following symptoms: abdominal pain, vomiting, diarrhea, rectal bleeding, severe internal cramps/muscle spasms in the region of the pelvis, and weight loss. Anaemia is the most prevalent extra-intestinal complication of inflammatory bowel disease.

The term "anti-allergic properties" refers to the properties of a molecule or an agent in reducing an allergic response.

As used herein, the term "polypeptide" is used in its conventional meaning, i.e., as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof Particular polypeptides of interest in the context of this invention are amino acid subsequences comprising tolerogenic fragments.

The term "fragment" refers to a polypeptide comprising a portion of peptide sequence corresponding to contiguous amino acids of a polypeptide set forth herein, including all intermediate lengths and variants thereof. In this case, a fragment is defined as a portion of polypeptide capable alone of forming one or more stable secondary structure elements, generally containing more than 20 amino acids. Larger fragments (> 50 amino acids) can also form stable tertiary structural elements and are included in this definition. Fragments of a polypeptide can also include domain substructures of the full-length polypeptide, and these fragments tend to be composed of more amino acids than those composing stable tertiary structural elements (100 to 550 amino acids).

By "tolerogenic fragment" is meant a fragment that can induce tolerance to antigens/allergens described in the present application. In certain embodiments, a tolerogenic fragment can induce tolerance to antigens/allergens at least as well as the full-length polypeptide can and in certain embodiments may be more effective than the full-length polypeptide at inducing tolerance. However, in certain embodiments, a tolerogenic fragment induces tolerance to antigens/allergens but may not induce tolerance as effectively as the full-length polypeptide. Such tolerogenic fragments may still be useful in the present invention particularly wherein said tolerogenic fragments have other advantageous properties, such as, but not limited to, ease of preparation or purification as compared to the full-length polypeptide. As would be recognized by the skilled person, a variety of known assays can be used to assess induction of tolerance, including measuring delayed-type hypersensitivity (DTH) responses, measuring cytokine productions by ELISA or other methods, T cell proliferation or cytotoxicity assays, B cell proliferation assays, antibody production, and the like. Such assays are known in the art and are described, for example, in Current Protocols in Immunology, or Current Protocols in Molecular Biology.

The term "variant" applies to both a polypeptide and an amino acid. A polypeptide "variant," as the term is used herein, is a peptide or a polypeptide substantially homologous to the referenced peptide sequence, but which has an amino acid sequence different from that of the referenced. Such variants may be synthetically generated, for example, by modifying one or more of the above polypeptide sequences of the invention described herein using any of a number of techniques well known in the art. Substantially homologous means a variant amino acid sequence which is identical to the referenced peptide sequence with the exception of the deletion, insertion and/or substitution of a few amino acids, e.g. 1, 2, 3, 4, or 5 amino acids amongst the defined non-variable amino acids of the referenced polypeptide sequences. The identity or homology of two amino acid sequences or of two nucleic acid sequences can be determined by visual inspection and/or mathematical calculation, or more easily by comparing sequence information using known computer program used for sequence comparison such as Clustal package version 2.1.

A conservative, or homologous, variant may comprise a sequence having at least one conservatively substituted amino acid.

A "conservative substitution" or "homologous variant" is one in which an amino acid is substituted for another amino acid that has similar properties, such that one skilled in the art of peptide chemistry would expect the secondary structure and hydropathic nature of the polypeptide to be substantially unchanged (e.g., having similar physiochemical characteristics). Modifications may be made in the structure of the polynucleotides and polypeptides of the present invention and still obtain a functional molecule that encodes a variant or derivative polypeptide with desirable characteristics according to the invention. When it is desired to alter the amino acid sequence of a polypeptide to create an equivalent, or even an improved, variant or portion of a polypeptide of the invention, one skilled in the art will typically change one or more of the codons of the encoding DNA sequence. In making such changes, the hydropathic index, polarity, charge, solubility, hydrophobicity, hydrophilicity and/or the amphipathic nature of the amino acids are considered. The importance of the hydropathic amino acid index in conferring interactive biologic function on a protein is generally understood in the art (Kyte, et al., 1982, J. MoI. Biol., 157: 105- 131). Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known (*Kyte, et al, 1982, supra*). For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired. Exemplary amino acid substitutions are presented in **Table 1** below. The term "variant" also includes a peptide or polypeptide substantially homologous to the referenced peptide sequence, but which has an amino acid sequence different from that of the referenced sequence because one, two, three, four or five amino acids amongst the defined non-variable amino acids of the reference polypeptide sequences have been chemically modified or substituted by amino acids analogues. This term also includes glycosylated polypeptides or other residues that have been post-translationally modified, in which case, the number of post-translationally modified amino acids is unlimited.

**Table 1**

| **Original residues** | **Examples of substitutions** |
|---|---|
| Ala (A) | Val, Leu, Ile |
| Arg (R) | Lys, Gln, Asn |
| Asn (N) | Gln |
| Asp (D) | Glu |
| Cys (C) | Ser, Ala |
| Gln (Q) | Asn |
| Glu (E) | Asp |
| Gly (G) | Pro, Ala |
| His (H) | Asn, Gln, Lys, Arg |
| Ile (I) | Leu, Val, Met, Ala, Phe |
| Leu (L) | Ile, Val, Met, Ala, Phe |
| Lys (K) | Arg, Gln, Asn |
| Met (M) | Leu, Ile, Phe |
| Phe (F) | Leu, Val, Ile, Ala, Tyr |
| Pro (P) | Ala, Gly |
| Ser (S) | Thr, Ala, Cys |
| Trp (W) | Phe, Tyr |
| Thr (T) | Ser |
| Tyr (Y) | Trp, Phe, Thr, Ser |
| Val (V) | Ile, Met, Leu, Phe, Ala |

Polypeptides of the invention and variants thereof are capable of inducing tolerance to an antigen/allergen as measured in cellular assays or when administered *in vivo*.

Polypeptides of the invention are prepared using any of a variety of well-known synthetic and/or recombinant techniques, the latter of which are further described below.

The term "pharmaceutically acceptable" refers to a carrier comprised of a material that is not biologically or otherwise undesirable.

The term "carrier" refers to any components present in a pharmaceutical formulation other than the active agent and thus includes diluents, binders, lubricants, disintegrants, fillers, coloring agents, wetting, or emulsifying agents, pH buffering agents, preservatives and the like.

As used herein, "treatment" and "treating" and the like generally mean obtaining a desired pharmacological and physiological effect. The effect may be prophylactic in terms of preventing or partially preventing a disease, symptom, or condition thereof and/or may be therapeutic in terms of a partial or complete cure of a disease, condition, symptom, or adverse effect attributed to the disease. The term "treatment" as used herein covers any treatment of a disease in a mammal, particularly a human, and isnot necessarily meant to imply cure or complete abolition of symptoms, but refers to any type of treatment that imparts a benefit to a patient and includes: (a) preventing the disease from occurring in a subject which may be predisposed to the disease but has not yet been diagnosed as having it for example based on familial history, overweight status or age; (b) inhibiting the disease, i.e., arresting its development; or relieving the disease, i.e., causing regression of the disease and/or its symptoms or conditions such as improvement or remediation of damage.

In particular, prevention and/or treatment of allergic disorders according to the invention comprises normalization or decrease of the antigen/allergen sensitivity of an individual. The term "treatment" refers to any type of treatment or prevention that imparts a benefit to a subject afflicted with or at risk of developing a hypersensitivity immune response to an allergen/allergen of interest, including improvement in the condition of the subject (e.g., in one or more symptoms), delay in the onset of symptoms or slowing the progression of symptoms, etc.

According to one aspect, effects of a treatment according to the invention may be observed through one or more the following: prevention or reduction of the airway hyper-responsiveness, prevention or reduction of cell penetration into bronchial tubes (typically through the functional mechanisms for inhibiting production of IL-4, which is a cytokine secreted by Th2 cells and involved in inflammatory mechanisms of allergic reaction) prevention or reduction of pulmonary inflammation, of bronchoalveolar eosinophilia, of goblet cell metaplasia, of mucus production and Th2 cytokine production that are hallmarks of allergen-induced asthma. Treatment success may also be evident by the observation of the generation of IL-10 in regulatory lymphocytes or other cells or in total lungs (BALF, sputum) or serum, which can be assessed by ELISA or multiplex techniques.

The term "high-risk" subjects or individuals are subjects that are at risk to develop hypersensitivity to allergens/antigens, in particular of developing atopic or allergen-induced asthma. Those include genetic predisposition such as a family history of atopic diseases in close relatives, smoking mother during pregnancy, and smoking environment after birth, viral respiratory infections, such as by respiratory syncytial virus and rhinovirus and occupational exposure to known occupational allergens (e.g. flour). The risk or predisposition of developing hypersensitivity to allergens/antigens, in particular of developing atopic or allergen-induced asthma can be assessed by recording complete history including family history of the patient, skin prick testing, assessment of serum IgE, specific serum IgE levels and measurement of airway hyperreactivity. The term "subject" as used herein refers to mammals. For example, mammals contemplated by the present invention include human, primates, domesticated animals such as cattle, sheep, pigs, horses, laboratory rodents and the like.

The term "efficacy" of a treatment or method according to the invention can be measured based on changes in the course of disease or condition in response to a use or a method according to the invention. For example, the efficacy of a treatment or method according to the invention can be measured by measuring the level of tolerance of the subject before and after the treatment, for example as described below.

The term "tolerance" as referred herein is defined as immune unresponsiveness to an antigen/allergen, usually an antigen/allergen implicated in causing disease. Although tolerance may be induced by administering antigens/allergens by different routes, oral tolerance refers to the oral administration of the composition, which results in inducing tolerance to an antigen/allergen when administered in vivo. The induction of tolerance can therefore be monitored by various techniques including measuring the response to allergen in skin prick test, assessment of allergen specific IgE and assessment of specific T cell responses for the allergen (proliferation and cytokine production).

The term "tolerogenic effective amount" as used herein refers to an amount of at least one polypeptide or a variant or a pharmaceutical formulation thereof according to the invention that elicits a detectable tolerogenic response in a subject that that is being administered the said polypeptide.

As used herein, the term "antigen" refers to a foreign substance that that when introduced into the body triggers an immune system response, resulting in production of an antibody as part of the body's defense against disease.

The term "allergen" is meant to designate an antigen capable of eliciting a hypersensitivity immune response (such as described herein) in an individual, such as in an animal, such as in a human. The allergen may be a sensitizing allergen or a cross-reacting allergen.

The polypeptides of the invention and formulations thereof have immunomodulatory properties that can be useful for tolerization strategies such as in allergic disorders and in particular allergic asthma. The polypeptides of the invention and formulations thereof can be useful in particular in tolerization treatments for asthma prevention in high-risk individuals.

### Peptides

According to one embodiment, is provided a polypeptide of the invention having a sequence selected from SEQ ID NO: 1 and SEQ ID NO: 2 or a homologous variant or a fragment thereof.

According to a particular embodiment, Insert X₁, when present, is the sequence **NTPNDPIHSESR** (SEQ ID NO: 20).

According to another embodiment, X₂ is Threonine or a homologous variant thereof.

According to another embodiment, X₃ is Threonine or a homologous variant thereof.

According to another embodiment, X₄ is Alanine, or a homologous variant thereof.

According to another embodiment, X₅ is Serine or a homologous variant thereof.

According to another embodiment, X₆ is Tryptophan or a homologous variant thereof.

According to another embodiment, X₇ is Glycine or a homologous variant thereof.

According to another embodiment, X₈ is Glutamine or a homologous variant thereof.

According to another embodiment, X₉ is Alanine or a homologous variant thereof.

According to another embodiment, X₁₀ is Glutamate, or a homologous variant thereof.

According to another embodiment, X₁₁ is Threonine or a homologous variant thereof.

According to another embodiment, X₁₂ is Proline or a homologous variant thereof.

According to another embodiment, X₁₃ is Lysine, or a homologous variant thereof.

According to another embodiment, X₁₄ is Aspartate, or a homologous variant thereof.

According to another embodiment, X₁₅ is Tryptophan, or a homologous variant thereof.

According to another embodiment, X₁₆ is Arginine, or a homologous variant thereof.

According to another embodiment, X₁₇ is Alanine or a homologous variant thereof.

According to another embodiment, X₁₈ is Glycine or a homologous variant thereof.

According to another embodiment, X₁₉ is Lysine or a homologous variant thereof.

According to another embodiment, X₂₀ is Asparagine, or a homologous variant thereof.

According to another embodiment, X₂₁ is Glutamate or a homologous variant thereof.

According to another embodiment, X₂₂ is Proline or a homologous variant thereof.

According to another embodiment, X₂₃ is Asparagine or a homologous variant thereof.

According to another embodiment, X₂₄ is Lysine or a homologous variant thereof.

According to another embodiment, X₂₅ is Glutamate or a homologous variant thereof.

According to another embodiment, X₂₆ is Aspartate or a homologous variant thereof.

According to another embodiment, X₂₇ is Lysine or a homologous variant thereof.

According to another embodiment, X₂₈ is Serine or a homologous variant thereof.

According to another embodiment, X₃₀ is Aspartate or a homologous variant thereof.

According to another embodiment, X₃₁ is Glycine or a homologous variant thereof.

According to another embodiment, X₃₂ is Tryptophan or a homologous variant thereof.

According to another embodiment, X₃₃ is Alanine or a homologous variant thereof.

According to another embodiment, X₃₄ is Alanine or a homologous variant thereof.

According to another embodiment, X₃₅ is Arginine or a homologous variant thereof.

According to another embodiment, X₃₆ is Glycine or a homologous variant thereof.

According to another embodiment, X₃₇ is Tryptophan or a homologous variant thereof.

According to another embodiment, X₃₈ is Valine or a homologous variant thereof.

According to another embodiment, X₃₉ is Aspartate or a homologous variant thereof.

According to another embodiment, X₄₀ is Lysine or a homologous variant thereof.

According to another embodiment, X₄₁ is Aspartate or a homologous variant thereof.

According to another embodiment, X₄₂ is Alanine or a homologous variant thereof.

According to another embodiment, X₄₃ is Threonine or a homologous variant thereof.

According to another embodiment, X₄₄ is Lysine or a homologous variant thereof.

According to another embodiment, X₄₅ is Arginine or a homologous variant thereof.

According to another embodiment, X₄₆ is Aspartate or a homologous variant thereof.

According to another embodiment, X₄₇ is Valine or a homologous variant thereof.

According to another embodiment, X₄₈ is Arginine or a homologous variant thereof.

According to another embodiment, X₄₉ is Aspartate or a homologous variant thereof.

According to another embodiment, X₅₀ is Serine or a homologous variant thereof.

According to another embodiment, X₅₁ is Alanine or a homologous variant thereof.

According to another embodiment, X₅₂ is Aspartate or a homologous variant thereof.

According to another embodiment, X₅₃ is Aspartate or a homologous variant thereof.

According to another embodiment, X₅₄ is Asparagine or a homologous variant thereof.

According to another embodiment, X₅₅ is Lysine or a homologous variant thereof.

According to another embodiment, X₅₆ is Serine or a homologous variant thereof.

According to another embodiment, X₅₇ is Isoleucine or a homologous variant thereof.

According to another embodiment, X₅₈ is Aspartate or a homologous variant thereof.

According to another embodiment, X₅₉ is Alanine or a homologous variant thereof.

According to another embodiment, X₆₀ is Arginine or a homologous variant thereof.

According to another embodiment, X₆₁ is Serine or a homologous variant thereof.

According to another embodiment, X₆₂ is Glutamine or a homologous variant thereof.

According to another embodiment, X₆₃ is Alanine or a homologous variant thereof.

According to another embodiment, X₆₄ is Serine or a homologous variant thereof.

According to another embodiment, X₆₅ is Glutamate or a homologous variant thereof.

According to another embodiment, X₆₆ is Glycine or a homologous variant thereof.

According to another embodiment, X₆₇ is Threonine, or a homologous variant thereof.

According to another embodiment, X₆₈ is Serine or a homologous variant thereof.

According to another embodiment, X₆₉ is Serine or a homologous variant thereof.

According to another embodiment, X₇₀ is Lysine or a homologous variant thereof.

According to another embodiment, X₇₁ is Alanine or a homologous variant thereof.

According to another embodiment, X₇₂ is Alanine or a homologous variant thereof.

According to another embodiment, X₇₃ is Threonine or a homologous variant thereof.

According to another embodiment, X₇₄ is Asparagine, or a homologous variant thereof.

According to another embodiment, X₇₅ is Alanine or a homologous variant thereof.

According to another embodiment, X₇₆ is Serine, or a homologous variant thereof.

According to another embodiment, X₇₇ is Asparagine or a homologous variant thereof.

According to one embodiment, Insert X₇₈ is present.

According to another embodiment, Insert X₇₈ is absent.

According to another embodiment, X₇₉ is Lysine or a homologous variant thereof.

According to another embodiment, X₈₀ is Methionine or a homologous variant thereof.

According to another embodiment, X₈₁ is Asparagine or a homologous variant thereof.

According to another embodiment, X₈₂ is Tryptophan or a homologous variant thereof.

According to another embodiment, X₈₃ is Arginine or a homologous variant thereof.

According to another embodiment, X₈₄ is Leucine or a homologous variant thereof.

According to another embodiment, X₈₅ is Proline or a homologous variant thereof.

According to another embodiment, X₈₆ is Serine or a homologous variant thereof.

According to another embodiment, X₈₇ is Tyrosine or a homologous variant thereof.

According to another embodiment, X₈₈ is Asparagine, or a homologous variant thereof.

According to another embodiment, X₈₉ is Threonine, or a homologous variant thereof.

According to another embodiment, X₉₀ is Serine, or a homologous variant thereof.

According to another embodiment, X₉₁ is Threonine, or a homologous variant thereof.

According to another embodiment, X₉₂ is Glutamate or a homologous variant thereof.

According to another embodiment, X₉₃ is Valine, or a homologous variant thereof.

According to another embodiment, X₉₄ is Asparagine or a homologous variant thereof.

According to another embodiment, X₉₅ is Asparagine or a homologous variant thereof.

According to another embodiment, X₉₆ is Threonine or a homologous variant thereof.

According to another embodiment, X₉₇ is Valine or a homologous variant thereof.

According to another embodiment, X₉₈ is Aspartate or a homologous variant thereof.

According to another embodiment, X₉₉ is Lysine or a homologous variant thereof.

According to another embodiment, X₁₀₀ is Alanine or a homologous variant thereof.

According to another embodiment, X₁₀₁ is Glutamine or a homologous variant thereof.

According to another embodiment, X₁₀₂ is Alanine or a homologous variant thereof.

According to another embodiment, X₁₀₃ is Alanine or a homologous variant thereof.

According to another embodiment, X₁₀₄ is Serine or a homologous variant thereof.

According to another embodiment, X₁₀₅ is Asparagine or a homologous variant thereof.

According to another embodiment, X₁₀₆ is Lysine or a homologous variant thereof.

According to another embodiment, X₁₀₇ is Threonine or a homologous variant thereof.

According to another embodiment, X₁₀₈ is Histidine or a homologous variant thereof.

According to another embodiment, X₁₀₉ is Glycine or a homologous variant thereof.

According to another embodiment, X₁₁₀ is Threonine or a homologous variant thereof.

According to another embodiment X₁₁₁ is Aspartate or a homologous variant thereof.

According to another embodiment, X₁₁₂ is Serine or a homologous variant thereof.

According to another embodiment, X₁₁₃ is Alanine or a homologous variant thereof.

According to another embodiment, X₁₁₄ is Asparagine or a homologous variant thereof.

According to another embodiment, X₁₁₅ is Alanine or a homologous variant thereof.

According to another embodiment, X₁₁₆ is Proline or a homologous variant thereof.

According to another embodiment, X₁₁₈ is Glycine or a homologous variant thereof.

According to another embodiment, X₁₁₉ is Lysine or a homologous variant thereof.

According to another embodiment, X₁₂₀ is Aspartate or a homologous variant thereof.

According to another embodiment, X₁₂₁ is Valine or a homologous variant thereof.

According to another embodiment, X₁₂₂ is Asparagine or a homologous variant thereof.

According to another embodiment, X₁₂₃ is Asparagine or a homologous variant thereof.

According to another embodiment, X₁₂₄ is Arginine or a homologous variant thereof.

According to another embodiment, X₁₂₅ is Asparagine or a homologous variant thereof.

According to another embodiment, X₁₂₆ is Asparagine or a homologous variant thereof.

According to another embodiment, X₁₂₇ is Alanine or a homologous variant thereof.

According to another embodiment, X₁₂₈ is Lysine or a homologous variant thereof.

According to another embodiment, X₁₂₉ is Serine or a homologous variant thereof.

According to another embodiment, X₁₃₀ is Histidine or a homologous variant thereof.

According to another embodiment, X₁₃₁ is Leucine or a homologous variant thereof.

According to another embodiment, X₁₃₂ is Asparagine or a homologous variant thereof.

According to another embodiment, X₁₃₃ is Lysine or a homologous variant thereof.

According to another embodiment, X₁₃₄ is Glycine or a homologous variant thereof.

According to another embodiment, X₁₃₅ is Glycine or a homologous variant thereof.

According to another embodiment, X₁₃₆ is Glutamine, or a homologous variant thereof.

According to another embodiment, X₁₃₇ is Alanine, or a homologous variant thereof.

According to another embodiment, X₁₃₈ is Arginine or a homologous variant thereof.

According to another embodiment, X₁₃₉ is Serine or a homologous variant thereof.

According to another embodiment, X₁₄₀ is Leucine or a homologous variant thereof.

According to another embodiment, X₁₄₁ is Asparagine, or a homologous variant thereof.

According to another embodiment, X₁₄₂ is Aspartate, or a homologous variant thereof.

According to another embodiment, X₁₄₃ is Proline, or a homologous variant thereof.

According to another embodiment, X₁₄₄ is Arginine or a homologous variant thereof.

According to another embodiment, X₁₄₅ is Asparagine or a homologous variant thereof.

According to another embodiment, X₁₄₅ is Asparagine or a homologous variant thereof.

According to another embodiment, X₁₄₆ is Valine or a homologous variant thereof.

According to another embodiment, X₁₄₇ is Glycine or a homologous variant thereof.

According to another embodiment, X₁₄₈ is Tyrosine or a homologous variant thereof.

According to another embodiment, X₁₄₉ is Alanine, or a homologous variant thereof.

According to another embodiment, X₁₅₀ is Alanine or a homologous variant thereof.

According to another embodiment, X₁₅₁ is Serine or a homologous variant thereof.

According to another embodiment, X₁₅₂ is Alanine or a homologous variant thereof.

According to another embodiment, X₁₅₃ is Asparagine or a homologous variant thereof.

According to another embodiment, X₁₅₄ is Valine or a homologous variant thereof.

According to another embodiment, X₁₅₅ is Aspartate, or a homologous variant thereof.

According to another embodiment, X₁₅₆ is Lysine or a homologous variant thereof.

According to another embodiment, X₁₅₇ is Asparagine or a homologous variant thereof.

According to another embodiment, X₁₅₈ is Glycine or a homologous variant thereof.

According to another embodiment, X₁₅₉ is Threonine or a homologous variant thereof.

According to another embodiment, X₁₆₀ is Alanine or a homologous variant thereof.

According to another embodiment, X₁₆₁ is Threonine or a homologous variant thereof.

According to another embodiment, X₁₆₂ is Asparagine or a homologous variant thereof.

According to another embodiment, X₁₆₃ is Aspartate or a homologous variant thereof.

According to another embodiment, X₁₆₄ is Serine or a homologous variant thereof.

According to another embodiment, X₁₆₅ is Glycine or a homologous variant thereof.

According to another embodiment, X₁₆₆ is Phenylalanine or a homologous variant thereof.

According to another embodiment, X₁₆₇ is Valine or a homologous variant thereof.

According to another embodiment, X₁₆₈ is Lysine or a homologous variant thereof.

According to another embodiment, X₁₆₉ is Valine or a homologous variant thereof.

According to another embodiment, X₁₇₀ is Aspartate or a homologous variant thereof.

According to another embodiment, X₁₇₁ is Alanine or a homologous variant thereof.

According to another embodiment, X₁₇₂ is Histidine or a homologous variant thereof.

According to another embodiment, Insert X₁₇₃, when present, is of SEQ ID NO:6 or a homologous variant thereof.

According to one embodiment, polypeptides of the invention are of SEQ ID NO:2 wherein Insert X₁₇₃ is the following sequence:
**AYFNGNIYLGKSTNLRVNGHS** (SEQ ID NO:23).

According to one embodiment, Insert X₁₇₃ is absent.

According to one embodiment, X₁₇₄ is Alanine or a homologous variant thereof.

According to one embodiment, X₁₇₅ is Asparagine or a homologous variant thereof.

According to one embodiment, X₁₇₆ is Phenylalanine or a homologous variant thereof.

According to one embodiment, X₁₇₇ is Lysine or a variant thereof.

According to one embodiment, X₁₇₈ is Glycine or a variant thereof.

According to one embodiment, X₁₇₉ is Isoleucine or a homologous variant thereof.

According to one embodiment, X₁₈₀ is Aspartate or a homologous variant thereof.

According to one embodiment, X₁₈₁ is Threonine or a homologous variant thereof.

According to one embodiment, Insert X₁₈₂ is present.

According to one embodiment, Insert X₁₈₂ is absent.

According to one embodiment, X₁₈₃ is Glycine or a homologous variant thereof.

According to one embodiment, X₁₈₄ is Asparagine or a homologous variant thereof.

According to one embodiment, X₁₈₅ is Glycine or a homologous variant thereof.

According to one embodiment, X₁₈₆ is Asparagine or a homologous variant thereof.

According to one embodiment, Insert X₁₈₇ is present.

According to one embodiment, Insert X₁₈₇ is absent.

According to one embodiment, X₁₈₇ is an Insert with the following sequence: **TS.**

According to one embodiment, X₁₈₈ is Threonine or a homologous variant thereof.

According to one embodiment, X₁₈₉ is Aspartate or a homologous variant thereof.

According to one embodiment, X₁₉₀ is Serine or a homologous variant thereof.

According to one embodiment, X₁₉₁ is Glycine or a homologous variant thereof.

According to one embodiment, X₁₉₂ is Asparagine or a homologous variant thereof.

According to one embodiment, X₁₉₃ is Asparagine or a homologous variant thereof.

According to one embodiment, X₁₉₄ is Asparagine or a homologous variant thereof.

According to one embodiment, X₁₉₅ is Lysine or a homologous variant thereof.

According to one embodiment, X₁₉₆ is Isoleucine or a homologous variant thereof.

According to another embodiment, X₁₉₇ is Alanine, or a homologous variant thereof.

According to another embodiment, X₁₉₈ is Serine or a homologous variant thereof.

According to another embodiment, X₂₀₀ is Asparagine or a homologous variant thereof.

According to another embodiment, X₂₀₁ is Phenylalanine or a homologous variant thereof.

According to another embodiment, X₂₀₂ is Asparagine or a homologous variant thereof.

According to another embodiment, X₂₀₃ is Asparagine or a homologous variant thereof.

According to another embodiment, X₂₀₄ is Glutamate or a homologous variant thereof.

According to another embodiment, X₂₀₅ is Lysine or a homologous variant thereof.

According to another embodiment, X₂₀₆ is Asparagine or a homologous variant thereof.

According to another embodiment, X₂₀₇ is Glycine or a homologous variant thereof.

According to another embodiment, X₂₀₈ is Isoleucine or a homologous variant thereof.

According to another embodiment, X₂₀₉ is Serine or a homologous variant thereof.

According to another embodiment, X₂₁₀ is Glutamine or a homologous variant thereof.

According to another embodiment, X₂₁₁ is Histidine or a homologous variant thereof.

According to another embodiment, X₂₁₂ is Serine or a homologous variant thereof.

According to another embodiment, X₂₁₃ is Glutamate or a homologous variant thereof.

According to another embodiment, X₂₁₄ is Aspartate or a homologous variant thereof.

According to another embodiment, X₂₁₅ is Serine or a homologous variant thereof.

According to another embodiment, X₂₁₆ is Glutamine or a homologous variant thereof.

According to another embodiment, X₂₁₇ is Arginine or a homologous variant thereof.

According to another embodiment, X₂₁₈ is Asparagine or a homologous variant thereof.

According to another embodiment, X₂₁₉ is Threonine or a homologous variant thereof.

According to another embodiment, X₂₂₀ is Arginine-or a homologous variant thereof.

According to another embodiment, X₂₂₁ is Glutamate or a homologous variant thereof.

According to another embodiment, X₂₂₂ is Threonine, or a homologous variant thereof.

According to another embodiment, X₂₂₃ is Threonine or a homologous variant thereof.

According to another embodiment X₂₂₄ is Arginine or a homologous variant thereof.

According to another embodiment, X₂₂₃ is Serine or a homologous variant thereof.

According to another embodiment, X₂₂₆ is Serine, or a homologous variant thereof.

According to another embodiment, X₂₂₇ is Lysine or a homologous variant thereof.

According to another embodiment, X₂₂₈ is Glycine or a homologous variant thereof.

According to another embodiment, X₂₂₉ is Glutamate or a homologous variant thereof.

According to another embodiment, X₂₃₀ is Lysine or a homologous variant thereof.

According to another embodiment X₂₃₁ is Valine or a homologous variant thereof.

According to another embodiment, X₂₃₂ is Asparagine or a homologous variant thereof.

According to another embodiment, X₂₃₃ is Tyrosine or a homologous variant thereof.

According to another embodiment, X₂₃₄ is Tyrosine or a homologous variant thereof.

According to another embodiment, X₂₃₅ is Serine or a homologous variant thereof.

According to another embodiment, X₂₃₆ is Arginine or a homologous variant thereof.

According to another embodiment, X₂₃₇ is Asparagine, or a homologous variant thereof.

According to another embodiment, X₂₃₈ is Lysine or a homologous variant thereof.

According to another embodiment, X₂₃₉ is Asparagine or a homologous variant thereof.

According to another embodiment, X₂₃₉ is Asparagine or a homologous variant thereof.

According to another embodiment, X₂₄₀ is Threonine or a homologous variant thereof.

According to another embodiment, X₂₄₁ is Asparagine or a homologous variant thereof.

According to another embodiment, X₂₄₂ is Alanine or a homologous variant thereof.

According to another embodiment, X₂₄₃ is Serine or a homologous variant thereof.

According to another embodiment, X₂₄₄ is Serine or a homologous variant thereof.

According to another embodiment, X₂₄₅ is Threonine or a homologous variant thereof.

According to another embodiment, X₂₄₆ is Proline or a homologous variant thereof.

According to another embodiment, X₂₄₇ is Glutamate or a homologous variant thereof.

According to another embodiment, X₂₄₈ is Asparagine or a homologous variant thereof.

According to another embodiment, X₂₄₉ is Proline or a homologous variant thereof.

According to another embodiment, X₂₅₀ is Tryptophan or a homologous variant thereof.

According to another embodiment, X₂₅₁ is Threonine or a homologous variant thereof.

According to another embodiment, X₂₅₂ is Serine or a homologous variant thereof.

According to another embodiment, X₂₅₃ is Lysine or a homologous variant thereof.

According to another embodiment, X₂₅₄ is Methionine or a homologous variant thereof.

According to another embodiment, X₂₅₅ is Phenylalanine or a homologous variant thereof.

According to another embodiment, X₂₅₆ is Threonine or a homologous variant thereof.

According to another embodiment, X₂₅₇ is Glycine or a homologous variant thereof.

According to another embodiment, X₂₅₈ is Glutamine or a homologous variant thereof.

According to another embodiment, X₂₅₉ is Valine or a homologous variant thereof.

According to another embodiment, X₂₆₀ is Aspartate or a homologous variant thereof.

According to another embodiment, X₂₆₁ is Serine or a homologous variant thereof.

According to another embodiment, X₂₆₂ is Glutamine or a homologous variant thereof.

According to another embodiment, X₂₆₃ is Aspartate or a homologous variant thereof.

According to another embodiment, X₂₆₄ is Serine or a homologous variant thereof.

According to another embodiment, X₂₆₅ is Asparagine or a homologous variant thereof.

According to another embodiment, X₂₆₆ is Threonine or a homologous variant thereof.

According to another embodiment, X₂₆₇ is Glutamine or a homologous variant thereof.

According to another embodiment, X₂₆₈ is Glycine or a homologous variant thereof.

According to another embodiment, X₂₆₉ is Aspartate or a homologous variant thereof.

According to another embodiment, X₂₇₀ is Isoleucine or a homologous variant thereof.

According to another embodiment, X₂₇₁ is Glutamine, or a homologous variant thereof.

According to another embodiment, X₂₇₂ is Glycine, or a homologous variant thereof.

According to another embodiment, X₂₇₃ is Threonine, or a homologous variant thereof.

According to another embodiment, X₂₇₄ is Isoleucine or a homologous variant thereof.

According to another embodiment, X₂₇₅ is Tyrosine or a homologous variant thereof.

According to another embodiment, X₂₇₆ is Arginine or a homologous variant thereof.

According to another embodiment, X₂₇₇ is Glycine or a homologous variant thereof.

According to another embodiment, X₂₇₈ is Glycine or a homologous variant thereof.

According to another embodiment, X₂₇₉ is Lysine or a homologous variant thereof.

According to another embodiment, X₂₈₀ is Valine or a homologous variant thereof.

According to another embodiment, X₂₈₁ is Alanine or a homologous variant thereof.

According to another embodiment, X₂₈₂ is Threonine or a homologous variant thereof.

According to another embodiment, X₂₈₃ is Asparagine or a homologous variant thereof.

According to another embodiment, X₂₈₄ is Glycine or a homologous variant thereof.

According to another embodiment, X₂₈₅ is Asparagine or a homologous variant thereof.

According to another embodiment, X₂₈₆ is Alanine or a homologous variant thereof.

According to another embodiment, X₂₈₇ is Alanine or a homologous variant thereof.

According to another embodiment, X₂₈₈ is Alanine or a homologous variant thereof.

According to another embodiment, X₂₈₉ is Methionine or a homologous variant thereof.

According to another embodiment, X₂₉₀ is Asparagine or a homologous variant thereof.

According to another embodiment, X₂₉₁ is Aspartate or a homologous variant thereof.

According to another embodiment, X₂₉₂ is Serine or a homologous variant thereof.

According to another embodiment, X₂₉₃ is Alanine or a homologous variant thereof.

According to another embodiment, X₂₉₄ is Lysine or a homologous variant thereof.

According to another embodiment, X₂₉₅ is Leucine or a homologous variant thereof.

According to another embodiment, X₂₉₆ is Lysine or a homologous variant thereof.

According to another embodiment, X₂₉₇ is Asparagine or a homologous variant thereof.

According to another embodiment, X₂₉₈ is Serine or a homologous variant thereof.

According to another embodiment, X₂₉₉ is Aspartate or a homologous variant thereof.

According to another embodiment, X₃₀₀ is Isoleucine or a homologous variant thereof.

According to another embodiment, X₃₀₁ is Lysine or a homologous variant thereof.

According to another embodiment, X₃₀₂ is Lysine or a homologous variant thereof.

According to another embodiment, X₃₀₃ is Glutamate, or a homologous variant thereof.

According to another embodiment, X₃₀₄ is Lysine, or a homologous variant thereof.

According to another embodiment, X₃₀₅ is Lysine, or a homologous variant thereof.

According to another embodiment, X₃₀₆ is Isoleucine or a homologous variant thereof.

According to another embodiment, X₃₀₇ is Glycine or a homologous variant thereof.

According to another embodiment, X₃₀₈ is Glycine or a homologous variant thereof.

According to another embodiment, X₃₀₉ is Asparagine or a homologous variant thereof.

According to one embodiment, polypeptides of the invention are of SEQ ID NO:1 wherein Insert X₃₁₀ is present.

According to one embodiment, polypeptides of the invention are of SEQ ID NO:1 wherein Insert X₃₁₀ is absent.

According to another embodiment, X₃₁₁ is Threonine or a homologous variant thereof.

According to another embodiment, X₃₁₂ is Glycine or a homologous variant thereof.

According to another embodiment, X₃₁₃ is Threonine or a homologous variant thereof.

According to another embodiment, X₃₁₄ is Asparagine or a homologous variant thereof.

According to another embodiment, X₃₁₅ is Serine or a homologous variant thereof.

According to another embodiment, X₃₁₆ is Isoleucine or a homologous variant thereof.

According to another embodiment, X₃₁₇ is Serine or a homologous variant thereof.

According to another embodiment, X₃₁₈ is Asparagine or a homologous variant thereof.

According to another embodiment, X₃₁₉ is Valine or a homologous variant thereof.

According to one embodiment, X₃₂₀ is Asparagine or a homologous variant thereof.

According to one embodiment, X₃₂₁ is Leucine or a homologous variant thereof.

According to one embodiment, X₃₂₂ is Glutamate or a homologous variant thereof.

According to one embodiment, X₃₂₃ is Glutamine or a homologous variant thereof.

According to one embodiment, X₃₂₄ is Phenylalanine or a homologous variant thereof.

According to one embodiment, X₃₂₅ is Lysine or a homologous variant thereof.

According to one embodiment, X₃₂₆ is Glutamate or a homologous variant thereof.

According to one embodiment, X₃₂₇ is Asparagine or a homologous variant thereof.

According to one embodiment, X₃₂₈ is Asparagine or a homologous variant thereof.

According to one embodiment, X₃₂₉ is Threonine or a homologous variant thereof.

According to one embodiment, X₃₃₀ is Arginine or a homologous variant thereof.

According to one embodiment, polypeptides of the invention are of SEQ ID NO:1 wherein Insert X₃₃₁ is present.

According to one embodiment, polypeptides of the invention are of SEQ ID NO:1 wherein Insert X₃₃₁ is absent.

According to one embodiment, X₃₃₂ is Asparagine or a homologous variant thereof.

According to one embodiment, X₃₃₃ is Threonine or a homologous variant thereof.

According to one embodiment, X₃₃₄ is Aspartate or a homologous variant thereof.

According to one embodiment, X₃₃₅ is Alanine or a homologous variant thereof.

According to another embodiment, X₃₃₆ is Glycine, or a homologous variant thereof.

According to another embodiment, X₃₃₇ is Methionine or a homologous variant thereof.

According to another embodiment, X₃₃₈ is Asparagine or a homologous variant thereof.

According to another embodiment, X₃₃₉ is Glutamine or a homologous variant thereof.

According to another embodiment, X₃₄₀ is Serine or a homologous variant thereof.

According to another embodiment, X₃₄₁ is Asparagine or a homologous variant thereof.

According to another embodiment, X₃₄₂ is Asparagine or a homologous variant thereof.

According to another embodiment, X₃₄₃ is Aspartate or a homologous variant thereof.

According to another embodiment, X₃₄₄ is Asparagine or a homologous variant thereof.

According to another embodiment, X₃₄₅ is Lysine or a homologous variant thereof.

According to another embodiment, X₃₄₆ is Isoleucine or a homologous variant thereof.

According to another embodiment, X₃₄₇ is Lysine or a homologous variant thereof.

According to another embodiment, X₃₄₈ is Isoleucine or a homologous variant thereof.

According to another embodiment, X₃₄₉ is Glycine or a homologous variant thereof.

According to another embodiment, X₃₅₀ is Serine or a homologous variant thereof.

According to another embodiment, X₃₅₁ is Threonine or a homologous variant thereof.

According to another embodiment, X₃₅₂ is Asparagine or a homologous variant thereof.

According to another embodiment, X₃₅₃ is Glycine or a homologous variant thereof.

According to another embodiment, X₃₅₄ is Lysine or a homologous variant thereof.

According to another embodiment, X₃₅₅ is Serine or a homologous variant thereof.

According to another embodiment, X₃₅₆ is Tyrosine or a homologous variant thereof.

According to another embodiment, X₃₅₇ is Tyrosine or a homologous variant thereof.

According to another embodiment, X₃₅₈ is Leucine or a homologous variant thereof.

According to another embodiment, X₃₅₉ is Glycine or a homologous variant thereof.

According to another embodiment, X₃₆₀ is Asparagine or a homologous variant thereof.

According to another embodiment, X₃₆₁ is Serine or a homologous variant thereof.

According to another embodiment, X₃₆₂ is Threonine or a homologous variant thereof.

According to another embodiment, X₃₆₃ is Proline or a homologous variant thereof.

According to another embodiment, X₃₆₄ is Threonine or a homologous variant thereof.

According to another embodiment, X₃₆₅ is Glutamate or a homologous variant thereof.

According to another embodiment, X₃₆₆ is Asparagine or a homologous variant thereof.

According to another embodiment, X₃₆₇ is Glycine or a homologous variant thereof.

According to another embodiment, X₃₆₈ is Glycine or a homologous variant thereof.

According to another embodiment, X₃₆₉ is Asparagine or a homologous variant thereof.

According to another embodiment, X₃₇₀ is Threonine or a homologous variant thereof.

According to another embodiment, X₃₇₁ is Threonine or a homologous variant thereof.

According to another embodiment, X₃₇₂ is Asparagine or a homologous variant thereof.

According to another embodiment, X₃₇₃ is Leucine or a homologous variant thereof.

According to another embodiment, X₃₇₄ is Proline or a homologous variant thereof.

According to another embodiment, X₃₇₅ is Threonine or a homologous variant thereof.

According to another embodiment, X₃₇₆ is Asparagine or a homologous variant thereof.

According to another embodiment, X₃₇₇ is Threonine or a homologous variant thereof.

According to another embodiment, X₃₇₈ is Threonine or a homologous variant thereof.

According to one embodiment, Insert X₃₇₉ is absent.

According to one embodiment, polypeptides of the invention are of SEQ ID NO:1 wherein Insert X₃₇₉ is the following sequence **NNARFASYA.**

According to one embodiment, polypeptides of the invention are of SEQ ID NO:1 wherein Inserts X₇₈, X₁₇₃, X₁₈₂, X₁₈₇, X₃₁₀, X₃₃₁ and X₃₇₉ are absent.

According to one embodiment, a polypeptide of the invention is a variant of SEQ ID NO:1, in particular a polypeptide of SEQ ID NO: 10.

According to one embodiment, a polypeptide of SEQ ID NO: 1 has a sequence selected from SEQ ID NO: 10-13.

According to one embodiment, a polypeptide of SEQ ID NO: 1 has a sequence of SEQ ID NO: 10.

According to one embodiment, a polypeptide of SEQ ID NO: 1 has a sequence selected from SEQ ID NO: 31-34.

According to one embodiment, a polypeptide of SEQ ID NO: 1 or SEQ ID NO10 has a has a sequence of SEQ ID NO: 31.

According to one embodiment, polypeptides of the invention are of SEQ ID NO:2 wherein Inserts X₇₈, X₁₇₃, X₁₈₂, X₁₈₇, X₃₁₀ and X₃₃₁ are present.

According to one embodiment, polypeptides of the invention are of SEQ ID NO:2 wherein Insert X₁₇₃ is absent.

According to one embodiment, polypeptides of the invention are of SEQ ID NO:2 wherein Insert X₃₇₉ is absent.

According to a particular embodiment, a polypeptide of the invention is a variant of SEQ ID NO: 2, in particular a polypeptide of SEQ ID NO: 14.

According to a further particular embodiment, a polypeptide of the invention of SEQ ID NO:2 has a sequence selected from SEQ ID NO: 14-17 and 19.

According to a further particular embodiment, a polypeptide of the invention of SEQ ID NO:2 has a sequence selected from SEQ ID NO: 37-40 and 40-43.

The sequences are detailed under **Table 2** below and in the sequence listing.

**Table 2**

| **SEQ ID** | **Name** |
|---|---|
| SEQ ID NO: 1 | Consensus s1m1 family |
| SEQ ID NO: 2 | Consensus s2m2 family |
| SEQ ID NO: 3 | Insert X₁ |
| SEQ ID NO: 6 | Insert X₁₇₃ |
| SEQ ID NO: 10 | s1m1 family |
| SEQ ID NO: 11 | s1m1 family with G14 & G18 mutations |
| SEQ ID NO: 12 | s1m1 family lacking Insert X₃₇₉ |
| SEQ ID NO: 13 | s1m1 family with G14A & G18A mutations lacking Insert X₃₇₉ |
| SEQ ID NO: 14 | s2m2 family with G26A and G30A mutations |
| SEQ ID NO: 15 | s2m2 family lacking Insert X₁₇₃ |
| SEQ ID NO: 16 | s2m2 family |
| SEQ ID NO: 17 | s2m2 family lacking Insert X₁₇₃ |
| SEQ ID NO: 19 | s2m2 family lacking Inserts X₁₇₃, and X₃₇₉ |
| SEQ ID NO: 20 | A specific sequence of Insert X₁ |
| SEQ ID NO: 23 | A specific sequence of Insert X₁₇₃ |
| SEQ ID NO: 24 | A specific sequence of Insert X₃₁₀ |
| SEQ ID NO: 25 | A specific sequence of Insert X₃₇₉ |
| SEQ ID NO: 26 | Wild-type reference protein |
| SEQ ID NO: 28 | Comparative sequence, not from the invention |
| SEQ ID NO: 30 | Comparative sequence, not from the invention |
| SEQ ID NO: 31 | A specific polypeptide of SEQ ID NO: 10 |
| SEQ ID NO: 32 | A specific polypeptide of SEQ ID NO: 11 |
| SEQ ID NO: 33 | A specific polypeptide of SEQ ID NO: 12 |
| SEQ ID NO: 34 | A specific peptide of s1m1 family with G14A & G18A mutations |
| SEQ ID NO: 37 | A specific polypeptide of SEQ ID NO: 16 |
| SEQ ID NO: 38 | A specific peptide of s2m2 family lacking Insert X₁₇₃ |
| SEQ ID NO: 40 | A specific polypeptide of SEQ ID NO: 19 |
| SEQ ID NO: 41 | A specific polypeptide of SEQ ID NO:19 containing Insert X₁₇₃ |
| SEQ ID NO: 42 | A specific polypeptide of SEQ ID NO: 14 |
| SEQ ID NO: 43 | A specific polypeptide of SEQ ID NO: 14 containing Insert X₁₇₃ |

The present invention provides polypeptides of the invention, including fragments and variants thereof as defined herein.

The preparation of polypeptide, fragments, and variants thereof according to the invention recombinantly, can be achieved by various techniques known in the art. Nucleic acid sequence encoding for said polypeptide, fragments and variants thereof can be inserted in the recombinant expression vector by methods well known to a person skilled in the art such as, for example, those that are described in Green et al., Molecular cloning: a laboratory manual. 4th ed. 2012, Cold Spring Harbor, N.Y.: Cold Spring Harbor Laboratory Press*.*

In a further embodiment, it is provided a host cell comprising a recombinant vector according to the invention.

The introduction of the recombinant vector in a host cell can be carried out according to methods that are well known to a person skilled in the art, such as those described in *Green et al., 2012, supra* and Davis et al., Basic methods in molecular biology, 2nd ed. 1994, Norwalk, Conn.: Appleton & Lange. xiv, 777, such as transfection by calcium phosphate, transfection by DEAE dextran, transfection, microinjection, transfection by cationic lipids, electroporation, transduction, or infection.

The host cell can be, for example, bacterial cells such as *E. coli*, cells of fungi such as yeast cells and cells of Aspergillus, Streptomyces, insect cells, Chinese Hamster Ovary cells (CHO), C127 mouse cell line, BHK cell line of Syrian hamster cells, Human Embryonic Kidney 293 (HEK 293) cells. In a particular embodiment, the host cell is a CHO cell or a HEK 293 cell.

The host cells can be used, for example, to express a polypeptide of the invention. After purification by standard methods, the polypeptide of the invention can be used in a method described hereinafter.

For instance, when expression systems that secrete the recombinant protein are employed, the culture medium may first be concentrated using a commercially available protein concentration filter, for example, an ultrafiltration unit. Following the concentration step, the concentrate can be applied to a purification matrix such as a gel filtration medium. Alternatively, an anion exchange and/or an affinity resin can be employed. The matrices can be acrylamide, agarose, dextran, cellulose, or other types commonly employed in protein purification. Alternatively, a cation exchange step can be employed. Some or all of the foregoing purification steps, in various combinations, are well known and can be employed to provide a substantially homogeneous recombinant protein.

Recombinant polypeptides produced in bacterial culture can be isolated by initial disruption of the host cells, centrifugation, extraction from cell pellets if an insoluble polypeptide, or from the supernatant fluid if a soluble polypeptide, followed by one or more concentration, salting-out, ion exchange, affinity purification or size exclusion chromatography steps. Microbial cells can be disrupted by any convenient method, including freeze-thaw cycling, sonication, mechanical disruption, or use of cell lysing agents, including detergents.

In another aspect, the polypeptide, fragments, and variants thereof can be used associated with a pharmaceutically acceptable salt or a combination of pharmaceutically acceptable salts.

### Compositions

The invention provides variants or fragments thereof, pharmaceutical compositions thereof, and methods for treating a subject, in particular a mammalian subject, and most particularly a human patient who is suffering from a hypersensitivity to an allergen/antigen or a risk of developing hypersensitivity to an allergen/antigen, in particular allergen-induced or atopic asthma, food allergy, atopic dermatitis, allergic rhinitis, and eosinophilic esophagitis.

According to another aspect, the invention provides variants or fragments thereof, pharmaceutical compositions thereof and methods for controlling hypersensitivity to an antigen/allergen in a subject, in particular inducing a tolerance to said antigen/allergen.

In a particular embodiment, the invention provides variants or fragments thereof and a pharmaceutical formulation according to the invention for use as a medicament.

Pharmaceutical compositions of the invention can contain at least one variant or fragment thereof according to the invention in any form described herein. Compositions of this invention may further comprise one or more pharmaceutically acceptable additional ingredient(s) such as alum, stabilizers, antimicrobial agents, buffers, coloring agents, flavoring agents, adjuvants, and the like.

The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline, or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. According to a particular embodiment, compositions according to the invention are injectable.

Compositions of this invention may be liquid formulations including, but not limited to, aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween^{®}, and Span^{®}.

Further materials as well as formulation processing techniques and the like are set out in Remington's "The Science and Practice of Pharmacy", 23rd Edition, 2020, Adeboye Adejare, University of the Sciences, Philadelphia, PA, USA*,* the content of which is incorporated herein by reference.

Compositions of this invention may also be formulated as a depot preparation, which may be administered by implantation or by intramuscular injection.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, acacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Compositions of this invention may also be formulated for inhalation, which may be in a form including, but not limited to, a solution, suspension, or emulsion that may be administered as a dry powder or in the form of an aerosol or spray using a propellant.

The compounds of this invention can also be administered in sustained release forms or from sustained release drug delivery systems.

In certain embodiments, the therapeutic compound(s) are directly administered as a pressurized aerosol or nebulized formulation to the patient's lungs via inhalation. Such formulations may contain any of a variety of known aerosol propellants useful for endopulmonary and/or intranasal inhalation administration. In addition, water may be present, with or without any of a variety of cosolvents, surfactants, stabilizers (e.g., antioxidants, chelating agents, inert gases, and buffers). For compositions to be administered from multiple dose containers, antimicrobial agents are typically added. Such compositions are also generally filtered and sterilized and may be lyophilized to provide enhanced stability and to improve solubility.

The pharmaceutical composition of the invention may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of compounds of the invention may be delivered in single phase, bi-phasic, or tri-phasic systems in order to deliver the active ingredient(s). Delivery of the aerosol includes the necessary container, activators, valves, subcontainers, and the like, which together may form a kit. One of ordinary skill in the art, without undue experimentation may determine preferred aerosols.

According to one aspect, the invention provides an oral pharmaceutical composition.

According to one aspect, the invention provides an injectable pharmaceutical composition.

According to one aspect, the invention provides an inhalable pharmaceutical composition.

As described elsewhere herein, in certain embodiments, a prophylactic/therapeutically effective dose of a variant or fragment thereof, a pharmaceutical composition thereof as used herein is a dose sufficient to induce tolerance to an antigen/allergen measured using any of a variety of methods as described herein. In a further embodiment, a prophylactic/therapeutically effective dose of a variant or fragment thereof, pharmaceutical composition thereof as used herein is a dose sufficient to induce T cell tolerance to an antigen/allergen as measured using any of a variety of methods as described herein, such as cytokine release assays, intracellular cytokine staining and flow cytometry, and the like. Functional T-cell assays, T-cell suppression assays measuring the suppression of proliferation or cytokine secretion by co-cultured effector T-cells may also be used.

### Mode of administration

Extracts, extract components, polypeptides and compositions of this invention may be administered in any manner including intravenous injection, intraperitoneal injection, subcutaneous injection, oral route, intranasal administration, intrapulmonary instillation or by inhalation. In certain embodiments, a combination of different routes may also be used.

The exact dose of polypeptides and compositions is readily determined by one of skill in the art based on the teachings herein, along with the potency of the specific polypeptide and composition, the age, weight, sex, and physiological condition of the subject.

By way of example, in various embodiments the dosage of a tolerizing polypeptide and composition required to achieve (or maintain) tolerance in a subject is low relative to traditional tolerization regimens. For instance, as few as one or a few doses (e.g., fewer than about three, or fewer than about five doses) of agent may be sufficient to induce tolerance. By way of example, a weekly from about 5 to about 500 mg/dose might be used to achieve tolerization effects.

According to one embodiment, polypeptides and compositions of the invention are administered before or at the beginning of the onset of the allergic symptoms. For example, polypeptides and compositions of the invention are administered before the subject is subjected to the allergen(s), typically in case of patients at risk of suffering from a seasonal allergic disorder such as pollen allergy or before the patient has already developed allergic symptoms in case of infants at risk of suffering from an allergic disorder (e.g. genetic or environmental risk) such as atopic asthma. Administration during pregnancy (oral, intranasal or via any other route) to pregnant mothers at risk of atopy can be envisioned as well, either alone or in combination with continued treatment of the newborn infant such as described in Pfefferle et al., 2013, J Allergy Clin Immunol, 2013. 131(6): p. 1453-63*; quiz 1464.*

According to a further embodiment, the polypeptides and compositions of the invention are administered at least two weeks (e.g., from about two to about 12 weeks) before the usual period of allergen exposure. In case of administration to pregnant mothers with the goal to reduce asthma and allergic risk in the newborn, the administration should be initiated early, ideally already in the first trimenon.

### Combination

According to the invention, a polypeptide or variant or fragment thereof and pharmaceutical formulations thereof can be administered alone or in combination with a co-agent useful in the prevention and/or treatment of hypersensitivity, in particular allergic disorders such as atopic asthma e.g. for example a co-agent selected from a bronchodilator, a co-administered allergen used for hypo-sensitization with a co-agent useful in the tolerization to an antigen/allergen e.g. for example antibodies or other reagents that interfere with co-stimulation or co-inhibition (e.g. via PD1, CTLA-4, CD28, CD40 and others expressed on lymphocytes and other immune cells).

The invention encompasses the administration of a polypeptide or variant or fragment thereof and pharmaceutical formulations thereof to an individual prior to, simultaneously or sequentially with other therapeutic/prophylactic/immunotherapeutic regimens or co-agents in the prevention or treatment of antigen/allergen hypersensitivity, in particular allergic disorders such as atopic asthma (e.g., combined tolerization regimen), in a therapeutically effective amount. A polypeptide or variant or fragment thereof the pharmaceutical formulation thereof that is administered simultaneously with said co-agents can be administered in the same or different composition(s) and by the same or different route(s) of administration.

According to another aspect, the polypeptide or variant or fragment thereof according to the invention may be used in an immunotherapy regimen wherein the polypeptide or variant or fragment thereof according to the invention are associated with at least one antigen of a broad range of antigens (allergens). Polypeptides or variants or fragments thereof according to the invention could be mixed and administered with house dust mite allergen, pollen-derived allergens, or any other allergens to promote desensitization in an allergen-specific manner (Khinchi et al., 2004, Allergy, 59(1): p. 45-53*).*

According to one embodiment, is provided a pharmaceutical formulation comprising a polypeptide or variant or fragment thereof, combined with at least one co-agent useful in the prevention and/or treatment of hypersensitivity, in particular allergic disorders such as atopic asthma, and at least one pharmaceutically acceptable carrier.

The dose administered, as single or multiple doses, to an individual will vary depending upon a variety of factors, including pharmacokinetic properties, patient conditions, and characteristics (sex, age, body weight, health, size), extent of symptoms, concurrent treatments, frequency of treatment and the effect desired.

### Patients

In an embodiment, patients according to the invention are patients suffering from a disorder selected from an allergic disorder such as allergen-induced or atopic asthma, atopic dermatitis (eczema), atopic rhinitis (hay fever), allergic conjunctivitis, food allergy, occupational allergy, allergic broncho-pulmonal aspergillosis and eosinophilic esophagitis.

In another embodiment, patients according to the invention are patients at risk of suffering from an allergic disorder.

In another further embodiment, patients according to the invention are suffering from allergen-induced or atopic asthma.

In another embodiment, patients according to the invention are patients at risk of suffering from a seasonal allergic disorder such as pollen allergy.

In another further embodiment, patients according to the invention are children or infants, for example infants before the age of about three years.

In another further embodiment, patients according to the invention are pregnant mothers with a high risk of atopy or pregnant mothers of children with a high risk of atopy, which may be treated during pregnancy.

In another further embodiment, patients according to the invention are suffering from an allergic disorder selected from atopic dermatitis, atopic rhinitis, eosinophilic esophagitis, and allergic conjunctivitis.

In another further embodiment, patients according to the invention are suffering food allergy.

In an embodiment, patients according to the invention are patients suffering from a disorder selected from an inflammatory response to an allergen, an autoimmune disease or disorder or a fibrotic disease or disorder, in particular inflammatory bowel disease, including Crohn's disease and ulcerative colitis.

In a particular embodiment, patients according to the invention are suffering from an auto-immune disease.

### Use according to the invention.

In one embodiment of the invention is provided a polypeptide of the invention, a fragment, or a variant thereof for use in the prevention and/or treatment of an allergic disorder, in particular atopic asthma and/or inducing a tolerization response to an allergen.

According to another aspect, is provided a use of a polypeptide or a variant or fragment or a formulation thereof according to the invention for the preparation of a pharmaceutical composition for the prevention, repression and/or treatment of an allergic disorder or an allergic response, in particular as allergen-induced or atopic asthma, atopic dermatitis (eczema), atopic rhinitis (hay fever), allergic conjunctivitis, food allergy, occupational allergy, allergic broncho-pulmonal aspergillosis, hypersensitivity pneumonitis.

In another embodiment of the invention is provided a use of a polypeptide or a formulation thereof according to the invention for the preparation of a pharmaceutical composition for the repression or treatment of allergen hypersensitivity.

In another embodiment of the invention is provided a use of a polypeptide or a formulation thereof according to the invention for the preparation of a pharmaceutical composition for tolerizing a subject or inducing a tolerization response in said subject. In another embodiment of the invention is provided a method for tolerizing a subject or inducing a tolerization response in said subject, said method comprising administering in a subject in need thereof an effective or tolerizing amount of a polypeptide or a formulation thereof according to the invention.

In another embodiment of the invention is provided a method for preventing, repressing or treating an allergic response, in particular, an allergic disorder in a subject, in particular atopic asthma, said method comprising administering in a subject in need thereof a therapeutically effective amount a polypeptide according to the invention, a fragment or a variant thereof, or a pharmaceutical formulation thereof according to the invention.

According to another embodiment, the invention is provided a method for treating allergen intolerance in a subject, said method comprising administering sequentially or simultaneously to said subject a polypeptide according to the invention or a composition thereof and the allergen(s) or an antigenic component or fragment or analog thereof in an amount effective to induce tolerance to said allergen in said subject.

In a further embodiment of the invention is provided a use or a method according to the invention, wherein the subject is predisposed or at risk to develop an allergic disorder, in particular atopic asthma, for example based on familial history, overweight status or age.

The polypeptide, formulation, or combination according to the invention is to be administered in an amount and in accordance with a dosage regimen that is effective for inducing tolerance in a subject.

According to another embodiment, the invention relates to a pharmaceutical formulation comprising a polypeptide selected from a polypeptide, a fragment or a variant thereof, combined with at least one co-agent useful in the prevention, repression and/or treatment of an allergic disorder, in particular atopic asthma and/or for inducing a tolerization response to an allergen, and at least one pharmaceutically acceptable carrier. In another embodiment, is provided a use or a method according to the invention, wherein a polypeptide or a composition of the invention are to be used in combination with an allergen.

In another embodiment, is provided polypeptide, a composition, or a method according to the invention wherein said polypeptide or a composition thereof is to be administered by the oral, intranasal, intrapulmonary, parenteral, or systemic route.

In another embodiment, is provided polypeptide, a composition, or a method according to the invention wherein the polypeptide is of SEQ ID NO: 10.

Examples illustrating the invention will be described hereinafter in a more detailed manner and by reference to the embodiments represented in the Figures.

### EXAMPLES

The following abbreviations refer respectively to the definitions below:
**BALF** (Broncho-alveolar lavage fluid), **BCA** (Bicinchoninic acid assay), **BMD (Bone Marroy derived), EDTA** (ethylene-diaminetetraacetic acid), **FCS** (Foetal Calf Serum), **GM-CSF** (granulocyte macrophage- colony stimulating factor), **H&E** (Hematoxylin and eosin, **i.p.** (intraperiotoneally), **PAS** (periodic acid Schiff), **PBS** (Phosphate Buffer Sulfate), **RPMI** (Royal Park Memorial Institute (culture medium).

### Example 1: Effects of polypeptides of the invention on induction of IL-10 and TGF-β expression in human and murine immune cells

To identify VacA peptides variants that are likely to induce immunotolerance, the activity of these variants was monitored in a series of cellular assays by measuring the induction of the expression of interleukin-10 (IL-10) and/or TGF-β. IL-10 is known to inhibit the expression of pro-inflammatory cytokines such as IFN-y, IL-2, IL-3, TNF-α and GM-CSF produced by macrophages and Th1 cells and inhibits the anti-presentation capacity of antigen-presenting cells such as dendritic cells *(*Saraiva et al., 2020, J Exp Med, 217(1*)*).

TGF-β induces the expression of FoxP3 and is necessary for the differentiation of peripheral undifferentiated T-cells into immunomodulatory Tregs which play an important role in immunotolerance (Chen, 2023, Annu Rev Immunol, 41: p. 483-512). Induction of the expression of both IL-10 and TGF-β by the peptides would suggest that those can induce the maturation of peripheral T-cells into the FoxP3⁺ CD4⁺ Tregs observed in animal models (*Reuter et al., 2023, supra; Altobelli et al., 2019, supra;* Kyburz et al., 2017, Clin Exp Allergy, 47(10): p. 1331-1341). The effect of the peptides of the invention on IL-10 and TGF-β expression was followed in 6 types of immune cells of human or murine origin as described below.

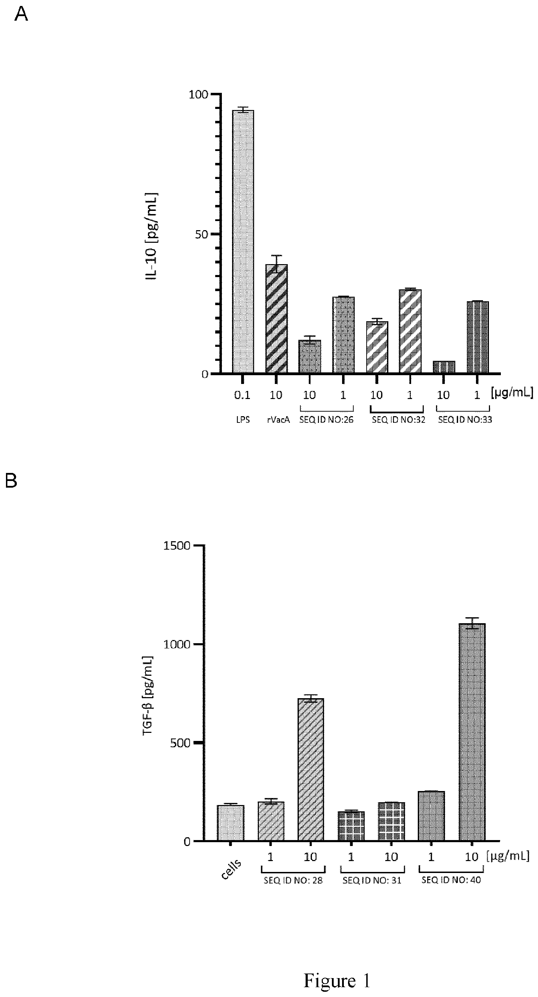

Polypeptides of SEQ ID NO:1 or SEQ ID NO:2 that were prepared by molecular biology, expressed either in CHO or HEK cells and purified using standard protein purification protocols were tested for their propensity to induce the expression of IL-10 and/or TGF-β using human or mouse cells produced as described below. **Figure 1A** shows the increase in the levels of TGF-β in the supernatant of THP-1 derived M2-macrophages when exposed to two different concentrations of a partially purified preparation of polypeptides derived from SEQ ID NO:1 or SEQ ID NO:2 (peptides of the invention SEQ ID: 32 and 33 and wild-type reference peptide of SEQ ID NO: 26). Most polypeptides displayed similar potencies in this assay, and when important differences in potency were observed, they were usually due to differences in the level of purity of the polypeptides, and not of the intrinsic activity of the polypeptide itself (this was proven subsequently when the highly purified polypeptides were used to measure the binding affinity of the polypeptides (**Figures 3** **and** **4**). **Figure 1B** displays similar data for another series of partially purified preparations of peptides of the invention (peptides of the invention SEQ ID: 31 and 40 and comparative peptide of SEQ ID NO: 28.

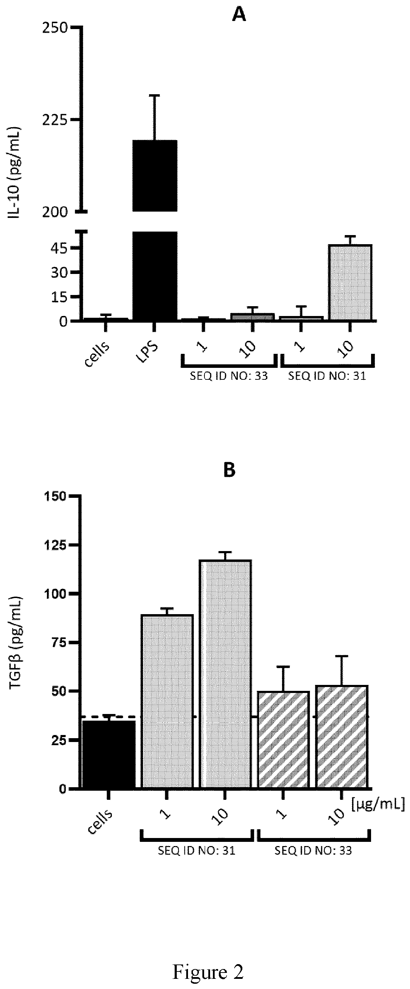

**Figure 2A** show such data for the induction of the expression of IL-10 in human PBMC-derived dendritic cells (DCs) when exposed to peptides of SEQ ID NO:1 (SEQ ID:30 and 31). **Figure 2B** displays similar data for the peptides (SEQ ID NO:31 and SEQ ID NO:33) but displays the levels of TGF-β, rather than IL-10 in the supernatant.

Once a peptide of SEQ ID NO:1 or of SEQ ID NO:2 was identified, based on the data obtained in experiments such as those described in Figures 1 & 2, the effect of increasing concentration of the variants against the mouse and human DCs and M2-macrophages in order to confirm that the binding efficiency of the variant had not been modified by the changes in amino acid sequence and that the variant was thus likely to maintain its anti-allergic properties.

**Figure 3A** shows the results of the measurement of the induction of expression of mIL-10 in mouse bone-marrow derived dendritic cells as a function of increasing doses of a highly purified preparation of polypeptide of SEQ ID NO:31. These experiments typically permit the determination of an EC₅₀ value (in this case, approximately 64 µg/mL or 771 nM for a monomeric SEQ ID NO:31). **Figure 3B** shows the results of a similar experiment using mouse bone-marrow-derived dendritic cells but monitoring the increase in levels of mTGF-β in the supernatant of the cells after 24 hours of incubation with increasing doses of a highly purified preparation of polypeptide of SEQ ID NO:31.

An EC₅₀ value of 1.3 µg/mg or 15 nM was obtained in this experiment. **Figure 3C** shows the results of an experiment using mouse bone-marrow-derived M2-macrophages, monitoring the increase in levels of mIL-10 in the supernatant of the cells after 24 hours of incubation with increasing doses of a highly purified preparation of peptide of SEQ ID NO:31. An EC₅₀ value of 1 µg/mg or 12 nM was obtained in this experiment. **Figure 3D** shows the results of an experiment using mouse bone-marrow-derived M2-macrophages, monitoring the increase in levels of mTGF-β in the supernatant of the cells after 24 hours of incubation with increasing doses of a highly purified preparation of peptide of SEQ ID NO:31. An EC₅₀ value of 1.1 µg/mg or 13 nM was obtained in this experiment.

**Figure 4A** shows the results of an experiment using human PBMC-derived dendritic cells, monitoring the increase in levels of hIL-10 in the supernatant of the cells after 24 hours of incubation with increasing doses of a highly purified preparation of peptide of SEQ ID NO:31. An EC₅₀ value of 1.26 µg/mg or 15 nM was obtained in this experiment. **Figure 4B** shows the results of an experiment using human PBMC-derived dendritic cells, monitoring the increase in levels of hTGF-β1 in the supernatant of the cells after 24 hours of incubation with increasing doses of a highly purified preparation of peptide of SEQ ID NO:31. An EC₅₀ value of 0.78 µg/mg or 9 nM was obtained in this experiment. **Figure 4C** shows the results of an experiment using human PBMC-derived M2-macrophages, monitoring the increase in levels of hIL-10 in the supernatant of the cells after 24 hours of incubation with increasing doses of a highly purified preparation of peptide of SEQ ID NO:31. An EC₅₀ value of 0.16 µg/mg or 1.9 nM was obtained in this experiment. **Figure 4D** shows the results of an experiment using human PBMC-derived M2-macrophages, monitoring the increase in levels of hTGF-β1 in the supernatant of the cells after 24 hours of incubation with increasing doses of a highly purified preparation of peptide of SEQ ID NO:31. An EC₅₀ value of 0.9 µg/mg or 1 nM was obtained in this experiment.

**Figure 5** is an example of a time course experiment, monitoring the levels of IL-10 in the supernatant of human PBMC-derived M2-macrophages exposed to 10 µg/mL of a polypeptide of SEQ ID NO:31 (the dose of SEQ ID NO:31 having been chosen because it is close to the EC₅₀ values measured for this compound on these cells). This time course experiment suggests that after 24 hours, the cells have produced the vast majority of the cytokine, and there is no need to measure the generation of IL-10 or other cytokines beyond 24 hours.

### Preparation of murine bone-marrow derived M2-macrophages

After bone-marrow derived (BMD) cell isolation, 1x10⁶ cells/mL were seeded in T-75 cm² tissue culture flasks with RPMI 1640 media supplemented with 10% fetal calf serum (FCS) and 20 ng/mL of murine M-CSF (mM-CSF). Cells were incubated for 3 days at 37°C with 5% CO₂ in incubator. The BMD cells were observed daily and, after 3 days, the old media was removed, and the cells washed once with RPMI media 1640, and fresh RPMI 1640 media supplemented with 10% fetal calf serum (FCS) and 20 ng/mL of mM-CSF added. Cells were incubated for four more days at 37°C with 5% CO₂ in incubator. During these 4 days, the cells differentiate into M2-like macrophages. On day 7, the cells were scraped and seeded at 1x10⁶ cells/mL in 48-well tissue culture plates in RPMI 1640 media. Peptides were added to the cell culture at various concentrations along with positive controls, and the cells incubated for 24h at 37°C with 5% CO₂ in incubator. After the 24 hours, the supernatants were collected and stored at - 70°C for mouse IL-10 and TGF-β1 ELISA measurements.

### Preparation of murine bone-marrow derived dendritic cells (BMDCs)

After bone-marrow derived (BMD) cell isolation, 1x10⁶ cells/mL were seeded in T-75 cm² tissue culture flasks with RPMI 1640 media supplemented with 10% fetal calf serum (FCS) and 20 ng/mL of murine GM-CSF (mGM-CSF). Cells were incubated for 3 days at 37°C with 5% CO₂ in incubator. The BMD cells were observed daily and, after 3 days, the old media was removed, and the cells washed once with RPMI media 1640, and fresh RPMI 1640 media supplemented with 10% fetal calf serum (FCS) and 20 ng/mL of mGM-CSF added. Cells were incubated for four more days at 37°C with 5% CO₂ in incubator. During these 4 days, the BMD cells differentiate into mature dendritic cells. On day 7, the cells were scraped and seeded at 1x10⁶ cells/mL in 48-well tissue culture plates in RPMI 1640 media. Peptides were added to the cell culture at various concentrations along with positive controls, and the cells incubated for 24h at 37°C with 5% CO₂ in incubator. After the 24 hours, the supernatants were collected and stored at -70°C for mouse IL-10 and TGF-β1 ELISA measurements.

### Preparation of human THP1-derived dendritic cells (DCs)

Human THP-1 monocyte cells at a cell density of 0.2x10⁶ cells/mL were seeded in 48-well tissue culture plates with RPMI 1640 media supplemented with 10% fetal calf serum (FCS), 5 ng/mL human IL-4 (hIL-4) and 5 ng/mL human GM-CSF (hGM-CSF). Cells were incubated for 5 days at 37°C with 5% CO₂ in incubator. THP-1 monocytes differentiate into immature dendritic cells. Cells were examined, old media removed, and cells washed once with RPMI 1640 or DPBS. Fresh RPMI 1640 supplemented with 10% FBS, 5 ng/mL hIL-4, 5 ng/mL hGM-CSF, 20 ng/mL hTNF-α & 200 ng/mL of Ionomycin were added and the cells incubated for 1-3 days at 37°C, 5% CO₂ in incubator. During this time, immature dendritic cells convert to mature dendritic cells. Cells were examined, old media replaced with fresh RPMI, and the cells treated with VacA variants at various concentrations along with positive controls. The plate was incubated at 37°C, 5% CO₂ in incubator. After 24hrs, the supernatant collected and stored at -70°C for human IL-10 & TGF-β1 ELISA.

### Preparation of human THP-1-derived M2-macrophages

Human THP-1 monocyte cells at a cell density of 0.5x10⁶ cells/mL were seeded in 48-well tissue culture plates with RPMI 1640 media supplemented with 10% fetal calf serum (FCS). 150 nM of PMA were added to the wells and the cells incubated for 24hrs at 37°C, 5% CO₂ in incubator. THP-1 monocytes differentiate into macrophages. Cells were examined, old media removed, and cells washed once with RPMI 1640 or DPBS. Fresh RPMI 1640 supplemented with 10% FBS, 20 ng/mL hIL-4 and 20 ng/mL human IL-13 were added and the cells incubated for 3 days at 37°C, 5% CO₂ in incubator. THP-1 macrophages differentiate into polarized M2-macrophages. Cells were examined, old media replaced with fresh RPMI, and the cells treated with peptides of the invention at various concentrations along with positive controls. The plate was incubated at 37°C, 5% CO₂ in incubator. After 24hrs, the supernatant collected and stored at -70°C for human IL-10 & TGF-β1 ELISA.

### Preparation of human PBMC-derived M2-macrophages

Frozen PBMCs obtained from healthy volunteers were revived and cells distributed into three T-75 flasks at a seeding density of 1x10⁶ cells/mL in a total volume of 25 ml (Each T-75 containing 25 million cells in 25 ml media with 50 ng/ml hGM-CSF). The cells were incubated for 3 days at 37°C, 5% CO₂ in incubator. These cells differentiate into M0 monocytes. M0 monocytes will be adherent, while the other immune cells (T cells, NK cells and B cells) will remain in suspension. Spent media from each flask was transferred to a 50 mL tube and spun at 1400 rpm for 5 minutes to pellet the immune cells. This is the conditioned media; half of this media was added back to the flask, along with fresh media. 12.5 mL of fresh RPMI-10% FBS, and 12.5 mL of conditioned media were mixed and supplemented with 50 ng/mL hM-CSF and added to each T-75 flask. Cells were incubated for 3 days at 37°C, 5% CO₂ in incubator. After 3 days, the old media was removed and cells supplemented with fresh RPMI-10% FBS containing 100 ng/mL hIL-10 in a total of 15 ml media per flask. The cells were incubated for 3 days at 37°C, 5% CO₂ in incubator during which they differentiate into M2-macrophages. The adherent M2-macrophages were washed with 10 ml of PBS, 10 mL of Accutase and incubated at 37°C for 10 min to lift cells. The cells were gently scraped from the flask surface and transferred into conical tube. The cells were centrifuged at 1400 rpm for 15 min before being resuspended in 6 mL of media and count cells. Surface staining with CD163 & CD206 *(*Roszer, T. Understanding the Mysterious M2 Macrophage through Activation Markers and Effector Mechanisms. Mediators Inflamm 2015, 2015, 816460, 1-16*)* was performed to measure the percentage of M2-macrophages differentiation. The M2-macrophages were seeded in 96-well tissue culture plate at 25x10³ cells/well and incubated at 37°C, 5% CO₂ for 24 hours. The peptides were added at various concentrations to the cell culture and the cells incubated for a further 24 h. The supernatant was then collected and stored at -70°C for IL-10 and TGF-β ELISA.

### Preparation of human PBMC-derived dendritic cells

Frozen PBMCs obtained from healthy volunteers were revived and cells distributed into three T-75 flasks as described above. These cells differentiate into M0 monocytes. Spent media from each flask was transferred to a 50 mL tube and spun at 1400 rpm for 5 minutes to pellet the immune cells. This is the conditioned media; half of this media was added back to the flask, along with fresh media. 12.5 mL of fresh RPMI-10% FBS, and 12.5mL of conditioned media were mixed and supplemented with 50 ng/mL hM-CSF and added to each T-75 flask. Cells were incubated for 3 days at 37°C, 5% CO₂ in incubator. After 3 days, the old media was removed, and cells supplemented with fresh RPMI-10% FBS containing 100 ng/mL hIL-4 in a total of 15 ml media per flask. The cells were incubated for 3 days at 37°C, 5% CO₂ in incubator during which they differentiate into mature dendritic cells. The adherent M2-macrophages were washed with 10 ml of PBS, 10 mL of Accutase^{®} and incubated at 37°C for 10 min to lift cells. The cells were gently scraped from the flask surface and transferred into conical tube. Cells were centrifuged at 1400 rpm for 15 min before being resuspended in 6 mL of media and count cells. Surface staining with CD11c & HLADR was performed to measure the percentage of differentiation into dendritic cells. These dendritic cells were seeded in 96-well tissue culture plate at 25x10³ cells/well and incubated at 37°C, 5% CO₂ for 24 hours. The peptides were added at various concentrations to the cell culture and the cells incubated for a further 24 h. The supernatant was then collected and stored at -70°C for IL-10 and TGF-β ELISA.

### Example 2: Effects of the polypeptides of the invention on protection against allergic asthma in a murine model of acute allergic asthma

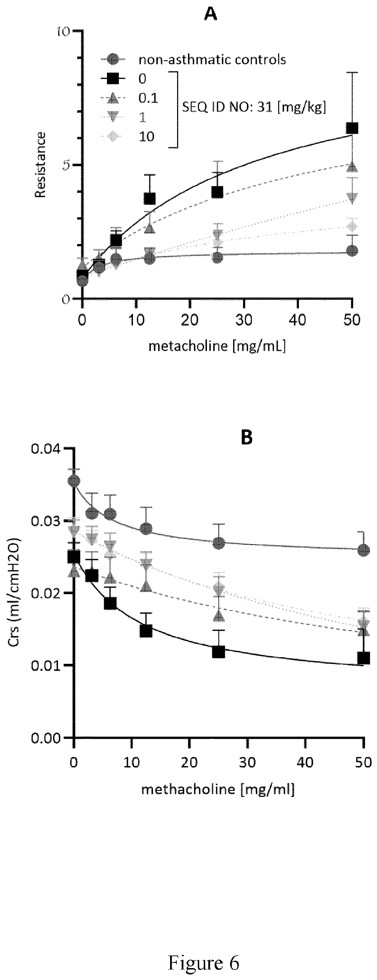

The effects of polypeptides of the invention, in particular of SEQ ID NO:10, are tested in an animal model as described below. As shown in **Figure 6A****,** HDM sensitization and challenge resulted in a robust increase in airway resistance. The increased Rrs could be dose-dependently prevented by treatment with a polypeptide of SEQ ID NO:31 with significant effects for both 1 and 10 mg/kg (p<0.001 vs Controls for both conditions; F-test 2-way ANOVA). For 10 mg/kg, additionally a significant interaction was observed between the methacholine effect and the treatment (p<0.01; F-test 2-way ANOVA). A decrease in airway compliance (Crs) could be observed in HDM-challenged animals as shown in **Figure 6B****.** The effectiveness of a peptide of SEQ ID NO:31 in inhibiting this decrease appeared to be dose-dependent, with a significant effect for both 1 and 10 mg/kg (**Figure 3****;** p<0.01 for both conditions; F-test 2-way ANOVA). No significant interaction was observed between the methacholine effect and the treatment (F-test 2-way ANOVA).

### Animals

Female C57BL/6J mice (8-12 weeks old, Charles River) were used in the experimental groups presented under **Table 3** below. All animals were housed in a conventional fashion under a 12h light-dark cycle and received food and water *ad libitum.* Mice were randomly assigned to the different experimental groups based on weight.

**Table 3**

| **Group** | **N** | **Challenge** | **Treatment** |
|---|---|---|---|
| 1 | 12 | Saline | Vehicle |
| 2 | 12 | HDM | Vehicle |
| 3 | 12 | HDM | SEQ ID NO:31 - 0,1 mg/kg |
| 4 | 12 | HDM | SEQ ID NO:31 - 1 mg/kg |
| 5 | 12 | HDM | SEQ ID NO:31 - 10 mg/kg |

### House dust mite challenge

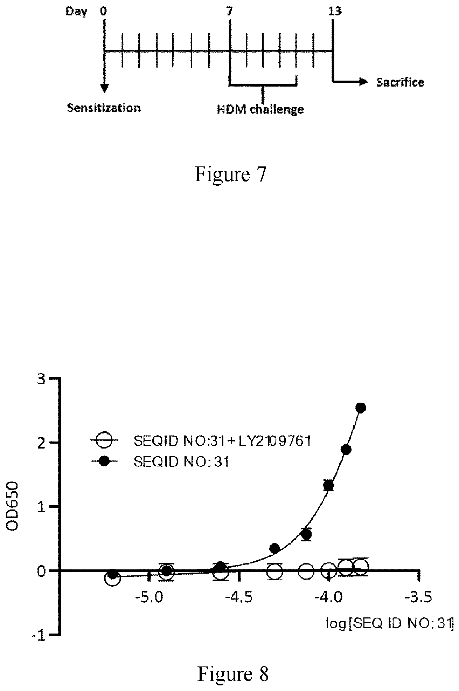

Mice were sensitized to house dust mite (HDM) by intranasal administration of 1 µg in 40 µl whole culture HDM extract (*Dermatophagoides pteronyssinus,* 15G10, Citeq, Groningen, The Netherlands, endotoxin level 1.65*10⁷ EU/gram) at day 0 of the protocol. From day 7 to 11, animals were challenged daily with 10 µg HDM in 40 µl via intranasal application as schematized under **Figure 7****.** Control animals were exposed to saline. Calculations of HDM concentration were based on protein content in the extract.

### Compound details and animal treatment

Animals received the compound based on their weight via oral gavage on days 6, 7, 9 and 11. On days of HDM challenge, treatment was administered post-challenge. The original stock was prepared in PBS pH3.5. Compound stocks were kept at -80°C until the day of use, and further dilutions were prepared in sterile PBS pH3.5.

### Airway hyperresponsiveness

Lung function was measured using the FlexiVent^{®} FX2 system (SCIREQ, Paris, France). Mice were anesthetized with ketamine + dexdomitor^{®} (75 mg/kg and 0.05 mg/kg, respectively), receiving a ¼ maintenance dose every 20 minutes. Under full anesthesia, the trachea was canulated. Prior to lung function measurements, the animals received a subcutaneous 150 µl injection of the muscle relaxant rocuronium (12.5 µg/ml). To ensure correct placing and check for leakage of the cannula, quantitative tests were performed before the first measurements. Airway resistance (Rrs, cmH₂O.s/mL), elastance (Ers, cmH₂O/mL) and compliance (Crs, ml/cmH₂O) were measured in response to increasing doses of nebulized acetyl-β-methylcholine chloride (Sigma Aldrich, Darmstadt, Germany).

### Data analysis

FlexiVent^{®} data was acquired using FlexiWare (version 8.2) software and analysed according to the single compartment model. Measurements not meeting the threshold criterion (COD>0.95) were excluded from further analysis. Statistical analysis of differences was evaluated using a two-way ANOVA. Cytokine multiplex data were analysed by a one-way ANOVA and Sidak's post-hoc test, when appropriate. Total and differential cell counts as well as the number of PAS+ cells/mm BM were compared using a Kruskal-Wallis test and Dunn's post-hoc test, when appropriate. Differences were considered to be statistically significant when p<0.05.

### Example 3: Effects of the polypeptides of the invention in engineered human embryonic kidney (HEK-293) TGF-β reporter cells

Polypeptides of the invention have been tested in the cells above in order to determine cell-surface receptors through which they signal and thereby induce immunotolerance. For this purpose, a peptide of the invention of SEQ ID NO:31 was administered to commercial engineered human embryonic kidney (HEK-293) TGF-β reporter cells that induce the production of Smad-inducible secreted embryonic alkaline phosphatase (SEAP) when human TGF-β triggers a signaling cascade leading to the formation of a Smad3/Smad4 complex which can be monitored by optical density at 650 nm as follows.

In a 96-well plate (200 µl total volume), 20 µl of a solution containing 3µM of the inhibitor LY-364947 in medium, or medium alone, are placed in a well, followed by the addition 160 µl of the HEK-Blue hTGFβ cell suspension. After 1 hour incubation, 20 µl of the test compound or the positive control are added to the wells. The negative control (NI) corresponds to 20 µl of the solvent used to prepare the dilution (PBS or water). After overnight incubation at 37°C with 5% CO₂, compounds are tested in duplicate. SEAP activity is assayed from the supernatant of HEK-Blue hTGFb cell by reading the optical density at 650 nm using a spectrophotometer after 3 hours incubation at 37°C with Quanti-blue, a SEAP detection reagent.

In this particular case, the formation of the Smad3/Smad4 complex (illustrated by the measured OD650 as shown on **Figure 8****)** was induced by the presence of SEQ ID NO:31 at the following concentrations: 6.25, 12.5, 25, 50, 75, 100, 125, and 150 µg/ml. This signaling cascade is antagonized by the presence of LY2109761 (CAS No.: 700874-71-1) which is a potent small molecule inhibitor of TGF-b1 and TGF-b2 receptor subunits, indicating that SEQ ID NO:31 binds and induces Smad3/Smad4 signaling through the TGF-β receptor. This was previously an unknown mechanism of action of VacA (assuming it is one of the original activities of VacA) and could only be determined once the protein was modified to remove the parts responsible for the pore formation and oligomerization. Therefore, those results could not be expected from the prior art.

### Example 4: Binding of polypeptides of the invention to immobilized human TGFβ R1-3

The affinity of compounds of the invention to the TGFβ receptors 1-3 (TGFβ R1, R2 or R3) was assessed as follows:
All experiments were performed using a Biacore T200 (Cytiva) and carried out at 25°C. A Series S CM5 sensor (Cytiva; Catalogue number: 100530) was docked, running buffer changed to 150 mM NaCl, 10 mM HEPES, 3 mM EDTA pH 7.4 + 0.05% Tween 20 (HBS-ET) and the sensor cleaned with a 30 second injection of 50 mM NaOH (10 µl/min flow rate). TGFβ receptors 1-3 (TGFβ R1, R2 or R3) were immobilised on the sensor surface by direct amine coupling. The reference or active channel surface was activated for 7 minutes with a 1:1 mixture of 200 mM 1-ethyl-3-(3-dimethylaminopropyl)-carbodiimide (EDC) and 50 mM N-hydroxy-succinimide (NHS). Proteins (in 10 mM sodium acetate immobilisation buffer at the previously determined optimal pH) were coupled to the surface, and then all unreacted EDC-NHS groups were inactivated with a 7-minute injection of 1 M ethanolamine. No protein was immobilised on the reference surface which was activated with EDC-NHS and capped with ethanolamine only. Analyte binding was either tested at a single concentration or at multiple concentrations in multi- and single-cycle kinetic affinity assays.

In the case of multi-cycle assays as shown in **Table 4,** the analyte samples were injected onto reference and TGFβ receptor ligand protein channels, dissociated in HBS-ET running buffer, and then the surface was regenerated with an injection of Pierce Gentle Ag/Ab Elution buffer. In binding test assays (Test), the analyte was tested at one or two concentrations with vehicle control running buffer injections included before and after each analyte for buffer blank subtraction. In kinetic affinity assays with multiple analyte concentrations tested (multi-cycle kinetics; MCK), the analyte samples were injected in ascending concentration series; in duplicate at each concentration. Vehicle control buffer blank injections were included before and after each binding series for buffer blank subtraction. All samples were injected at a flow rate of 30 µl/min.

The binding affinity of SEQ ID NO:31 to immobilized TGFβ receptors is monitored by the change in Response Units (RU) for TGFβ R1 and TGFβ R2 as shown in **Table 4** which the binding affinities of SEQ ID NO:31 to human TGF-bR1, TGFB-R2 are shown.

**Table 4**

| **Immobilized Ligand** | **TGFβ R1** | | | **TGFβ R2** | |
|---|---|---|---|---|---|
| **Analyte** | **SEQ ID NO:31** | | | **SEQ ID NO:31** | |
| **Model** | Kinetics - Two State | Equilibrium | | Kinetics - Two State | Equilibrium |
| **kₐ (M⁻¹s⁻¹)** | 2.20 × 10⁵ | | | 1.08 × 10⁹ | |
| **k_{d} (s⁻¹)** | 6.03 × 10⁻¹ | | | 6.67 × 10³ | |
| **kₐ₂ (s⁻¹)** | 6.55 × 10⁻⁴ | | | 9.04 × 10⁻⁴ | |
| **k_{d2} (s⁻¹)** | 3.53 × 10⁻³ | | | 4.06 × 10⁻³ | |
| **K_{D} (M)** | 2.31 × 10⁻⁶ | 2.90 × 10⁻⁶ | | 5.07 × 10⁻⁶ | 5.61 × 10⁻⁶ |
| **Rₘₐₓ (RU)** | 1594 | 1621 | | 4917 | 5289 |
| **Chi² (RU²)** | 123 | 308 | | 91.4 | 184 |

Altogether, those data support that the peptides according to the invention advantageously present anti-allergy activity while having lost capacity to generate vacuoles in epithelial cell line, their immunotolerizing properties are in particular achieved through the induction of the TGF-β receptor cascade in their target cells (dendritic cells and macrophages).

### LIST OF SEQUENCES

**Consensus s1m1 family** 
SEQ ID NO: 1
X1AFFX2TVIIPAIVGGIATGX3X4VGTVSGLLX5X6X7LKX8X9EX10ANKX11X12DX13PX14KV
X15X16IQX17X18X19GFX20X21FX22X23X24X25YX26LYX27X28LLSX29KIX30X31GWDX32GNX33X3
4X35HYWVKX36GQX37NKLEX38X39MX40X41X42VGX43YX44LSGLX45NFTGGDLX46X47NMQ
KATLX48LGQFNGNSFTSYKX49X50X51X52RTTRVX53FX54AX55NIX56X57X58NFVEINNRVG
SGX59GX60KAX61STVLTLX62X63X64X65X66IX67X68X69X70NX71EISLYDGX72X73LX74LX75X76
X77X78SVX79LX80GX81VX82MGX83LQYVGAYX84AX85X86X87STIX88X89X90KVX91GX92X93X9
4FX95HLX96X97GX98X99NX100AX101X102GIIX103X104X105X106X107X108IX109X110LX111LWQX112X1
13GLX114IIX115PX116X117GX118X119X120TX121VX122IX123X124IX125TX126X127DGTIRVGGYX128A
X129LTTNAAX130X131X132IGX133X134X135VNLSNX136X137SGX138X139LLVENX140TGX141ITVX
142GX143LX144VNX145QX146X147GX148X149LX150GSX151X152X153FEFKAGX154X155TX156X157X158
X159X160X161FNX162X163IX164LX165RX166X167NLX168X169X170X171X172TX173X174X175X176X177X178
X179X180X181X182X183X184X185GFX186X187X188LX189FX190X191VTX192KVX193IX194X195LX196TX197
X198TNVX199VKX200X201X202IX203X204LIVX205TX206X207X208X209VGX210YTX211FX212X213X214I
GX215X216SX217IX218X219VX220LX221X222GX223X224X225IFX226GGVX227FKX228GX229X230LX231I
X232EFX233X234X235PWNYFDAX236X237IX238X239VEIX240X241KFX242X243X244X245X246X247X248X
249X250GX251X252X253LX254X255NNLX256LX257X258NAX259MX260YX261X262X263X264X265LX266IX2
67X268X269FX270NNX271X272X273X274NX275LVX276X277X278X279X280X281X282LX283VX284X285X286X
287X288MX289FX290NX291IDX292X293TGFYX294PX295IX296IX297X298AQX299LX300X301NX302X303H
VLLX304AX305X306IX307YX308X309VX310SX311X312X313X314X315X316X317X318X319X320X321X322EX3
23X324X325X326RLALYX327X328NNRMDX329CVVX330X331X332X333X334DIKX335CX336X337AIGX3
38X339X340MVX341X342PX343X344YX345YLX346GKAWX347NX348X349IX350KX351AX352X353SX354I
X355VX356X357X358X359X360X361X362X363X364X365X366X367X368X369X370X371X372X373X374X375X376X
377X378X379
 wherein
   **X₁** is absent.
   **X₂** is selected from Threonine, Methionine, or a homologous variant thereof.
   **X₃** is selected from Threonine, Alanine, or a homologous variant thereof.
   **X₄** is selected from Alanine, Threonine, or a homologous variant thereof.
   **X₅** is selected from Glycine, Serine, Alanine, or a homologous variant thereof.
   **X₆** is selected from Tryptophan, Glycine, or a homologous variant thereof.
   **X₇** is selected from Glycine, Glutamate, or a homologous variant thereof.
   **X₈** is selected from Glutamine, Proline, Lysine, or a homologous variant thereof.
   **X₉** is selected from Alanine, Serine, or a homologous variant thereof.
   **X₁₀** is selected from Glutamate, Glutamine, or a homologous variant thereof.
   **X₁₁** is selected from Threonine, Asparagine, Isoleucine, Alanine, Proline, Serine, or a homologous variant thereof.
   **X₁₂** is selected from Proline, Glutamine, Threonine, or a homologous variant thereof.
   **X₁₃** is selected from Lysine, Threonine, or a homologous variant thereof.
   **X₁₄** is selected from Aspartate, Glutamate, Asparagine, Lysine, or a homologous variant thereof. **X₁₅** is selected from Tryptophan, Arginine, or a homologous variant thereof.
   **X₁₆** is selected from Arginine, Histidine, or a homologous variant thereof.
   **X₁₇** is selected from Alaine, Threonine, or a homologous variant thereof.
   **X₁₈** is selected from Glycine, Arginine, or a homologous variant thereof.
   **X₁₉** is selected from Lysine, Arginine, Isoleucine, Serine, Asparagine, or a homologous variant thereof.
   **X₂₀** is selected from Asparagine, Aspartate, or a homologous variant thereof.
   **X₂₁** is selected from Glutamate, Asparagine, Glutamine, Histidine, Serine, Aspartate, or a homologous variant thereof.
   **X₂₂** is selected from Proline, Leucine, Threonine, Serine, or a homologous variant thereof.
   **X₂₃** is selected from Asparagine, Histidine, Threonine, Serine, Lysine, or a homologous variant thereof.
   **X₂₄** is selected from Lysine, Glycine, or a homologous variant thereof.
   **X₂₅** is selected from Glutamate, Glutamine, or a homologous variant thereof.
   **X₂₆** is selected from Aspartate, Glycine, or a homologous variant thereof.
   **X₂₇** is selected from Lysine, Glutamine, Arginine, Threonine, or a homologous variant thereof. **X₂₈** is selected from Serine, Alanine, or a homologous variant thereof.
   **X₂₉** is selected from Serine, Asparagine, or a homologous variant thereof.
   **X₃₀** is selected from Aspartate, Asparagine, Histidine, Glutamate, or a homologous variant thereof.
   **X₃₁** is selected from Glycine, Tryptophan, or a homologous variant thereof.
   **X₃₂** is selected from Tryptophan, Tyrosine, Leucine, or a homologous variant thereof.
   **X₃₃** is selected from Alanine, Threonine, Valine, or a homologous variant thereof.
   **X₃₄** is selected from Alaine, Serine, Threonine, or a homologous variant thereof.
   **X₃₅** is selected from Arginine, Threonine, Glycine, Lysine, Asparagine, Serine, or a homologous variant thereof.
   **X₃₆** is selected from Glycine, Aspartate, Serine, or a homologous variant thereof.
   **X₃₇** is selected from Tryptophan, Glutamine, Arginine, Leucine, Serine, or a homologous variant thereof.
   **X₃₈** is selected from Valine, Methionine, or a homologous variant thereof.
   **X₃₉** is selected from Aspartate, Asparagine, Glycine, or a homologous variant thereof.
   **X₄₀** is selected from Lysine, Glutamine, Glutamate, or a homologous variant thereof.
   **X₄₁** is selected from Aspartate, Asparagine, or a homologous variant thereof.
   **X₄₂** is selected from Alanine, Serine, or a homologous variant thereof.
   **X₄₃** is selected from Threonine, Alanine, or a homologous variant thereof.
   **X₄₄** is selected from Lysine, Asparagine, Threonine, or a homologous variant thereof.
   **X₄₅** is selected from Arginine, Isoleucine, Lysine, or a homologous variant thereof.
   **X₄₆** is selected from Aspartate, Asparagine, or a homologous variant thereof.
   **X₄₇** is selected from Valine, Isoleucine, Methionine, or a homologous variant thereof.
   **X₄₈** is selected from Arginine, Histidine, or a homologous variant thereof.
   **X₄₉** is selected from Aspartate, Glycine, or a homologous variant thereof.
   **X₅₀** is selected from Serine, Alanine, Glycine, Asparagine, Lysine, or a homologous variant thereof.
   **X₅₁** is selected from Alanine, Threonine, or a homologous variant thereof.
   **X₅₂** is selected from Aspartate, Asparagine, or a homologous variant thereof.
   **X₅₃** is selected from Aspartate, Asparagine, or a homologous variant thereof.
   **X₅₄** is selected from Asparagine, Aspartate, Serine, or a homologous variant thereof.
   **X₅₅** is selected from Lysine, Histidine, or a homologous variant thereof.
   **X₅₆** is selected from Serine, Leucine, or a homologous variant thereof.
   **X₅₇** is selected from Isoleucine, Methionine, Valine, or a homologous variant thereof.
   **X₅₈** is selected from Aspartate, Glutamate, Asparagine, or a homologous variant thereof.
   **X₅₉** is selected from Alaine, Threonine, or a homologous variant thereof.
   **X₆₀** is selected from Arginine, Methionine, or a homologous variant thereof.
   **X₆₁** is selected from Serine, Arginine, Glycine, or a homologous variant thereof.
   **X₆₂** is selected from Glutamine, Leucine, Lysine, or a homologous variant thereof.
   **X₆₃** is selected from Alanine, Serine, or a homologous variant thereof.
   **X₆₄** is selected from Serine, Glutamine, or a homologous variant thereof.
   **X₆₅** is selected from Glutamate, Glutamine, Lysine, or a homologous variant thereof.
   **X₆₆** is selected from Glycine, Lysine and Arginine, or a homologous variant thereof.
   **X₆₇** is selected from Threonine, Lysine, or a homologous variant thereof.
   **X₆₈** is selected from Serine, Glycine, or a homologous variant thereof.
   **X₆₉** is selected from Serine, Glycine, Aspartate, Arginine, Asparagine, or a homologous variant thereof.
   **X₇₀** is selected from Lysine, Glutamate, or a homologous variant thereof.
   **X₇₁** is selected from Alanine, Threonine, or a homologous variant thereof.
   **X₇₂** is selected from Alanine, Threonine, or a homologous variant thereof.
   **X₇₃** is selected from Threonine, Methionine, or a homologous variant thereof.
   **X₇₄** is absent or present and when present is selected from Asparagine or Tyrosine, or a homologous variant thereof.
   **X₇₅** is selected from Alanine or Valine, or a homologous variant thereof.
   **X₇₆** is selected from Serine, Glutamine, or a homologous variant thereof.
   **X₇₇** is selected from Asparagine, Serine, Lysine, or a homologous variant thereof.
   **X₇₈** is absent or present and when present is a peptide sequence **Insert X78** or a homologous variant thereof.
   **X₇₉** is selected from Lysine, Aspartate, Asparagine, or a homologous variant thereof.
   **X₈₀** is selected from Methionine, Asparagine, Tyrosine, Tryptophan, or a homologous variant thereof.
   **X₈₁** is selected from Asparagine, Lysine, Histidine, or a homologous variant thereof.
   **X₈₂** is selected from Tryptophan, Leucine, or a homologous variant thereof.
   **X₈₃** is selected from Arginine, Leucine, or a homologous variant thereof.
   **X₈₄** is selected from Leucine, Phenylalanine, or a homologous variant thereof.
   **X₈₅** is selected from Proline, Leucine, or a homologous variant thereof.
   **X₈₆** is selected from Serine, Histidine, or a homologous variant thereof.
   **X₈₇** is selected from Tyrosine, Threonine, or a homologous variant thereof.
   **X₈₈** is selected from Asparagine, Aspartate, or a homologous variant thereof.
   **X₈₉** is selected from Threonine, Alanine, or a homologous variant thereof.
   **X₉₀** is selected from Serine, Leucine, or a homologous variant thereof.
   **X₉₁** is selected from Threonine, Alanine, Valine, Isoleucine, Glutamine, Glutamate, Lysine, or a homologous variant thereof.
   **X₉₂** is selected from Glutamate, Glutamine, or a homologous variant thereof.
   **X₉₃** is selected from Valine, Alanine, Methionine, Threonine, or a homologous variant thereof.
   **X₉₄** is selected from Asparagine, Aspartate, Histidine, Serine, or a homologous variant thereof.
   **X₉₅** is selected from Asparagine, Arginine, Serine, Aspartate, Threonine, or a homologous variant thereof.
   **X₉₆** is selected from Threonine, Alanine, Valine, Leucine, or a homologous variant thereof.
   **X₉₇** is selected from Valine, Alanine, Leucine, or a homologous variant thereof.
   **X₉₈** is selected from Aspartate, Asparagine, or a homologous variant thereof.
   **X₉₉** is selected from Lysine, Arginine, Glutamine, Histidine, Serine, or a homologous variant thereof.
   **X₁₀₀** is selected from Alanine, Threonine, Asparagine, Serine, Glycine, or a homologous variant thereof.
   **X₁₀₁** is selected from Glutamine, Tyrosine, or a homologous variant thereof.
   **X₁₀₂** is selected from Alanine, Valine, Glycine, or a homologous variant thereof.
   **X₁₀₃** is selected from Alanine, Leucine, Proline, or a homologous variant thereof.
   **X₁₀₄** is selected from Serine, Proline, Asparagine, Glycine, or a homologous variant thereof.
   **X₁₀₅** is selected from Asparagine, Lysine, Valine, or a homologous variant thereof.
   **X₁₀₆** is selected from Lysine, Arginine, Threonine, or a homologous variant thereof.
   **X₁₀₇** is selected from Threonine, Leucine, or a homologous variant thereof.
   **X₁₀₈** is selected from Histidine, Tyrosine, Asparagine, or a homologous variant thereof.
   **X₁₀₉** is selected from Glycine, Serine, or a homologous variant thereof.
   **X₁₁₀** is selected from Threonine, Valine, Alanine, or a homologous variant thereof.
   **X₁₁₁** is selected from Aspartate, Asparagine, or a homologous variant thereof.
   **X₁₁₂** is selected from Serine, Asparagine, or a homologous variant thereof.
   **X₁₁₃** is selected from Alanine, Valine, Threonine, or a homologous variant thereof.
   **X₁₁₄** is selected from asparagine, Serine, or a homologous variant thereof.
   **X₁₁₅** is selected from Alanine, Threonine, or a homologous variant thereof.
   **X₁₁₆** is selected from Proline, Glutamine, or a homologous variant thereof.
   **X₁₁₇** is absent.
   **X₁₁₈** is selected from Glycine, Alanine, or a homologous variant thereof.
   **X₁₁₉** is selected from Lysine, Glutamine, or a homologous variant thereof.
   **X₁₂₀** is selected from Aspartate, Asparagine, or a homologous variant thereof.
   **X₁₂₁** is selected from Valine, Alanine, Methionine, Isoleucine, or a homologous variant thereof.
   **X₁₂₂** is selected from Asparagine, Threonine, Asparagine, or a homologous variant thereof.
   **X₁₂₃** is selected from Asparagine, Aspartate, Phenylalanine, Serine, or a homologous variant thereof.
   **X₁₂₄** is selected from Arginine, Histidine, Serine, or a homologous variant thereof.
   **X₁₂₅** is selected from Asparagine, Serine, or a homologous variant thereof.
   **X₁₂₆** is selected from Asparagine, Lysine, Serine, or a homologous variant thereof.
   **X₁₂₇** is selected from Alaine, Serine, Threonine, or a homologous variant thereof.
   **X₁₂₈** is selected from Lysine, Threonine, or a homologous variant thereof.
   **X₁₂₉** is selected from Serine, Leucine, or a homologous variant thereof.
   **X₁₃₀** is selected from Histidine, Asparagine, Serine, Aspartate, or a homologous variant thereof.
   **X₁₃₁** is selected from Leucine, Phenylalanine, or a homologous variant thereof.
   **X₁₃₂** is selected from Asparagine, Histidine, Tyrosine, Alanine, Aspartate, or a homologous variant thereof.
   **X₁₃₃** is selected from Lysine, Glutamate, Glutamine, or a homologous variant thereof.
   **X₁₃₄** is selected from Glycine, Missing, or a homologous variant thereof.
   **X₁₃₅** is selected from Glycine, Alanine, or a homologous variant thereof.
   **X₁₃₆** is selected from Glutamine, Serine, or a homologous variant thereof.
   **X₁₃₇** is selected from Alanine, Threonine, Valine, or a homologous variant thereof.
   **X₁₃₈** is selected from Arginine, Histidine, or a homologous variant thereof.
   **X₁₃₉** is selected from Serine, Threonine, Leucine, or a homologous variant thereof.
   **X₁₄₀** is selected from Leucine, Isoleucine, Arginine, or a homologous variant thereof.
   **X₁₄₁** is selected from Asparagine, Aspartate, or a homologous variant thereof.
   **X₁₄₂** is selected from Aspartate, Asparagine, Glutamate, Serine, or a homologous variant thereof.
   **X₁₄₃** is selected from Proline, Alanine, Threonine, Serine, Leucine, or a homologous variant thereof.
   **X₁₄₄** is selected from Arginine, Methionine, Lysine, Glutamate, or a homologous variant thereof.
   **X₁₄₅** is selected from Asparagine, Glycine, Aspartate, or a homologous variant thereof.
   **X₁₄₆** is selected from Valine, Alanine, Glycine, or a homologous variant thereof.
   **X₁₄₇** is selected from Glycine, Serine, or a homologous variant thereof.
   **X₁₄₈** is selected from Tyrosine, Alanine, Serine, or a homologous variant thereof.
   **X₁₄₉** is selected from Alanine, Threonine, or a homologous variant thereof.
   **X₁₅₀** is selected from Alanine, Serine, Glutamine, or a homologous variant thereof.
   **X₁₅₁** is selected from Serine, Asparagine, or a homologous variant thereof.
   **X₁₅₂** is selected from Alanine, Threonine, or a homologous variant thereof.
   **X₁₅₃** is selected from Asparagine, Serine, or a homologous variant thereof.
   **X₁₅₄** is selected from Valine, Threonine, Alanine, Glutamate, or a homologous variant thereof.
   **X₁₅₅** is selected from Aspartate, Asparagine, or a homologous variant thereof.
   **X₁₅₆** is selected from Lysine, Asparagine, or a homologous variant thereof.
   **X₁₅₇** is selected from Asparagine, Lysine, Glutamine, or a homologous variant thereof.
   **X₁₅₈** is selected from Glycine, Alanine, Serine, or a homologous variant thereof.
   **X₁₅₉** is selected from Threonine, Isoleucine, or a homologous variant thereof.
   **X₁₆₀** is selected from Alanine, Isoleucine, Valine, or a homologous variant thereof.
   **X₁₆₁** is selected from Threonine, Alanine, or a homologous variant thereof.
   **X₁₆₂** is selected from Asparagine, Serine, Threonine, or a homologous variant thereof.
   **X₁₆₃** is selected from Aspartate, Asparagine, Isoleucine, Glutamate, or a homologous variant thereof.
   **X₁₆₄** is selected from Serine, Histidine, Asparagine, or a homologous variant thereof.
   **X₁₆₅** is selected from Glycine, Arginine, or a homologous variant thereof.
   **X₁₆₆** is selected from Phenylalanine, Alanine, Leucine, Serine, or a homologous variant thereof.
   **X₁₆₇** is selected from Valine, Glycine, or a homologous variant thereof.
   **X₁₆₈** is selected from Lysine, Arginine, Serine, Glutamate, or a homologous variant thereof.
   **X₁₆₉** is selected from Valine, Alanine, Leucine, Threonine, or a homologous variant thereof.
   **X₁₇₀** is selected from Aspartate, Serine, Asparagine, or a homologous variant thereof.
   **X₁₇₁** is selected from Alanine, Serine, or a homologous variant thereof.
   **X₁₇₂** is selected from Histidine, Tyrosine, Arginine, or a homologous variant thereof.
   **X₁₇₃** is absent or present and when present is a peptide sequence of SEQ ID NO: 6 or a homologous variant thereof.
   **X₁₇₄** is selected from Alanine, Threonine, Valine, or a homologous variant thereof.
   **X₁₇₅** is selected from Asparagine, Lysine, Tyrosine, Histidine, Isoleucine, or a homologous variant thereof.
   **X₁₇₆** is selected from Phenylalanine, Leucine, or a homologous variant thereof.
   **X₁₇₇** is selected from Lysine, Asparagine, Glutamine, Threonine, Glycine, or a homologous variant thereof.
   **X₁₇₈** is selected from Glycine, Aspartate, Glutamate, Lysine, Asparagine, or a homologous variant thereof.
   **X₁₇₉** is selected from Isoleucine, Lysine, Leucine, Tryptophan, or a homologous variant thereof. **X₁₈₀** is selected from Aspartate, Asparagine, Isoleucine, or a homologous variant thereof.
   **X₁₈₁** is selected from Alanine, Serine, Threonine, Methionine, or a homologous variant thereof.
   **X₁₈₂** is absent or present and when present is a peptide sequence **Insert X182** or a homologous variant thereof.
   **X₁₈₃** is selected from Glycine, Serine, or a homologous variant thereof.
   **X₁₈₄** is selected from Aspartate, Asparagine, Serine, Methionine, or a homologous variant thereof.
   **X₁₈₅** is selected from Glycine, Asparagine, or a homologous variant thereof.
   **X₁₈₆** is selected from Asparagine, Threonine, Glycine, or a homologous variant thereof.
   **X₁₈₇** is absent or present and when present is a peptide sequence **Insert X187** or a homologous variant thereof.
   **X₁₈₈** is selected from Threonine, Alanine, Serine, Leucine, Asparagine, or a homologous variant thereof.
   **X₁₈₉** is selected from Aspartate, Asparagine, Histidine, Serine, or a homologous variant thereof.
   **X₁₉₀** is selected from Serine, Cysteine, Asparagine, or a homologous variant thereof.
   **X₁₉₁** is selected from Glycine, Serine, or a homologous variant thereof.
   **X₁₉₂** is selected from Asparagine, Aspartate, Glycine, or a homologous variant thereof.
   **X₁₉₃** is selected from Asparagine, Isoleucine, Serine, or a homologous variant thereof.
   **X₁₉₄** is selected from Asparagine, Serine, or a homologous variant thereof.
   **X₁₉₅** is selected from Lysine, Asparagine, or a homologous variant thereof.
   **X₁₉₆** is selected from Isoleucine, Threonine, Valine, or a homologous variant thereof.
   **X₁₉₇** is selected from Alanine, Serine, or a homologous variant thereof.
   **X₁₉₈** is selected from Serine, Alanine, or a homologous variant thereof.
   **X₁₉₉** is selected from Alanine, Asparagine, Valine, Serine, or a homologous variant thereof.
   **X₂₀₀** is selected from Asparagine, Threonine, or a homologous variant thereof.
   **X₂₀₁** is selected from Phenylalanine, Serine, or a homologous variant thereof.
   **X₂₀₂** is selected from Asparagine, Aspartate, Serine, Threonine, or a homologous variant thereof.
   **X₂₀₃** is selected from Asparagine, Lysine, or a homologous variant thereof.
   **X₂₀₄** is selected from Glutamate, Lysine, Aspartate, or a homologous variant thereof.
   **X₂₀₅** is selected from Lysine, Arginine, Threonine, or a homologous variant thereof.
   **X₂₀₆** is selected from Asparagine, Arginine, or a homologous variant thereof.
   **X₂₀₇** is selected from Glycine, Valine, or a homologous variant thereof.
   **X₂₀₈** is selected from Valine, Isoleucine, Leucine, Glutamine, or a homologous variant thereof.
   **X₂₀₉** is selected from Serine, Asparagine, or a homologous variant thereof.
   **X₂₁₀** is selected from Glutamate, Glutamine, or a homologous variant thereof.
   **X₂₁₁** is selected from Histidine, Asparagine, Tyrosine, Isoleucine, or a homologous variant thereof.
   **X₂₁₂** is selected from Serine, Glycine, Aspartate, Alanine, or a homologous variant thereof.
   **X₂₁₃** is selected from Glutamate, Lysine, Glycine, Aspartate, or a homologous variant thereof.
   **X₂₁₄** is selected from Aspartate, Asparagine, Isoleucine, or a homologous variant thereof.
   **X₂₁₅** is selected from Serine, Asparagine, Aspartate, or a homologous variant thereof.
   **X₂₁₆** is selected from Glutamine, Glutamate, Lysine, or a homologous variant thereof.
   **X₂₁₇** is selected from Arginine, Histidine, Tyrosine, or a homologous variant thereof.
   **X₂₁₈** is selected from Asparagine, Glycine, Serine
   **X₂₁₉** is selected from Threonine, Alanine, Valine, Isoleucine, or a homologous variant thereof.
   **X₂₂₀** is selected from Arginine, Serine, or a homologous variant thereof.
   **X₂₂₁** is selected from Glutamate, Glutamine, or a homologous variant thereof.
   **X₂₂₂** is selected from Threonine, Methionine, or a homologous variant thereof.
   **X₂₂₃** is selected from Threonine, Tyrosine, Isoleucine, or a homologous variant thereof.
   **X₂₂₄** is selected from Arginine, Serine, or a homologous variant thereof.
   **X₂₂₅** is selected from Serine, Glutamine, Proline, or a homologous variant thereof.
   **X₂₂₆** is selected from Serine, Alanine, or a homologous variant thereof.
   **X₂₂₇** is selected from Lysine, Glutamine, Threonine, Arginine
   **X₂₂₈** is selected from Glycine, Serine, or a homologous variant thereof.
   **X₂₂₉** is selected from Glutamate, Lysine, Glutamine, or a homologous variant thereof.
   **X₂₃₀** is selected from Lysine, Asparagine, or a homologous variant thereof.
   **X₂₃₁** is selected from Valine, Aspartate
   **X₂₃₂** is selected from Aspartate, Asparagine, or a homologous variant thereof.
   **X₂₃₃** is selected from Tyrosine, Cysteine, Phenylalanine
   **X₂₃₄** is selected from Tyrosine, Histidine, or a homologous variant thereof.
   **X₂₃₅** is selected from Serine, Alanine, or a homologous variant thereof.
   **X₂₃₆** is selected from Arginine, Lysine, Serine, or a homologous variant thereof.
   **X₂₃₇** is selected from Asparagine, Serine, or a homologous variant thereof.
   **X₂₃₈** is selected from Lysine, Threonine, Arginine, or a homologous variant thereof.
   **X₂₃₉** is selected from Asparagine, Aspartate, Isoleucine, or a homologous variant thereof.
   **X₂₄₀** is selected from Threonine, Asparagine, Alanine, Isoleucine, or a homologous variant thereof.
   **X₂₄₁** is selected from Arginine, Asparagine, Lysine, Aspartate, Glycine, or a homologous variant thereof.
   **X₂₄₂** is selected from Alanine, Leucine, Threonine, or a homologous variant thereof.
   **X₂₄₃** is selected from Phenylalanine, Serine, or a homologous variant thereof.
   **X₂₄₄** is selected from Glycine, Serine, or a homologous variant thereof.
   **X₂₄₅** is selected from Threonine, Proline, Alanine, Serine, or a homologous variant thereof.
   **X₂₄₆** is selected from Proline, Glutamine, or a homologous variant thereof.
   **X₂₄₇** is selected from Glutamate, Glycine, or a homologous variant thereof.
   **X₂₄₈** is selected from Asparagine, Serine, Tyrosine, or a homologous variant thereof.
   **X₂₄₉** is selected from Isoleucine, Proline, or a homologous variant thereof.
   **X₂₅₀** is selected from Alanine, Tryptophan, Proline, Tyrosine, or a homologous variant thereof.
   **X₂₅₁** is selected from Lysine, Threonine, Isoleucine, Alanine, or a homologous variant thereof.
   **X₂₅₂** is selected from Threonine, Serine, Alanine, or a homologous variant thereof.
   **X₂₅₃** is selected from Glycine, Lysine, Glutamine, Asparagine, or a homologous variant thereof.
   **X₂₅₄** is selected from Methionine, Tyrosine, Threonine, or a homologous variant thereof.
   **X₂₅₅** is selected from Phenylalanine, Valine, Tyrosine, or a homologous variant thereof.
   **X₂₅₆** is selected from Threonine, Alanine, Serine, or a homologous variant thereof.
   **X₂₅₇** is selected from Asparagine, Glycine, Threonine, Alanine, Aspartate, or a homologous variant thereof.
   **X₂₅₈** is selected from Serine, Glutamine, Proline, Tyrosine, Histidine, Asparagine, Leucine, or a homologous variant thereof.
   **X₂₅₉** is selected from Serine, Valine, Isoleucine, Asparagine, Alanine, or a homologous variant thereof.
   **X₂₆₀** is selected from Aspartate, Asparagine, or a homologous variant thereof.
   **X₂₆₁** is selected from Glycine, Serine, Lysine, Asparagine, or a homologous variant thereof.
   **X₂₆₂** is selected from Lysine, Glutamine, Serine, or a homologous variant thereof.
   **X₂₆₃** is selected from Aspartate, Phenylalanine, Isoleucine, or a homologous variant thereof.
   **X₂₆₄** is selected from Leucine, Serine, Glutamine, or a homologous variant thereof.
   **X₂₆₅** is selected from Aspartate, Asparagine, or a homologous variant thereof.
   **X₂₆₆** is selected from Threonine, Alanine, or a homologous variant thereof.
   **X₂₆₇** is selected from Glutamine, Serine, Histidine, Tyrosine, or a homologous variant thereof.
   **X₂₆₈** is selected from Glycine, Glutamate, Arginine, or a homologous variant thereof.
   **X₂₆₉** is selected from Histidine, Asparagine, Aspartate, or a homologous variant thereof.
   **X₂₇₀** is selected from Threonine, Isoleucine, Valine, or a homologous variant thereof.
   **X₂₇₁** is selected from Glutamine, Arginine, or a homologous variant thereof.
   **X₂₇₂** is selected from Glycine, Alanine, Serine, or a homologous variant thereof.
   **X₂₇₃** is selected from Threonine, Leucine, Valine, Alanine, Isoleucine, or a homologous variant thereof.
   **X₂₇₄** is selected from Methionine, Isoleucine, Glutamine, or a homologous variant thereof.
   **X₂₇₅** is selected from Leucine, Tyrosine, or a homologous variant thereof.
   **X₂₇₆** is selected from Glutamine, Arginine, or a homologous variant thereof.
   **X₂₇₇** is selected from Aspartate, Glycine, Asparagine, or a homologous variant thereof.
   **X₂₇₈** is selected from Glycine, Arginine, or a homologous variant thereof.
   **X₂₇₉** is selected from Arginine, Lysine, Glutamine, Asparagine, or a homologous variant thereof.
   **X₂₈₀** is selected from Valine, Isoleucine, Threonine, or a homologous variant thereof.
   **X₂₈₁** is selected from Alanine, Glutamate, Glutamine, Lysine, or a homologous variant thereof.
   **X₂₈₂** is selected from Threonine, Alanine, or a homologous variant thereof.
   **X₂₈₃** is selected from Asparagine, Serine, Glycine, or a homologous variant thereof.
   **X₂₈₄** is selected from Glycine, Aspartate, or a homologous variant thereof.
   **X₂₈₅** is selected from Histidine, Lysine, Asparagine, Tyrosine, Aspartate, or a homologous variant thereof.
   **X₂₈₆** is selected from Glutamine, Alanine, Threonine, Valine, or a homologous variant thereof.
   **X₂₈₇** is selected from Alanine, Valine, Threonine, or a homologous variant thereof.
   **X₂₈₈** is selected from Serine, Threonine, Alanine, Valine, or a homologous variant thereof.
   **X₂₈₉** is selected from Isoleucine, Methionine, Lysine, Valine, Serine, Leucine, Phenylalanine, Arginine, or a homologous variant thereof.
   **X₂₉₀** is selected from Asparagine, Serine, or a homologous variant thereof.
   **X₂₉₁** is selected from Leucine, Aspartate, Valine, Asparagine, Methionine, Glutamate, Serine, or a homologous variant thereof.
   **X₂₉₂** is selected from Serine, Asparagine, or a homologous variant thereof.
   **X₂₉₃** is selected from Alanine, Threonine, Serine, Methionine, or a homologous variant thereof.
   **X₂₉₄** is selected from Lysine, Glutamine, or a homologous variant thereof.
   **X₂₉₅** is selected from Leucine, Isoleucine, Serine, or a homologous variant thereof.
   **X₂₉₆** is selected from Lysine, Asparagine, Arginine, or a homologous variant thereof.
   **X₂₉₇** is selected from Asparagine, Aspartate, Threonine, or a homologous variant thereof.
   **X₂₉₈** is selected from Asparagine, Aspartate, Serine, or a homologous variant thereof.
   **X₂₉₉** is selected from Asparagine, Aspartate, or a homologous variant thereof.
   **X₃₀₀** is selected from Threonine, Isoleucine, Valine, or a homologous variant thereof.
   **X₃₀₁** is selected from Lysine, Glutamate, or a homologous variant thereof.
   **X₃₀₂** is selected from Lysine, Threonine, Arginine, Alanine, or a homologous variant thereof.
   **X₃₀₃** is selected from Glutamate, Lysine, or a homologous variant thereof.
   **X₃₀₄** is selected from Lysine, Arginine, Serine, or a homologous variant thereof.
   **X₃₀₅** is selected from Arginine, Glutamine, Lysine, Proline, or a homologous variant thereof.
   **X₃₀₆** is selected from Asparagine, Isoleucine, Valine, Threonine, or a homologous variant thereof.
   **X₃₀₇** is selected from Aspartate, Glycine, Asparagine, Serine, Arginine, or a homologous variant thereof.
   **X₃₀₈** is selected from Asparagine, Glycine, Glutamate, Aspartate, Valine, or a homologous variant thereof.
   **X₃₀₉** is selected from Leucine, Asparagine, or a homologous variant thereof.
   **X₃₁₀** is absent or present and when present is a peptide sequence **Insert X310** or a homologous variant thereof.
   **X₃₁₁** is selected from Tyrosine, Threonine, Leucine, Glutamate, Alanine, Isoleucine, Valine, or a homologous variant thereof.
   **X₃₁₂** is selected from Aspartate, Glycine, or a homologous variant thereof.
   **X₃₁₃** is selected from Asparagine, Threonine, Serine, Alanine, Isoleucine, or a homologous variant thereof.
   **X₃₁₄** is selected from Isoleucine, Asparagine, Lysine, Valine, Threonine, Serine, Aspartate, Alanine, or a homologous variant thereof.
   **X₃₁₅** is selected from Serine, Glycine, Asparagine, Histidine, Arginine, Alanine, or a homologous variant thereof.
   **X₃₁₆** is selected from Alanine, Isoleucine, Threonine, Phenylalanine, Serine, Valine, Glutamine, Lysine, or a homologous variant thereof.
   **X₃₁₇** is selected from Serine, Asparagine, Histidine, Arginine, Alanine, Glycine, or a homologous variant thereof.
   **X₃₁₈** is selected from Asparagine, Glycine, Alanine, Methionine, Aspartate, Glutamate, or a homologous variant thereof.
   **X₃₁₉** is selected from Threonine, Valine, Alanine, Leucine, Isoleucine, or a homologous variant thereof.
   **X₃₂₀** is selected from Asparagine, Serine, Lysine, Isoleucine, Leucine, or a homologous variant thereof.
   **X₃₂₁** is selected from Leucine, Isoleucine, Proline, Glutamine, or a homologous variant thereof.
   **X₃₂₂** is selected from Glutamine, Glutamate, Methionine, Isoleucine, Leucine, or a homologous variant thereof.
   **X₃₂₃** is selected from Glutamine, Serine, Glutamate, or a homologous variant thereof.
   **X₃₂₄** is selected from Phenylalanine, Valine, or a homologous variant thereof.
   **X₃₂₅** is selected from Lysine, Asparagine, or a homologous variant thereof.
   **X₃₂₆** is selected from Glutamate, Glycine, Aspartate, or a homologous variant thereof.
   **X₃₂₇** is selected from Asparagine, Glutamate, or a homologous variant thereof.
   **X₃₂₈** is selected from Asparagine, Histidine, or a homologous variant thereof.
   **X₃₂₉** is selected from Threonine, Isoleucine, or a homologous variant thereof.
   **X₃₃₀** is selected from Arginine, Glutamine, or a homologous variant thereof.
   **X₃₃₁** is absent or present and when present is a peptide sequence **Insert X331** or a homologous variant thereof.
   **X₃₃₂** is selected from Asparagine, Lysine, Aspartate, or a homologous variant thereof.
   **X₃₃₃** is selected from Threonine, Glutamate, Leucine, Isoleucine, or a homologous variant thereof.
   **X₃₃₄** is selected from Aspartate, Asparagine, or a homologous variant thereof.
   **X₃₃₅** is selected from Alanine, Valine, Threonine, Glutamine, or a homologous variant thereof.
   **X₃₃₆** is selected from Glycine, Tryptophan, or a homologous variant thereof.
   **X₃₃₇** is selected from Methionine, Threonine, Isoleucine, Lysine, Arginine, or a homologous variant thereof.
   **X₃₃₈** is selected from Asparagine, Aspartate, or a homologous variant thereof.
   **X₃₃₉** is selected from Glutamine, Threonine, Asparagine, or a homologous variant thereof.
   **X₃₄₀** is selected from Serine, Alanine, Aspartate, Glycine, Asparagine, or a homologous variant thereof.
   **X₃₄₁** is selected from Asparagine, Methionine, Serine, Threonine, or a homologous variant thereof.
   **X₃₄₂** is selected from Asparagine, Threonine, or a homologous variant thereof.
   **X₃₄₃** is selected from Aspartate, Glutamate, Asparagine, Serine, Lysine, Glycine, or a homologous variant thereof.
   **X₃₄₄** is selected from Asparagine, Lysine, Aspartate, Serine, or a homologous variant thereof.
   **X₃₄₅** is selected from Lysine, Arginine, Asparagine, Glutamate, or a homologous variant thereof.
   **X₃₄₆** is selected from Isoleucine, Glutamate, Leucine, Valine, Glycine, Lysine, or a homologous variant thereof.
   **X₃₄₇** is selected from Lysine, Arginine, Isoleucine, or a homologous variant thereof.
   **X₃₄₈** is selected from Isoleucine, Leucine, Threonine, or a homologous variant thereof.
   **X₃₄₉** is selected from Glycine, Serine, or a homologous variant thereof.
   **X₃₅₀** is selected from Serine, Asparagine, Aspartate, or a homologous variant thereof.
   **X₃₅₁** is selected from Threonine, Asparagine, Isoleucine, or a homologous variant thereof.
   **X₃₅₂** is selected from Asparagine, Aspartate, or a homologous variant thereof.
   **X₃₅₃** is selected from Glycine, Asparagine, Serine, Aspartate, Lysine, or a homologous variant thereof.
   **X₃₅₄** is selected from Lysine, Threonine, or a homologous variant thereof.
   **X₃₅₅** is selected from Serine, Alanine, Threonine, or a homologous variant thereof.
   **X₃₅₆** is selected from Tyrosine, Arginine, Histidine, Asparagine, Leucine, Serine, or a homologous variant thereof.
   **X₃₅₇** is selected from Tyrosine, Leucine, Valine, Phenylalanine, or a homologous variant thereof.
   **X₃₅₈** is selected from Leucine, Glycine, Arginine, or a homologous variant thereof.
   **X₃₅₉** is selected from Glycine, Asparagine, Lysine, Glutamine, or a homologous variant thereof.
   **X₃₆₀** is selected from Asparagine, Alanine, Aspartate, or a homologous variant thereof.
   **X₃₆₁** is selected from Serine, Alanine, Valine, Phenylalanine, Threonine, or a homologous variant thereof.
   **X₃₆₂** is selected from Threonine, Alanine, Tyrosine, or a homologous variant thereof.
   **X₃₆₃** is selected from Proline, Threonine, Leucine, Alanine, or a homologous variant thereof.
   **X₃₆₄** is selected from Threonine, Alanine, Serine, Proline, Asparagine, or a homologous variant thereof.
   **X₃₆₅** is selected from Glutamate, Aspartate, Threonine, Serine, Asparagine, or a homologous variant thereof.
   **X₃₆₆** is selected from Asparagine, Lysine, Serine, Aspartate, Proline, Glutamate, or a homologous variant thereof.
   **X₃₆₇** is selected from Glycine, Glutamate, Serine, Proline, Threonine, Asparagine, Aspartate, or a homologous variant thereof.
   **X₃₆₈** is selected from Glycine, Aspartate, Threonine, Serine, Valine, or a homologous variant thereof.
   **X₃₆₉** is selected from Asparagine, Aspartate, Serine, or a homologous variant thereof.
   **X₃₇₀** is selected from Threonine, Leucine, Isoleucine, Proline, or a homologous variant thereof.
   **X₃₇₁** is selected from Threonine, Proline, or a homologous variant thereof.
   **X₃₇₂** is selected from Asparagine, Aspartate, Lysine, Threonine, Serine, or a homologous variant thereof.
   **X₃₇₃** is selected from Leucine, Asparagine, Phenylalanine, or a homologous variant thereof.
   **X₃₇₄** is selected from Proline, Alanine, Threonine, or a homologous variant thereof.
   **X₃₇₅** is selected from Threonine, Lysine, Glutamate, Asparagine, Tyrosine, or a homologous variant thereof.
   **X₃₇₆** is selected from Asparagine, Serine, or a homologous variant thereof.
   **X₃₇₇** is selected from Threonine, Alanine, Serine, Lysine, Proline, or a homologous variant thereof.
   **X₃₇₈** is selected from Threonine, Glutamate, Alanine, Proline, Serine, Isoleucine, Lysine, or a homologous variant thereof and
   **X₃₇₉** is absent or present and when present is a peptide sequence **Insert X379** or a homologous variant thereof.
**Consensus s2m2 family** 
SEQ ID NO:2
X1AFFX2TVIIPAIVGGIATGX3X4VGTVSGLLSWGLKX8AEX10ANKX11PDX13PX14KVX15
X16IQAGX19GFX20X21FX22X23KX25YDLYX27SLLSSKIX30GGWDX32GNAARHYWVKX36G
QWNKLEVX39MX40X41AVGTYX44LSGLX45NFTGGDLDX47NMQKATLRLGQFNGNSFTS
YKDX50X51X52RTTRVX53FX54AKNIX56IDNFVEINNRVGSGAGRKASSTVLTLX62X63SX65
X66IX67SX69X70NAEISLYDGATLNLAX76SX78SVX79LX80GX81VWMGRLQYVGAYLAPSYS
TIX88TSKVX91GEX93NFX95HLX96VGX98X99NX100AQAGIIAX104X105KTX108IGX110LDLWQS
X113GLX114IIX115PPX117GGKX120TVVNIX123X124INTX126X127DGTIRVGGYKASLTTNAA
X130LX132IGX133GGVNLSNX136X137SGRX139LLVENLTGX141ITVX142GX143LRVNNQX146GG
X148X149LAGSSX152NFEFKAGX154X155TKNX158TATFNNX163IHLGRX166VNLX168VDAX172
TX173X174HFKX178IDX181X182SDGGFNX187X188LDFSGVTX192KVX193INKLTTX197ATNVX199
VKNFDIKELIVTTRVQSVGQYTIFX212X213X214IGDX216SX217IX218X219VSLQX222GYSPIF
X226GGVTFKX228GKKLVIDEFYHAPWNYFDARX237ITDVEIX240KKFLFGAPGX248IX250GK
TGLMFNNLX256LX257X258NAX259MDYGKDLDLTIX267X268X269FTNNX271X272X273MNLLVQ
X277X278RVAX282LNVX284X285X286AX288MX289FNNX291IDX292X293TGFYX294PX295IKINX298A
QNLTKNKX303HVLLX304AX305NIX307YX308X309VX310SX311X312X313X314X315X316X317X318X319
NX321X322EX323FKERLALYNNNNRMDX329CVVRX331X332X333X334DIKX335CX336X337AIGNQ
X340MVNNPX343X344YX345YLX346GKAWKNX348X349IX350KX351AX352X353SX354IX355VX356
X357X358X359NX361X362X363X364X365X366X367X368X369TTX372LPX375NX377X378X379
 wherein **X₁** is a peptide sequence of **SEQ ID NO:3** or a homologous variant thereof, and the other residues are as defined in SEQ ID NO: 1.
**Insert X₁**
   SEQ ID NO:3
   **NXₚ₃₂Xₚ₃₃NXₚ₃₅PIXₚ₃₈SEXₚ₃₁₁R** wherein:
   **X**ₚ₃₂ is absent or present and when present is Threonine or a homologous variant thereof.
   **X**ₚ₃₃ is absent or present, and when present is selected from Proline or Alanine or homologous variants thereof.
   **X**ₚ₃₅ is present or absent, and when present is selected from Aspartate or Glutamate or homologous variants thereof.
   **X**p₃₈ is absent or present, and when present is selected from Histidine or Arginine or homologous variants thereof and
   **Xp**₃₁₁ is present or absent, and when present is selected from Serine or Asparagine, or homologous variants thereof.
**Insert X₇₈**
   **SNXᵢ₇₈₃** wherein:
   **Xp**ᵢ₇₈₃ is selected from Glutamine or Histidine or homologous variants thereof.
**Insert X₁₇₃**
   SEQ ID NO:6
   **Xₚ₆₁Xₚ₆₂FNGNIXₚ₆₈LGKSTNLRVNXₚ₆₁₉Xₚ₆₂₀Xₚ₆₂₁** wherein:
   **X**ₚ₆₁ is selected from Alanine, Threonine, or homologous variants thereof.
   **X**ₚ₆₂ is selected from Asparagine, Lysine, Tyrosine, or homologous variants thereof.
   **X**ₚ₆₈ is selected from Tyrosine, Asparagine, or homologous variants thereof.
   **X**ₚ₆₁₉ is selected from Glycine, Alanine, or homologous variants thereof.
   **X**ₚ₆₂₀ is selected from Histidine, Asparagine, or homologous variants thereof and
   **X**ₚ₆₂₁ is selected from Serine, Threonine, or homologous variants thereof.
**Insert X₁₈₂**
   **Xᵢ₁₈₂₁K** wherein:
   **X**ᵢ₁₈₂₁ is selected from Threonine, Serine, or homologous variants thereof.
**Insert X₁₈₇**
**TS**
**Insert X₃₁₀**
   **Xᵢ₃₁₀₁Xᵢ₃₁₀₂X₁₃₁₀₃Xᵢ₃₁₀₄Xᵢ₃₁₀₅** wherein:
   **X**ᵢ₃₁₀₁ is selected from Glycine, Serine, or homologous variants thereof.
   **X**ᵢ₃₁₀₂ is selected from Valine, Threonine, Methionine, Alanine, or homologous variants thereof.
   **X**ᵢ₃₁₀₃ is selected from Glutamine, Asparagine, or homologous variants thereof.
   **X**ᵢ₃₁₀₄ is selected from Glycine, Alanine, or homologous variants thereof and
   **X**ᵢ₃₁₀₅ is selected from Alanine, Threonine, Asparagine, or homologous variants thereof.
**Insert X₃₃₁**
   **X**ᵢ₃₃₁₁**X**ᵢ₃₃₁₂ wherein:
   **X**ᵢ₃₃₁₁ is selected from Lysine, Asparagine, or homologous variants thereof and
   **X**ᵢ₃₃₁₂ is selected from Aspartate, Asparagine, Glycine, Glutamate, Tyrosine, Lysine, or homologous variants thereof.
**Insert X₃₇₉**
   **Xᵢ₃₇₉₁Xᵢ₃₇₉₂ Xᵢ₃₇₉₃ Xᵢ₃₇₉₄ Xᵢ₃₇₉₅ Xᵢ₃₇₉₆ Xᵢ₃₇₉₇YA** wherein:
   **X**ᵢ₃₇₉₁ is selected from Asparagine, Lysine, Aspartate, Glutamate, Serine, or homologous variants thereof.
   **X**ᵢ₃₇₉₂ is selected from Asparagine, Serine, Lysine, Aspartate, or homologous variants thereof.
   **X**ᵢ₃₇₉₃ is selected from Alanine, Threonine, Valine, or homologous variants thereof.
   **X**ᵢ₃₇₉₄ is selected from Arginine, Aspartate, or homologous variants thereof.
   **X**ᵢ₃₇₉₅ is selected from Phenylalanine, Alanine, Serine, or homologous variants thereof.
   **X**ᵢ₃₇₉₆ is selected from Alanine, Lysine, or homologous variants thereof and
   **X**ᵢ₃₇₉₇ is selected from Serine, Arginine, Asparagine, or homologous variants thereof.
**S1m1 family** 
SEQ ID NO:10
X1AFFTTVIIPAIVGGIATGTAVGTVSGLLGWGLKQAEEANKTPDKPDKVWRIQAGKGF
NEFPNKEYDLYKSLLSSKIDGGWDWGNAATHYWIKGGQWNKLEVDMKDAVGTYKLS
GLRNFTGGDLDVNMQKATLRLGQFNGNSFTSYKDSADRTTRVDFNAKNILIDNFLEIN
NRVGSGAGRKASSTVLTLQASEGITSSKNAEISLYDGATLNLASNX78SVKLNGNVWMG
RLQYVGAYLAPSYSTINTSKVTGEVNFNHLTVGDHNAAQAGIIASNKTHIGTLDLWQS
AGLNIIAPPX117GGKDTVVNIDRINTKADGTIKVGGFKASLTTNAAHLNIGKGGVNLSNQ
ASGRTLLVENLTGNITVDGPLRVNNQVGGYALAGSSANFEFKAGVDTKNGTATFNNDI
SLGRFVNLKVDAHTX173ANFKGIDTX182GNGGFNX187TLDFSGVTNKVNINKLITASTNV
 
AVKNFNINELIVKTNGVSVGEYTHFSEDIGSQSRINTVRLETGTRSIFSGGVKFKSGEKL
VIDEFYYSPWNYFDARNIKNVEITRKFASSTPENPWGTSKLMFNNLTLGQNAVMDYSQ
FSNLTIQGDFINNQGTINYLVRGGKVATLNVGNAAAMMFNNDIDSATGFYKPLIKINSA
QDLIKNTEHVLLKAKIIGYGNVX310STGTNGISNVNLEEQFKERLALYNNNNRMDTCVV
RX331NTDDIKACGMAIGNQSMVNNPDNYKYLIGKAWKNIGISKTANGSKISVYYLGNS
TPTENGGNTTNLPTNTTX379
 wherein Inserts X₁, X₇₈, X₁₁₇, X₁₇₃, X₁₈₂, X₁₈₇, **X₃₁₀** and **X₃₃₁** are absent, Insert **X₃₇₉** is present and is as defined in SEQ ID NO: 1.
**S1m1 family with G14A &G18A mutations** 
SEQ ID NO:11
X1AFFTTVIIPAIVGAIATATAVGTVSGLLGWGLKQAEEANKTPDKPDKVWRIQAGKGF
NEFPNKEYDLYKSLLSSKIDGGWDWGNAATHYWIKGGQWNKLEVDMKDAVGTYKLS
GLRNFTGGDLDVNMQKATLRLGQFNGNSFTSYKDSADRTTRVDFNAKNILIDNFLEIN
NRVGSGAGRKASSTVLTLQASEGITSSKNAEISLYDGATLNLASNX78SVKLNGNVWMG
RLQYVGAYLAPSYSTINTSKVTGEVNFNHLTVGDHNAAQAGIIASNKTHIGTLDLWQS
AGLNIIAPPX117GGKDTVVNIDRINTKADGTIKVGGFKASLTTNAAHLNIGKGGVNLSNQ
ASGRTLLVENLTGNITVDGPLRVNNQVGGYALAGSSANFEFKAGVDTKNGTATFNNDI
SLGRFVNLKVDAHTX173ANFKGIDTX182GNGGFNX187TLDFSGVTNKVNINKLITASTNV
AVKNFNINELIVKTNGVSVGEYTHFSEDIGSQSRINTVRLETGTRSIFSGGVKFKSGEKL
VIDEFYYSPWNYFDARNIKNVEITRKFASSTPENPWGTSKLMFNNLTLGQNAVMDYSQ
FSNLTIQGDFINNQGTINYLVRGGKVATLNVGNAAAMMFNNDIDSATGFYKPLIKINSA
QDLIKNTEHVLLKAKIIGYGNVX310STGTNGISNVNLEEQFKERLALYNNNNRMDTCVV
RX331NTDDIKACGMAIGNQSMVNNPDNYKYLIGKAWKNIGISKTANGSKISVYYLGNS
TPTENGGNTTNLPTNTTX379
 wherein Inserts X₁, X₇₈, X₁₁₇, X₁₇₃, X₁₈₂, X₁₈₇, X₃₁₀ and X₃₃₁ are absent, Insert X₃₇₉ is present and is as defined in SEQ ID NO:1.
**S1m1 family lacking Insert X₃₇₉** 
SEQ ID NO:12
X1AFFTTVIIPAIVGGIATGTAVGTVSGLLGWGLKQAEEANKTPDKPDKVWRIQAGKGF
NEFPNKEYDLYKSLLSSKIDGGWDWGNAATHYWIKGGQWNKLEVDMKDAVGTYKLS
GLRNFTGGDLDVNMQKATLRLGQFNGNSFTSYKDSADRTTRVDFNAKNILIDNFLEIN
NRVGSGAGRKASSTVLTLQASEGITSSKNAEISLYDGATLNLASNX78SVKLNGNVWMG
RLQYVGAYLAPSYSTINTSKVTGEVNFNHLTVGDHNAAQAGIIASNKTHIGTLDLWQS
AGLNIIAPPX117GGKDTVVNIDRINTKADGTIKVGGFKASLTTNAAHLNIGKGGVNLSNQ
ASGRTLLVENLTGNITVDGPLRVNNQVGGYALAGSSANFEFKAGVDTKNGTATFNNDI
SLGRFVNLKVDAHTX173ANFKGIDTX182GNGGFNX187TLDFSGVTNKVNINKLITASTNV
AVKNFNINELIVKTNGVSVGEYTHFSEDIGSQSRINTVRLETGTRSIFSGGVKFKSGEKL
VIDEFYYSPWNYFDARNIKNVEITRKFASSTPENPWGTSKLMFNNLTLGQNAVMDYSQ
FSNLTIQGDFINNQGTINYLVRGGKVATLNVGNAAAMMFNNDIDSATGFYKPLIKINSA
QDLIKNTEHVLLKAKIIGYGNVX310STGTNGISNVNLEEQFKERLALYNNNNRMDTCVV
RX331NTDDIKACGMAIGNQSMVNNPDNYKYLIGKAWKNIGISKTANGSKISVYYLGNS
TPTENGGNTTNLPTNTTX379
 wherein Inserts X₁, X₇₈, X₁₁₇, X₁₇₃, X₁₈₂, X₁₈₇, X₃₁₀, **X₃₃₁** and **X₃₇₉** are absent.
**S1m1 family with G14A & G18A mutations and lacking Insert X₃₇₉** SEQ ID NO:13 
X1AFFTTVIIPAIVGAIATATAVGTVSGLLGWGLKQAEEANKTPDKPDKVWRIQAGKGF
NEFPNKEYDLYKSLLSSKIDGGWDWGNAATHYWIKGGQWNKLEVDMKDAVGTYKLS
GLRNFTGGDLDVNMQKATLRLGQFNGNSFTSYKDSADRTTRVDFNAKNILIDNFLEIN
NRVGSGAGRKASSTVLTLQASEGITSSKNAEISLYDGATLNLASNX78SVKLNGNVWMG
RLQYVGAYLAPSYSTINTSKVTGEVNFNHLTVGDHNAAQAGIIASNKTHIGTLDLWQS
AGLNIIAPPX117GGKDTVVNIDRINTKADGTIKVGGFKASLTTNAAHLNIGKGGVNLSNQ
ASGRTLLVENLTGNITVDGPLRVNNQVGGYALAGSSANFEFKAGVDTKNGTATFNNDI
SLGRFVNLKVDAHTX173ANFKGIDTX182GNGGFNX187TLDFSGVTNKVNINKLITASTNV
AVKNFNINELIVKTNGVSVGEYTHFSEDIGSQSRINTVRLETGTRSIFSGGVKFKSGEKL
VIDEFYYSPWNYFDARNIKNVEITRKFASSTPENPWGTSKLMFNNLTLGQNAVMDYSQ
FSNLTIQGDFINNQGTINYLVRGGKVATLNVGNAAAMMFNNDIDSATGFYKPLIKINSA
QDLIKNTEHVLLKAKIIGYGNVX310STGTNGISNVNLEEQFKERLALYNNNNRMDTCVV
RX331NTDDIKACGMAIGNQSMVNNPDNYKYLIGKAWKNIGISKTANGSKISVYYLGNS
TPTENGGNTTNLPTNTTX379
 wherein Inserts X₁, X₇₈, X₁₁₇, X₁₇₃, X₁₈₂, X₁₈₇, X₃₁₀, X₃₃₁ and X₃₇₉ are absent.
**S2m2 family and G26A and G30A mutations** 
SEQ ID NO:14
X1AFFTTVIIPAIVGAIATAAAVGTVSGLLSWGLKQAEQANKAPDKPDKVWRIQAGRGF
DNFPHKQYDLYKSLLSSKIDGGWDWGNAARHYWVKDGQWNKLEVDMQNAVGTYN
LSGLINFTGGDLDVNMQKATLRLGQFNGNSFTSFKDGANRTTRVNFDAKNILIDNFVEI
NNRVGSGAGRKASSTVLTLKSSEKITSRENAEISLYDGATLNLVSSX78SVDLYGKVWM
GRLQYVGAYLAPSYSTIDTSKVQGEMNFRHLAVGDQNAAQAGIIANKKTNIGTLDLW
QSAGLSIITPPX117GGKDTVVNIFHLNTKADGTLRAGGFKASLSTNAAHLHIGEGGVNLS
NQASGRTLLVENLTGNITVEGTLRVNNQVGGAAIAGSSANFEFKAGEDTNNATATFNN
DIHLGKAVNLRVDAHTX173AHFKNIDAX182SDNGLNX187TLDFSGVTDKVNINKLTTAA
TNVNIKNFDIKELVVTTRVQSFGQYTIFGENIGDKSRIGVVSLQTGYSPAYSGGVTFKGG
KKLVIDEIYHAPWNYFDARNVTDVEINKRILFGAPGNIAGKTGLMFNNLTLNSNASMD
YGKDLDLTIQGHFTNNQGTMNLFVQDGRVATLNAGHQASMIFNNLVDSTTGFYKPLIK
INNAQNLTKNKEHVLVKARNIDYNLVX310SYDNISASNTNLQEQFKERLALYNNNNRM
DTCVVRX331NLNDIKACGMAIGNQSMVNNPENYKYLEGKAWKNTGINKTANNTTIAV
NLGNNSTPTNSTTDTTNLPTNTTX379
 wherein Inserts X₁, X₇₈, X₁₇₃, X₁₈₂, X₁₈₇, X₃₁₀, X₃₃₁ and X₃₇₉ are present and as defined in SEQ ID NO: 2, Insert X₁₁₇ is absent.
**S2m2 family lacking SEQ ID NO:6 Insert X₁₇₃)** 
SEQ ID NO:15
X1AFFTTVIIPAIVGGIATGAAVGTVSGLLSWGLKQAEQANKAPDKPDKVWRIQAGRGF
DNFPHKQYDLYKSLLSSKIDGGWDWGNAARHYWVKDGQWNKLEVDMQNAVGTYN
LSGLINFTGGDLDVNMQKATLRLGQFNGNSFTSFKDGANRTTRVNFDAKNILIDNFVEI
NNRVGSGAGRKASSTVLTLKSSEKITSRENAEISLYDGATLNLVSSX78SVDLYGKVWM
GRLQYVGAYLAPSYSTIDTSKVQGEMNFRHLAVGDQNAAQAGIIANKKTNIGTLDLW
QSAGLSIITPPX117GGKDTVVNIFHLNTKADGTLRAGGFKASLSTNAAHLHIGEGGVNLS
NQASGRTLLVENLTGNITVEGTLRVNNQVGGAAIAGSSANFEFKAGEDTNNATATFNN
DIHLGKAVNLRVDAHTX173AHFKNIDAX182SDNGLNX187TLDFSGVTDKVNINKLTTAA
TNVNIKNFDIKELVVTTRVQSFGQYTIFGENIGDKSRIGVVSLQTGYSPAYSGGVTFKGG
KKLVIDEIYHAPWNYFDARNVTDVEINKRILFGAPGNIAGKTGLMFNNLTLNSNASMD
YGKDLDLTIQGHFTNNQGTMNLFVQDGRVATLNAGHQASMIFNNLVDSTTGFYKPLIK
INNAQNLTKNKEHVLVKARNIDYNLVX310SYDNISASNTNLQEQFKERLALYNNNNRM
 
DTCVVRX331NLNDIKACGMAIGNQSMVNNPENYKYLEGKAWKNTGINKTANNTTIAV
NLGNNSTPTNSTTDTTNLPTNTTX379
 wherein Inserts X₁, X₇₈, X₁₈₂, X₁₈₇, X₃₁₀, **X₃₃₁** and X₃₇₉, are present and as defined in SEQ ID NO:2, Inserts X₁₁₇ and **X₁₇₃** are absent.
**S2m2 family** 
SEQ ID NO:16
X1AFFTTVIIPAIVGGIATGAAVGTVSGLLSWGLKQAEQANKAPDKPDKVWRIQAGRGF
DNFPHKQYDLYKSLLSSKIDGGWDWGNAARHYWVKDGQWNKLEVDMQNAVGTYN
LSGLINFTGGDLDVNMQKATLRLGQFNGNSFTSFKDGANRTTRVNFDAKNILIDNFVEI
NNRVGSGAGRKASSTVLTLKSSEKITSRENAEISLYDGATLNLVSSX78SVDLYGKVWM
GRLQYVGAYLAPSYSTIDTSKVQGEMNFRHLAVGDQNAAQAGIIANKKTNIGTLDLW
QSAGLSIITPPX117GGKDTVVNIFHLNTKADGTLRAGGFKASLSTNAAHLHIGEGGVNLS
NQASGRTLLVENLTGNITVEGTLRVNNQVGGAAIAGSSANFEFKAGEDTNNATATFNN
DIHLGKAVNLRVDAHTX173AHFKNIDAX182SDNGLNX187TLDFSGVTDKVNINKLTTAA
TNVNIKNFDIKELVVTTRVQSFGQYTIFGENIGDKSRIGVVSLQTGYSPAYSGGVTFKGG
KKLVIDEIYHAPWNYFDARNVTDVEINKRILFGAPGNIAGKTGLMFNNLTLNSNASMD
YGKDLDLTIQGHFTNNQGTMNLFVQDGRVATLNAGHQASMIFNNLVDSTTGFYKPLIK
INNAQNLTKNKEHVLVKARNIDYNLVX310SYDNISASNTNLQEQFKERLALYNNNNRM
DTCVVRX331NLNDIKACGMAIGNQSMVNNPENYKYLEGKAWKNTGINKTANNTTIAV
NLGNNSTPTNSTTDTTNLPTNTTX379
 wherein Inserts X₁, X₇₈, X₁₇₃, X₁₈₂, X₁₈₇, X₃₁₀, X₃₃₁, and X₃₇₉ are present and as defined in SEQ ID NO:2, Insert X₁₁₇ is absent.
S2m2 family lacking SEQ ID NO:6 (Insert X₁₇₃) 
SEQ ID NO:17
X1AFFTTVIIPAIVGGIATGAAVGTVSGLLSWGLKQAEQANKAPDKPDKVWRIQAGRGF
DNFPHKQYDLYKSLLSSKIDGGWDWGNAARHYWVKDGQWNKLEVDMQNAVGTYN
LSGLINFTGGDLDVNMQKATLRLGQFNGNSFTSFKDGANRTTRVNFDAKNILIDNFVEI
NNRVGSGAGRKASSTVLTLKSSEKITSRENAEISLYDGATLNLVSSX78SVDLYGKVWM
GRLQYVGAYLAPSYSTIDTSKVQGEMNFRHLAVGDQNAAQAGIIANKKTNIGTLDLW
QSAGLSIITPPX117GGKDTVVNIFHLNTKADGTLRAGGFKASLSTNAAHLHIGEGGVNLS
NQASGRTLLVENLTGNITVEGTLRVNNQVGGAAIAGSSANFEFKAGEDTNNATATFNN
DIHLGKAVNLRVDAHTX173AHFKNIDAX182SDNGLNX187TLDFSGVTDKVNINKLTTAA
TNVNIKNFDIKELVVTTRVQSFGQYTIFGENIGDKSRIGVVSLQTGYSPAYSGGVTFKGG
KKLVIDEIYHAPWNYFDARNVTDVEINKRILFGAPGNIAGKTGLMFNNLTLNSNASMD
YGKDLDLTIQGHFTNNQGTMNLFVQDGRVATLNAGHQASMIFNNLVDSTTGFYKPLIK
INNAQNLTKNKEHVLVKARNIDYNLVX310SYDNISASNTNLQEQFKERLALYNNNNRM
DTCVVRX331NLNDIKACGMAIGNQSMVNNPENYKYLEGKAWKNTGINKTANNTTIAV
NLGNNSTPTNSTTDTTNLPTNTTX379
 wherein Inserts X₁, X₇₈, X₁₈₂, X₁₈₇, X₃₁₀, X₃₃₁ and X₃₇₉ are present and as defined in SEQ ID NO:2, Inserts X₁₁₇ and X₁₇₃ are absent.
**S2m2 family lacking SEQ ID NO:6 (X₁₇₃) and Insert X₃₇₉** 
SEQ ID NO:19
X1AFFTTVIIPAIVGGIATGAAVGTVSGLLSWGLKQAEQANKAPDKPDKVWRIQAGRGF
DNFPHKQYDLYKSLLSSKIDGGWDWGNAARHYWVKDGQWNKLEVDMQNAVGTYN
LSGLINFTGGDLDVNMQKATLRLGQFNGNSFTSFKDGANRTTRVNFDAKNILIDNFVEI
NNRVGSGAGRKASSTVLTLKSSEKITSRENAEISLYDGATLNLVSSX78SVDLYGKVWM
 
GRLQYVGAYLAPSYSTIDTSKVQGEMNFRHLAVGDQNAAQAGIIANKKTNIGTLDLW
QSAGLSIITPPX117GGKDTVVNIFHLNTKADGTLRAGGFKASLSTNAAHLHIGEGGVNLS
NQASGRTLLVENLTGNITVEGTLRVNNQVGGAAIAGSSANFEFKAGEDTNNATATFNN
DIHLGKAVNLRVDAHTX173AHFKNIDAX182SDNGLNX187TLDFSGVTDKVNINKLTTAA
TNVNIKNFDIKELVVTTRVQSFGQYTIFGENIGDKSRIGVVSLQTGYSPAYSGGVTFKGG
KKLVIDEIYHAPWNYFDARNVTDVEINKRILFGAPGNIAGKTGLMFNNLTLNSNASMD
YGKDLDLTIQGHFTNNQGTMNLFVQDGRVATLNAGHQASMIFNNLVDSTTGFYKPLIK
INNAQNLTKNKEHVLVKARNIDYNLVX310SYDNISASNTNLQEQFKERLALYNNNNRM
DTCVVRX331NLNDIKACGMAIGNQSMVNNPENYKYLEGKAWKNTGINKTANNTTIAV
NLGNNSTPTNSTTDTTNLPTNTTX379
 wherein Inserts X₁, X₇₈, X₁₈₂, X₁₈₇, X₃₁₀, and X₃₃₁ are present, and as defined in SEQ ID NO:2, Inserts X₁₁₇, X₁₇₃ and X₃₇₉ are absent.
**A specific sequence of Insert X₁**
   SEQ ID NO:20
   NTPNDPIHSESR
**A specific sequence of Insert X₁₇₃ (SEQ ID NO:6)**
   SEQ ID NO:23
   AYFNGNIYLGKSTNLRVNGHS
**A specific sequence of Insert X₃₁₀**
   SEQ ID NO:24
   GVQGA
**A specific sequence of Insert X₃₇₉**
   SEQ ID NO:25
   NNARFASYA
**Wild-type reference protein** 
SEQ ID NO:26
AFFTTVIIPAIVGGIATGTAVGTVSGLLSWGLKQAEEANKTPDKPDKVWRIQAGKGFNE
FPNKEYDLYKSLLSSKIDGGWDWGNAARHYWVKGGQWNKLEVDMKDAVGTYKLSG
LRNFTGGDLDVNMQKATLRLGQFNGNSFTSYKDSADRTTRVDFNAKNISIDNFVEINN
RVGSGAGRKASSTVLTLQASEGITSSKNAEISLYDGATLNLASNSVKLMGNVWMGRLQ
YVGAYLAPSYSTINTSKVTGEVNFNHLTVGDKNAAQAGIIASNKTHIGTLDLWQSAGL
NIIAPPEGGYKDKPNNTPSQSGAKNDKNESAKNNSNTQVINPPNSTQKTEIQPTQVIDGP
FAGGKDTVVNINRINTNADGTIRVGGYKASLTTNAAHLNIGKGGVNLSNQASGRSLLV
ENLTGNITVDGPLRVNNQVGGYALAGSSANFEFKAGVDTKNGTATFNNDISLGRFVNL
KVDAHTANFKGIDTGNGGFNTLDFSGVTNKVNINKLITASTNVAVKNFNINELIVKTNG
ISVGEYTHFSEDIGSQSRINTVRLETGTRSIFSGGVKFKGGEKLVIDEFYYSPWNYFDAR
NIKNVEITRKFASSTPENPWGTSKLMFNNLTLGQNAVMDYSQFSNLTIQGDFINNQGTI
NYLVRGGKVATLNVGNAAAMMFNNDIDSATGFYKPLIKINSAQDLIKNTEHVLLKAKII
GYGNVSTGTNSISNVNLEEQFKERLALYNNNNRMDTCVVRNTDDIKACGMAIGNQSM
VNNPDNYKYLIGKAWKNIGISKTANGSKISVYYLGNSTPTENGGNTTNLPTNTTNNARF
ARYA
**A comparative peptide not from the invention** SEQ ID NO:28 
AFFTTVIIPAIVGAIATATAVGTVSGLLGWGLKQAEEANKTPDKPDKVWRIQAGKGFNE
FPNKEYDLYKSLLSSKIDGGWDWGNAATHYWIKGGQWNKLEVDMKDAVGTYKLSGL
RNFTGGDLDVNMQKATLRLGQFNGNSFTSYKDSADRTTRVDFNAKNILIDNFLEINNR
VGSGAGRKASSTVLTLQASEGITSSKNAEISLYDGATLNLASNSVKLNGNVWMGRLQY
VGAYLAPSYSTINTSKVTGEVNFNHLTVGDHNAAQAGIIASNKTHIGTLDLWQSAGLNI
IAPPEGGYKDKPNNTPSQSGAKNDKQESSQNNSNTQVINPPNSTQKTEVQPTQVIDGPF
AGGKDTVVNIDRINTKADGTIKVGGFKASLTTNAAHLNIGKGGVNLSNQASGRTLLVE
NLTGNITVDGPLRVNNQVGGYALAGSSANFEFKAGVDTKNGTATFNNDISLGRFVNLK
VDAHTANFKGIDTGNGGFNTLDFSGVTNKVNINKLITASTNVAVKNFNINELIVKTNGV
SVGEYTHFSEDIGSQSRINTVRLETGTRSIFSGGVKFKSGEKLVIDEFYYSPWNYFDARNI
KNVEITRKFASSTPENPWGTSKLMFNNLTLGQNAVMDYSQFSNLTIQGDFINNQGTINY
LVRGGKVATLNVGNAAAMMFNNDIDSATGFYKPLIKINSAQDLIKNTEHVLLKAKIIGY
GNVSTGTNGISNVNLEEQFKERLALYNNNNRMDTCVVRNTDDIKACGMAIGNQSMVN
NPDNYKYLIGKAWKNIGISKTANGSKISVYYLGNSTPTENGGNTTNLPTNTTNNARFAS
YA
**A comparative peptide not from the invention** 
SEQ ID NO:30
AFFTTVIIPAIVGGIATGTAVGTVSGLLGWGLKQAEEANKTPDKPDKVWRIQAGKGFNE
FPNKEYDLYKSLLSSKIDGGWDWGNAATHYWIKGGQWNKLEVDMKDAVGTYKLSGL
RNFTGGDLDVNMQKATLRLGQFNGNSFTSYKDSADRTTRVDFNAKNILIDNFLEINNR
VGSGAGRKASSTVLTLQASEGITSSKNAEISLYDGATLNLASNSVKLNGNVWMGRLQY
VGAYLAPSYSTINTSKVTGEVNFNHLTVGDHNAAQAGIIASNKTHIGTLDLWQSAGLNI
IAPPEGGYKDKPNNTPSQSGAKNDKQESSQNNSNTQVINPPNSTQKTEVQPTQVIDGPF
AGGKDTVVNIDRINTKADGTIKVGGFKASLTTNAAHLNIGKGGVNLSNQASGRTLLVE
NLTGNITVDGPLRVNNQVGGYALAGSSANFEFKAGVDTKNGTATFNNDISLGRFVNLK
VDAHTANFKGIDTGNGGFNTLDFSGVTNKVNINKLITASTNVAVKNFNINELIVKTNGV
SVGEYTHFSEDIGSQSRINTVRLETGTRSIFSGGVKFKSGEKLVIDEFYYSPWNYFDARNI
KNVEITRKFASSTPENPWGTSKLMFNNLTLGQNAVMDYSQFSNLTIQGDFINNQGTINY
LVRGGKVATLNVGNAAAMMFNNDIDSATGFYKPLIKINSAQDLIKNTEHVLLKAKIIGY
GNVSTGTNGISNVNLEEQFKERLALYNNNNRMDTCVVRNTDDIKACGMAIGNQSMVN
NPDNYKYLIGKAWKNIGISKTANGSKISVYYLGNSTPTENGGNTTNLPTNTT
**A specific polypeptide of SEQ ID NO:10** 
SEQ ID NO:31
AFFTTVIIPAIVGGIATGTAVGTVSGLLGWGLKQAEEANKTPDKPDKVWRIQAGKGFNE
FPNKEYDLYKSLLSSKIDGGWDWGNAATHYWIKGGQWNKLEVDMKDAVGTYKLSGL
RNFTGGDLDVNMQKATLRLGQFNGNSFTSYKDSADRTTRVDFNAKNILIDNFLEINNR
VGSGAGRKASSTVLTLQASEGITSSKNAEISLYDGATLNLASNSVKLNGNVWMGRLQY
VGAYLAPSYSTINTSKVTGEVNFNHLTVGDHNAAQAGIIASNKTHIGTLDLWQSAGLNI
IAPPGGKDTVVNIDRINTKADGTIKVGGFKASLTTNAAHLNIGKGGVNLSNQASGRTLL
VENLTGNITVDGPLRVNNQVGGYALAGSSANFEFKAGVDTKNGTATFNNDISLGRFVN
LKVDAHTANFKGIDTGNGGFNTLDFSGVTNKVNINKLITASTNVAVKNFNINELIVKTN
GVSVGEYTHFSEDIGSQSRINTVRLETGTRSIFSGGVKFKSGEKLVIDEFYYSPWNYFDA
RNIKNVEITRKFASSTPENPWGTSKLMFNNLTLGQNAVMDYSQFSNLTIQGDFINNQGT
INYLVRGGKVATLNVGNAAAMMFNNDIDSATGFYKPLIKINSAQDLIKNTEHVLLKAKI
IGYGNVSTGTNGISNVNLEEQFKERLALYNNNNRMDTCVVRNTDDIKACGMAIGNQS
MVNNPDNYKYLIGKAWKNIGISKTANGSKISVYYLGNSTPTENGGNTTNLPTNTTNNA
RFASYA
**A specific polypeptide of SEQ ID NO:11** SEQ ID NO:32 
AFFTTVIIPAIVGAIATATAVGTVSGLLGWGLKQAEEANKTPDKPDKVWRIQAGKGFNE
FPNKEYDLYKSLLSSKIDGGWDWGNAATHYWIKGGQWNKLEVDMKDAVGTYKLSGL
RNFTGGDLDVNMQKATLRLGQFNGNSFTSYKDSADRTTRVDFNAKNILIDNFLEINNR
VGSGAGRKASSTVLTLQASEGITSSKNAEISLYDGATLNLASNSVKLNGNVWMGRLQY
VGAYLAPSYSTINTSKVTGEVNFNHLTVGDHNAAQAGIIASNKTHIGTLDLWQSAGLNI
IAPPGGKDTVVNIDRINTKADGTIKVGGFKASLTTNAAHLNIGKGGVNLSNQASGRTLL
VENLTGNITVDGPLRVNNQVGGYALAGSSANFEFKAGVDTKNGTATFNNDISLGRFVN
LKVDAHTANFKGIDTGNGGFNTLDFSGVTNKVNINKLITASTNVAVKNFNINELIVKTN
GVSVGEYTHFSEDIGSQSRINTVRLETGTRSIFSGGVKFKSGEKLVIDEFYYSPWNYFDA
RNIKNVEITRKFASSTPENPWGTSKLMFNNLTLGQNAVMDYSQFSNLTIQGDFINNQGT
INYLVRGGKVATLNVGNAAAMMFNNDIDSATGFYKPLIKINSAQDLIKNTEHVLLKAKI
IGYGNVSTGTNGISNVNLEEQFKERLALYNNNNRMDTCVVRNTDDIKACGMAIGNQS
MVNNPDNYKYLIGKAWKNIGISKTANGSKISVYYLGNSTPTENGGNTTNLPTNTTNNA
RFASYA
**A specific polypeptide of SEQ ID NO:12** 
SEQ ID NO:33
AFFTTVIIPAIVGGIATGTAVGTVSGLLGWGLKQAEEANKTPDKPDKVWRIQAGKGFNE
FPNKEYDLYKSLLSSKIDGGWDWGNAATHYWIKGGQWNKLEVDMKDAVGTYKLSGL
RNFTGGDLDVNMQKATLRLGQFNGNSFTSYKDSADRTTRVDFNAKNILIDNFLEINNR
VGSGAGRKASSTVLTLQASEGITSSKNAEISLYDGATLNLASNSVKLNGNVWMGRLQY
VGAYLAPSYSTINTSKVTGEVNFNHLTVGDHNAAQAGIIASNKTHIGTLDLWQSAGLNI
IAPPGGKDTVVNIDRINTKADGTIKVGGFKASLTTNAAHLNIGKGGVNLSNQASGRTLL
VENLTGNITVDGPLRVNNQVGGYALAGSSANFEFKAGVDTKNGTATFNNDISLGRFVN
LKVDAHTANFKGIDTGNGGFNTLDFSGVTNKVNINKLITASTNVAVKNFNINELIVKTN
GVSVGEYTHFSEDIGSQSRINTVRLETGTRSIFSGGVKFKSGEKLVIDEFYYSPWNYFDA
RNIKNVEITRKFASSTPENPWGTSKLMFNNLTLGQNAVMDYSQFSNLTIQGDFINNQGT
INYLVRGGKVATLNVGNAAAMMFNNDIDSATGFYKPLIKINSAQDLIKNTEHVLLKAKI
IGYGNVSTGTNGISNVNLEEQFKERLALYNNNNRMDTCVVRNTDDIKACGMAIGNQS
MVNNPDNYKYLIGKAWKNIGISKTANGSKISVYYLGNSTPTENGGNTTNLPTNTT
**A specific peptide of s1m1 family with G14A & G18A mutations** 
SEQ ID NO:34
AFFTTVIIPAIVGAIATATAVGTVSGLLGWGLKQAEEANKTPDKPDKVWRIQAGKGFNE
FPNKEYDLYKSLLSSKIDGGWDWGNAATHYWIKGGQWNKLEVDMKDAVGTYKLSGL
RNFTGGDLDVNMQKATLRLGQFNGNSFTSYKDSADRTTRVDFNAKNILIDNFLEINNR
VGSGAGRKASSTVLTLQASEGITSSKNAEISLYDGATLNLASNSVKLNGNVWMGRLQY
VGAYLAPSYSTINTSKVTGEVNFNHLTVGDHNAAQAGIIASNKTHIGTLDLWQSAGLNI
IAPPGGKDTVVNIDRINTKADGTIKVGGFKASLTTNAAHLNIGKGGVNLSNQASGRTLL
VENLTGNITVDGPLRVNNQVGGYALAGSSANFEFKAGVDTKNGTATFNNDISLGRFVN
LKVDAHTANFKGIDTGNGGFNTLDFSGVTNKVNINKLITASTNVAVKNFNINELIVKTN
GVSVGEYTHFSEDIGSQSRINTVRLETGTRSIFSGGVKFKSGEKLVIDEFYYSPWNYFDA
RNIKNVEITRKFASSTPENPWGTSKLMFNNLTLGQNAVMDYSQFSNLTIQGDFINNQGT
INYLVRGGKVATLNVGNAAAMMFNNDIDSATGFYKPLIKINSAQDLIKNTEHVLLKAKI
IGYGNVSTGTNGISNVNLEEQFKERLALYNNNNRMDTCVVRNTDDIKACGMAIGNQS
MVNNPDNYKYLIGKAWKNIGISKTANGSKISVYYLGNSTPTENGGNTTNLPTNTT
**A specific polypeptide of SEQ ID NO:16** SEQ ID NO:37 
NTPNDPIHSESRAFFTTVIIPAIVGGIATGAAVGTVSGLLSWGLKQAEQANKAPDKPDKV
WRIQAGRGFDNFPHKQYDLYKSLLSSKIDGGWDWGNAARHYWVKDGQWNKLEVDM
QNAVGTYNLSGLINFTGGDLDVNMQKATLRLGQFNGNSFTSFKDSADRTTRVNFDAK
NILIDNFVEINNRVGSGAGRKASSTVLTLKSSEKITSRENAEISLYDGATLNLVSSSNQSV
DLYGKVWMGRLQYVGAYLAPSYSTIDTSKVQGEMNFRHLAVGDQNAAQAGIIANKK
TNIGTLDLWQSAGLSIITPPGGKDTVVNIFHLNTKADGTLRAGGFKASLSTNAAHLNIGE
GGVNLSNQASGRSLLVENLTGNITVEGTLRVNNQVGGAAVAGSSANFEFKAGEDTNN
ATATFNNDIHLGKAVNLRVDAHTAYFNGNIYLGKSTNLRVNGHSAHFKNIDASKSDNG
LNTSALDFSGVTDKVNINKLTTSATNVNIKNFDIKELVVTTRVQSFGQYTIFGENIGDKS
RIGVVSLQTGYSPAYSGGVTFKSGKKLVIDEIYHAPWNYFDARNVTDVEINKKILFGAP
GNIAGKTGLMFNNLTLNSNASMDYGKDLDLTIQGHFTNNQGTMNLFVQDGRVATLNA
GHQASMIFNNLVDSATGFYKPLIKINNAQNLTKNKEHVLVKARNIDYNLVGVQGASYD
NISASNTNLQEQFKERLALYNNNNRMDTCVVRKDNLNDIKACGMAIGNQSMVNNPEN
YKYLEGKAWKNTGINKTANNTTIAVNLGNNSTPTSSESNTTNLPTNTTNNARFARYA
**A specific polypeptide of s2m2 family lacking Insert X₁₇₃** 
SEQ ID NO:38
NTPNDPIHSESRAFFTTVIIPAIVGGIATGAAVGTVSGLLSWGLKQAEQANKAPDKPDKV
WRIQAGRGFDNFPHKQYDLYKSLLSSKIDGGWDWGNAARHYWVKDGQWNKLEVDM
QNAVGTYNLSGLINFTGGDLDVNMQKATLRLGQFNGNSFTSFKDSADRTTRVNFDAK
NILIDNFVEINNRVGSGAGRKASSTVLTLKSSEKITSRENAEISLYDGATLNLVSSSNQSV
DLYGKVWMGRLQYVGAYLAPSYSTIDTSKVQGEMNFRHLAVGDQNAAQAGIIANKK
TNIGTLDLWQSAGLSIITPPGGKDTVVNIFHLNTKADGTLRAGGFKASLSTNAAHLNIGE
GGVNLSNQASGRSLLVENLTGNITVEGTLRVNNQVGGAAVAGSSANFEFKAGEDTNN
ATATFNNDIHLGKAVNLRVDAHTAHFKNIDASKSDNGLNTSALDFSGVTDKVNINKLT
TSATNVNIKNFDIKELVVTTRVQSFGQYTIFGENIGDKSRIGVVSLQTGYSPAYSGGVTF
KSGKKLVIDEIYHAPWNYFDARNVTDVEINKKILFGAPGNIAGKTGLMFNNLTLNSNAS
MDYGKDLDLTIQGHFTNNQGTMNLFVQDGRVATLNAGHQASMIFNNLVDSATGFYKP
LIKINNAQNLTKNKEHVLVKARNIDYNLVGVQGASYDNISASNTNLQEQFKERLALYN
NNNRMDTCVVRKDNLNDIKACGMAIGNQSMVNNPENYKYLEGKAWKNTGINKTAN
NTTIAVNLGNNSTPTSSESNTTNLPTNTTNNARFARYA
**A specific polypeptide of SEQ ID NO:19** 
SEQ ID NO:40
NTPNDPIHSESRAFFTTVIIPAIVGGIATGAAVGTVSGLLSWGLKQAEQANKAPDKPDKV
WRIQAGRGFDNFPHKQYDLYKSLLSSKIDGGWDWGNAARHYWVKDGQWNKLEVDM
QNAVGTYNLSGLINFTGGDLDVNMQKATLRLGQFNGNSFTSFKDSADRTTRVNFDAK
NILIDNFVEINNRVGSGAGRKASSTVLTLKSSEKITSRENAEISLYDGATLNLVSSSNQSV
DLYGKVWMGRLQYVGAYLAPSYSTIDTSKVQGEMNFRHLAVGDQNAAQAGIIANKK
TNIGTLDLWQSAGLSIITPPGGKDTVVNIFHLNTKADGTLRAGGFKASLSTNAAHLNIGE
GGVNLSNQASGRSLLVENLTGNITVEGTLRVNNQVGGAAVAGSSANFEFKAGEDTNN
ATATFNNDIHLGKAVNLRVDAHTAHFKNIDASKSDNGLNTSALDFSGVTDKVNINKLT
TSATNVNIKNFDIKELVVTTRVQSFGQYTIFGENIGDKSRIGVVSLQTGYSPAYSGGVTF
KSGKKLVIDEIYHAPWNYFDARNVTDVEINKKILFGAPGNIAGKTGLMFNNLTLNSNAS
MDYGKDLDLTIQGHFTNNQGTMNLFVQDGRVATLNAGHQASMIFNNLVDSATGFYKP
LIKINNAQNLTKNKEHVLVKARNIDYNLVGVQGASYDNISASNTNLQEQFKERLALYN
NNNRMDTCVVRKDNLNDIKACGMAIGNQSMVNNPENYKYLEGKAWKNTGINKTAN
NTTIAVNLGNNSTPTSSESNTTNLPTNTT
**A specific polypeptide of SEQ ID NO:19 containing Insert X₁₇₃** 
SEQ ID NO:41
NTPNDPIHSESRAFFTTVIIPAIVGGIATGAAVGTVSGLLSWGLKQAEQANKAPDKPDKV
WRIQAGRGFDNFPHKQYDLYKSLLSSKIDGGWDWGNAARHYWVKDGQWNKLEVDM
QNAVGTYNLSGLINFTGGDLDVNMQKATLRLGQFNGNSFTSFKDSADRTTRVNFDAK
NILIDNFVEINNRVGSGAGRKASSTVLTLKSSEKITSRENAEISLYDGATLNLVSSSNQSV
DLYGKVWMGRLQYVGAYLAPSYSTIDTSKVQGEMNFRHLAVGDQNAAQAGIIANKK
TNIGTLDLWQSAGLSIITPPGGKDTVVNIFHLNTKADGTLRAGGFKASLSTNAAHLNIGE
GGVNLSNQASGRSLLVENLTGNITVEGTLRVNNQVGGAAVAGSSANFEFKAGEDTNN
ATATFNNDIHLGKAVNLRVDAHTANFNGNIYLGKSTNLRVNGHTAHFKNIDASKSDNG
LNTSALDFSGVTDKVNINKLTTSATNVNIKNFDIKELVVTTRVQSFGQYTIFGENIGDKS
RIGVVSLQTGYSPAYSGGVTFKSGKKLVIDEIYHAPWNYFDARNVTDVEINKKILFGAP
GNIAGKTGLMFNNLTLNSNASMDYGKDLDLTIQGHFTNNQGTMNLFVQDGRVATLNA
GHQASMIFNNLVDSATGFYKPLIKINNAQNLTKNKEHVLVKARNIDYNLVGVQGASYD
NISASNTNLQEQFKERLALYNNNNRMDTCVVRKDNLNDIKACGMAIGNQSMVNNPEN
YKYLEGKAWKNTGINKTANNTTIAVNLGNNSTPTSSESNTTNLPTNTT
**A specific polypeptide of SEQ ID NO:14** 
SEQ ID NO:42
NTPNDPIHSESRAFFTTVIIPAIVGAIATAAAVGTVSGLLSWGLKQAEQANKAPDKPDKV
WRIQAGRGFDNFPHKQYDLYKSLLSSKIDGGWDWGNAARHYWVKDGQWNKLEVDM
QNAVGTYNLSGLINFTGGDLDVNMQKATLRLGQFNGNSFTSFKDSADRTTRVNFDAK
NILIDNFVEINNRVGSGAGRKASSTVLTLKSSEKITSRENAEISLYDGATLNLVSSSNQSV
DLYGKVWMGRLQYVGAYLAPSYSTIDTSKVQGEMNFRHLAVGDQNAAQAGIIANKK
TNIGTLDLWQSAGLSIITPPGGKDTVVNIFHLNTKADGTLRAGGFKASLSTNAAHLNIGE
GGVNLSNQASGRSLLVENLTGNITVEGTLRVNNQVGGAAVAGSSANFEFKAGEDTNN
ATATFNNDIHLGKAVNLRVDAHTAHFKNIDASKSDNGLNTSALDFSGVTDKVNINKLT
TSATNVNIKNFDIKELVVTTRVQSFGQYTIFGENIGDKSRIGVVSLQTGYSPAYSGGVTF
KSGKKLVIDEIYHAPWNYFDARNVTDVEINKKILFGAPGNIAGKTGLMFNNLTLNSNAS
MDYGKDLDLTIQGHFTNNQGTMNLFVQDGRVATLNAGHQASMIFNNLVDSATGFYKP
LIKINNAQNLTKNKEHVLVKARNIDYNLVGVQGASYDNISASNTNLQEQFKERLALYN
NNNRMDTCVVRKDNLNDIKACGMAIGNQSMVNNPENYKYLEGKAWKNTGINKTAN
NTTIAVNLGNNSTPTSSESNTTNLPTNTT
**A specific polypeptide of SEQ ID NO:14 containing Insert X₁₇₃**

SEQ ID NO:43
NTPNDPIHSESRAFFTTVIIPAIVGAIATAAVGTVSGLLSWGLKQAEQANKAPDKPDKV
WRIQAGRGFDNFPHKQYDLYKSLLSSKIDGGWDWGNAARHYWVKDGQWNKLEVDM
QNAVGTYNLSGLINFTGGDLDVNMQKATLRLGQFNGNSFTSFKDSADRTTRVNFDAK
NILIDNFVEINNRVGSGAGRKASSTVLTLKSSEKITSRENAEISLYDGATLNLVSSSNQSV
DLYGKVWMGRLQYVGAYLAPSYSTIDTSKVQGEMNFRHLAVGDQNAAQAGIIANKK
TNIGTLDLWQSAGLSIITPPGGKDTVVNIFHLNTKADGTLRAGGFKASLSTNAAHLNIGE
GGVNLSNQASGRSLLVENLTGNITVEGTLRVNNQVGGAAVAGSSANFEFKAGEDTNN
ATATFNNDIHLGKAVNLRVDAHTANFNGNIYLGKSTNLRVNGHTAHFKNIDASKSDNG
LNTSALDFSGVTDKVNINKLTTSATNVNIKNFDIKELVVTTRVQSFGQYTIFGENIGDKS
RIGVVSLQTGYSPAYSGGVTFKSGKKLVIDEIYHAPWNYFDARNVTDVEINKKILFGAP
GNIAGKTGLMFNNLTLNSNASMDYGKDLDLTIQGHFTNNQGTMNLFVQDGRVATLNA
GHQASMIFNNLVDSATGFYKPLIKINNAQNLTKNKEHVLVKARNIDYNLVGVQGASYD
NISASNTNLQEQFKERLALYNNNNRMDTCVVRKDNLNDIKACGMAIGNQSMVNNPEN
YKYLEGKAWKNTGINKTANNTTIAVNLGNNSTPTSSESNTTNLPTNTT

## Claims

1. An isolated polypeptide having a sequence of SEQ ID NO:1 or SEQ ID NO:2 or a homologous variant, wherein a variant amino acid sequence is identical to the referenced peptide sequence with the exception of the deletion, insertion and/or substitution of a few amino acids, i.e.. 1, 2, 3, 4, or 5 amino acids amongst the defined non-variable amino acids of the referenced polypeptide sequence.

2. An isolated polypeptide according to claim 1, wherein said peptide is of SEQ ID NO:1 or a homologous variant or fragment thereof and Glycine 14 is mutated to Alanine (G14A), Glycine 18 is mutated to Alanine (G18A).

3. An isolated polypeptide according to claim 1 or 2, wherein Insert X₃₇₉ is absent.

4. An isolated polypeptide according to any one of claim 1 to 3, wherein Inserts X₇₈, X₁₇₃, X₁₈₂, X₁₈₇, X₃₁₀, X₃₃₁ and X₃₇₉ are absent.

5. An isolated polypeptide according to claim 1 or 2, wherein Insert X₃₇₉ is present.

6. An isolated polypeptide according to any one of claims 1 to 5, wherein Insert X₁₇₃ is present.

7. An isolated polypeptide according to any one of claims 1 to 5, wherein Insert X₁₇₃ is absent.

8. An isolated polypeptide according to claim 1, having sequence selected from SEQ ID 10-13.

9. An isolated polypeptide according to claim 8, having a sequence selected from SEQ ID NO: 31-34.

10. An isolated polypeptide according to claim 1, wherein said peptide is of SEQ ID NO: 2 wherein Glycine 26 is mutated to Alanine (G26A), Glycine 30 is mutated to Alanine (G30A).

11. An isolated polypeptide according to claim 1, wherein said peptide is of SEQ ID NO: 2 or a homologous variant or fragment thereof wherein Insert X₁₇₃ is absent.

12. An isolated polypeptide according to any one of claims 1, wherein said peptide is of SEQ ID NO: 2 and has a sequence selected from SEQ ID NO: 14-17 and 19.

13. An isolated polypeptide according to claim 12, wherein said peptide has the sequence selected from SEQ ID NO: 37-38 and 40-43.

14. An isolated peptide according to any more of claims 1 to 13 for use as a medicament.

15. An isolated peptide according to any more of claims 1 to 13 for use in the prevention and/or treatment of an allergic disorder and/or inducing a tolerization response to an allergen, and/or reducing an inflammatory response caused by an allergen/or the prevention and/or treatment of an autoimmune disease or disorder or a fibrotic disease or disorder.

16. An isolated peptide for use according to claim 15, wherein said an allergic disorder is selected from allergen-induced or atopic asthma, atopic dermatitis, atopic rhinitis, allergic conjunctivitis, food allergy, occupational allergy, allergic broncho-pulmonal aspergillosis and eosinophilic esophagitis.

17. An isolated peptide for use according to claim 15, wherein said inflammatory response caused by an allergen is an inflammatory bowel disease (IBD).

18. An isolated peptide for use according to claim 15, wherein said autoimmune disease or disorder is selected from systemic rheumatic diseases like systemic lupus erythematosus, systemic sclerosis, Sjogren syndrome, vasculitis, and others, or organ-specific, such as endocrine and neurologic disorders, including autoimmune thyroiditis and multiple sclerosis.

19. An isolated peptide for use according to claim 15, wherein said fibrotic disease or disorder is selected from systemic sclerosis, sclerodermatous graft vs. host disease, as well as numerous organ-specific disorders including radiation-induced fibrosis and cardiac, pulmonary, liver, and kidney fibrosis.

20. A pharmaceutical formulation comprising at least one peptide according to anyone of claims 1 to 13 and at least one pharmaceutically acceptable carrier, diluent, or excipient thereof.

## Patentansprüche

1. Isoliertes Polypeptid, aufweisend eine Sequenz von SEQ ID Nr. 1 oder SEQ ID Nr. 2 oder eine homologe Variante, wobei eine Aminosäuresequenzvariante identisch mit der referenzierten Peptidsequenz ist, mit Ausnahme der Deletion, Insertion und/oder Substitution einiger weniger Aminosäuren, d. h. 1, 2, 3, 4 oder 5 Aminosäuren, von den definierten nichtvariablen Aminosäuren der referenzierten Polypeptidsequenz.

2. Isoliertes Polypeptid nach Anspruch 1, wobei das Peptid von SEQ ID Nr. 1 oder einer homologen Variante oder einem Fragment davon ist und Glycin 14 zu Alanin mutiert ist (G14A), Glycin 18 zu Alanin mutiert ist (G18A).

3. Isoliertes Polypeptid nach Anspruch 1 oder 2, wobei das Insert X₃₇₉ fehlt.

4. Isoliertes Polypeptid nach einem der Ansprüche 1 bis 3, wobei die Inserte X₇₈, X₁₇₃, X₁₈₂, X₁₈₇, X₃₁₀, X₃₃₁ und X₃₇₉ fehlen.

5. Isoliertes Polypeptid nach Anspruch 1 oder 2, wobei das Insert X₃₇₉ vorliegt.

6. Isoliertes Polypeptid nach einem der Ansprüche 1 bis 5, wobei das Insert X₁₇₃ vorliegt.

7. Isoliertes Polypeptid nach einem der Ansprüche 1 bis 5, wobei das Insert X₁₇₃ fehlt.

8. Isoliertes Polypeptid nach Anspruch 1, aufweisend eine Sequenz, die aus SEQ ID 10-13 ausgewählt ist.

9. Isoliertes Polypeptid nach Anspruch 8, aufweisend eine Sequenz, die aus SEQ ID Nr. 31-34 ausgewählt ist.

10. Isoliertes Polypeptid nach Anspruch 1, wobei das Peptid von SEQ ID Nr. 2 ist, wobei Glycin 26 zu Alanin mutiert ist (G26A), Glycin 30 zu Alanin (G30A) mutiert ist.

11. Isoliertes Polypeptid nach Anspruch 1, wobei das Peptid von SEQ ID Nr. 2 oder einer homologen Variante oder einem Fragment davon ist, wobei das Insert X₁₇₃ fehlt.

12. Isoliertes Polypeptid nach einem der Ansprüche, wobei das Peptid von SEQ ID Nr. 2 ist und eine Sequenz aufweist, die aus SEQ ID Nr. 14-17 und 19 ausgewählt ist.

13. Isoliertes Polypeptid nach Anspruch 12, wobei das Peptid die Sequenz aufweist, die aus SEQ ID Nr. 37-38 und 40-43 ausgewählt ist.

14. Isoliertes Peptid nach einem oder mehreren der Ansprüche 1 bis 13 zur Verwendung als ein Arzneimittel.

15. Isoliertes Peptid nach einem oder mehreren der Ansprüche 1 bis 13 zur Verwendung bei der Prävention und/oder Behandlung einer allergischen Erkrankung und/oder Induktion einer Tolerierungsantwort auf ein Allergen und/oder Verringerung einer Entzündungsreaktion, die von einem Allergen verursacht wird, oder der Prävention und/oder Behandlung einer Autoimmunkrankheit oder -erkrankung oder einer Fibrosekrankheit oder -erkrankung.

16. Isoliertes Peptid zur Verwendung nach Anspruch 15, wobei die allergische Erkrankung aus allergeninduziertem oder atopischem Asthma, atopischer Dermatitis, atopischer Rhinitis, allergischer Konjunktivitis, Lebensmittelallergie, Berufsallergie, allergischer bronchopulmonaler Aspergillose und eosinophiler Ösophagitis ausgewählt ist.

17. Isoliertes Peptid zur Verwendung nach Anspruch 15, wobei die Entzündungsreaktion, die von einem Allergen verursacht wird, eine entzündliche Darmerkrankung (IBD) ist.

18. Isoliertes Peptid zur Verwendung nach Anspruch 15, wobei die Autoimmunkrankheit oder -erkrankung aus systemischen Rheumakrankheiten, wie systemischem Lupus erythematodes, systemischer Sklerose, Sjögren-Syndrom, Vaskulitis und andere, oder organspezifischen, wie endokrinen und neurologischen Erkrankungen, einschließlich Autoimmunthyreopathie und multipler Sklerose, ausgewählt ist.

19. Isoliertes Peptid zur Verwendung nach Anspruch 15, wobei die Fibrosekrankheit oder -erkrankung aus systemischer Sklerose, sklerodermieähnlicher Graft-versus-Host-Reaktion sowie zahlreichen organspezifischen Erkrankungen, einschließlich strahlungsinduzierter Fibrose und Herz-, Lungen-, Leber- und Nierenfibrose, ausgewählt ist.

20. Pharmazeutische Formulierung, umfassend mindestens ein Peptid nach einem der Ansprüche 1 bis 13 und mindestens einen pharmazeutisch unbedenklichen Trägerstoff, ein pharmazeutisch unbedenkliches Verdünnungsmittel oder einen pharmazeutisch unbedenklichen Hilfsstoff davon.

## Revendications

1. Polypeptide isolé ayant la séquence de la SEQ ID NO : 1 ou de la SEQ ID NO : 2 ou un variant homologue, dans lequel une séquence d'acides aminés variante est identique à la séquence peptidique référencée à l'exception de la délétion, de l'insertion et/ou de la substitution de quelques acides aminés, c'est-à-dire de 1, 2, 3, 4 ou 5 acides aminés parmi les acides aminés non variables définis de la séquence polypeptidique référencée.

2. Polypeptide isolé selon la revendication 1, dans lequel ledit peptide est de la SEQ ID NO : 1 ou un fragment ou variant homologue de celle-ci et la glycine 14 est mutée en alanine (G14A), la glycine 18 est mutée en alanine (G18A).

3. Polypeptide isolé selon la revendication 1 ou 2, dans lequel l'insert X₃₇₉ est absent.

4. Polypeptide isolé selon l'une quelconque des revendications 1 à 3, dans lequel les inserts X₇₈, X₁₇₃, X₁₈₂, X₁₈₇, X₃₁₀, X₃₃₁ et X₃₇₉ sont absents.

5. Polypeptide isolé selon la revendication 1 ou 2, dans lequel l'insert X₃₇₉ est présent.

6. Polypeptide isolé selon l'une quelconque des revendications 1 à 5, dans lequel l'insert X₁₇₃ est présent.

7. Polypeptide isolé selon l'une quelconque des revendications 1 à 5, dans lequel l'insert X₁₇₃ est absent.

8. Polypeptide isolé selon la revendication 1, ayant une séquence choisie parmi les SEQ ID: 10 à 13.

9. Polypeptide isolé selon la revendication 8, ayant une séquence choisie parmi les SEQ ID NO : 31 à 34.

10. Polypeptide isolé selon la revendication 1, dans lequel ledit peptide est de la SEQ ID NO : 2 dans laquelle la glycine 26 est mutée en alanine (G26A), la glycine 30 est mutée en alanine (G30A).

11. Polypeptide isolé selon la revendication 1, dans lequel ledit peptide est de la SEQ ID NO : 2 ou un fragment ou variant homologue de celle-ci dans lequel l'insert X₁₇₃ est absent.

12. Polypeptide isolé selon l'une quelconque des revendications, dans lequel ledit peptide est de la SEQ ID NO : 2 et a une séquence choisie parmi les SEQ ID NO : 14 à 17 et 19.

13. Polypeptide isolé selon la revendication 12, dans lequel ledit peptide a la séquence choisie parmi les SEQ ID NO : 37 à 38 et 40 à 43.

14. Peptide isolé selon l'une ou plusieurs des revendications 1 à 13, pour une utilisation en tant que médicament.

15. Peptide isolé selon l'une ou plusieurs des revendications 1 à 13, pour une utilisation dans la prévention et/ou le traitement d'un trouble allergique et/ou l'induction d'une réponse de tolérisation à un allergène et/ou la réduction d'une réponse inflammatoire causée par un allergène ou la prévention et/ou le traitement d'une maladie ou d'un trouble auto-immun(e) ou d'une maladie ou d'un trouble fibrotique.

16. Peptide isolé pour une utilisation selon la revendication 15, dans lequel ledit trouble allergique est choisi parmi un asthme allergique ou atopique, une dermatite atopique, une rhinite atopique, une conjonctivite allergique, une allergie alimentaire, une allergie professionnelle, une aspergillose bronchopulmonaire allergique et une œsophagite à éosinophiles.

17. Peptide isolé pour une utilisation selon la revendication 15, dans lequel ladite réponse inflammatoire causée par un allergène est une maladie intestinale inflammatoire (IBD).

18. Peptide isolé pour une utilisation selon la revendication 15, dans lequel ladite maladie ou ledit trouble auto-immun(e) est choisi parmi les maladies rhumatismales systémiques telles qu'un lupus érythémateux disséminé, une sclérodermie généralisée, un syndrome de Sjögren, une vascularite, et d'autres, ou à spécificité d'organe, comme les troubles endocriniens et neurologiques, y compris une thyroïdite auto-immune et une sclérose en plaques.

19. Peptide isolé pour une utilisation selon la revendication 15, dans lequel ladite maladie ou ledit trouble fibrotique est choisi parmi une sclérodermie généralisée, une réaction sclérodermateuse du greffon contre l'hôte, ainsi que de nombreux troubles à spécificité d'organe y compris une fibrose induite par une irradiation et une fibrose cardiaque, pulmonaire, hépatique et rénale.

20. Formulation pharmaceutique comprenant au moins un peptide selon l'une quelconque des revendications 1 à 13 et au moins un véhicule, diluant ou excipient pharmaceutiquement acceptable de celui-ci.
